# EUROPEAN PATENT APPLICATION

(11) **EP 3 712 272 A1**
(43) Date of publication of application: **23.09.2020**
(21) Application number: 18837474.8
(22) Date of filing: 24.07.2018
(51) Int. Cl.: C12N 15/85

(54) **METHOD FOR MODULATING RNA SPLICING BY INDUCING BASE MUTATION AT SPLICE SITE OR BASE SUBSTITUTION IN POLYPYRIMIDINE REGION**

(30) Priority: 25.07.2017 CN 201710611651
(71) Applicant: Shanghai Institutes for Biological Sciences, Chinese Academy of Sciences, Shanghai 200031 (CN)
(72) Inventor: CHANG, Xing, Shanghai 200031 (CN); YUAN, Juanjuan, Shanghai 200031 (CN); MA, Yunqing, Shanghai 200031 (CN)
(74) Representative: V.O.
(86) International application number: PCT/CN2018/096810
(87) International publication number: WO 2019/020007

(57) **Abstract**

Provided is a method for modulating RNA splicing by inducing a base mutation at a splice site or a base substitution in a polypyrimidine region. The method comprises expressing a targeting cytosine deaminase in a cell, to induce AG at a 3' splice site of an intron of interest in a gene of interest to mutate into AA, or to induce GT at a 5' splice site of the intron of interest in a gene of interest to mutate to AT, or to induce a plurality of Cs in a polypyrimidine region of the intron of interest in a gene of interest to respectively mutate into Ts. The method specifically blocks an exon recognition process, modulates a selective splicing process of endogenous mRNA, induces exon skipping, activates an alternative splice site, induces mutually exclusive exon conversion, induces intron retention, and enhances an exon.

## Description

### Technical Field

The disclosure relates to a method for modulating RNA splicing by inducing base mutation at splice site or base substitution in polypyrimidine region.

### Background

The correct expression of eukaryotic genes requires the removal of introns in the pre-mRNA and the splicing of exons to form mature mRNA. More than 98% of introns are excised by a highly dynamic protein complex, the spliceosome. The spliceosome consists of more than 150 small nuclear ribonucleoproteins (snRNPs), such as U1, U2, U4, U5, and U6. During the splicing process, the U1 snRNP recognizes the GU sequence at the 5' splice site of the intron, splicing factor 1 (SF1) binds to the bifurcation point of the intron, and the 35KD subunit of the U2 auxiliary factor (U2AF) binds to the AG sequence at the 3' splice site of the intron, and its 65KD subunit binds to the polypyrimidine region sequence to complete the exon recognition process; then U5 and U6 proteins catalyze the intron removal process by regulating RNA structure reconstruction and RNA-protein interaction. The RNA splicing process plays an important role in the regulation of gene expression. Studies have found that 15% of heritable human diseases are caused by abnormal processing of pre-mRNAs, therefore the RNA splicing process can be a possible therapeutic target for these diseases. For example, the use of antisense oligonucleotides (ASO) to regulate RNA splicing of a disease-related gene can alleviate Duchenne muscular dystrophy and spinal muscular atrophy.

In addition to intron splicing, 75% of human genes undergo alternative RNA splicing during expression, greatly increasing the abundance of the human proteome. However, functions of most alternative splicing protein isoforms are not clear due to the lack of convenient and effective methods to regulate the alternative splicing process.

Antisense oligonucleotides can bind to cis-acting elements of RNA (such as exonic splicing enhancers) to block splicing of exons, but the use of antisense oligonucleotides to regulate splicing requires careful design and strict screening, and also requires continuous administration during treatment. Meanwhile, the synthesis of the antisense oligonucleotides is time-consuming and very expensive. Therefore, there is a dire need to provide a one-time cure for these diseases.

### SUMMARY

Provided herein is a method for regulating RNA splicing of a gene of interest in a cell, characterized in that the method includes expressing targeting cytosine deaminase in the cell to induce mutation of the 3' splice site AG of an intron of interest of the gene of interest in the cell to AA, or mutation of the 5' splice site GT of an intron of interest of the gene of interest in the cell to AT, or mutation of multiple Cs in the polypyrimidine region of an intron of interest of the gene of interest in the cell to Ts.

In one or more embodiments, the targeting cytosine deaminase used in the methods described herein may be selected from the group consisting of:
(1) a fusion protein of a cytosine deaminase, or a fragment or mutant thereof retaining enzyme activity, and a Cas enzyme with helicase activity and partial or no nuclease activity;
(2) a fusion protein of a cytosine deaminase, or a fragment or mutant thereof retaining enzyme activity, and a TALEN protein that specifically recognizes a target sequence;
(3) a fusion protein of a cytosine deaminase, or a fragment or mutant thereof retaining enzyme activity, and a zinc finger protein that specifically recognizes a target sequence;
(4) a fusion protein of a cytosine deaminase, or a fragment or mutant thereof retaining enzyme activity, and a Cpf enzyme with helicase activity and partial or no nuclease activity; and
(5) a fusion protein of a cytosine deaminase, or a fragment or mutant thereof retaining enzyme activity, and an Ago protein.

In one or more embodiments, the targeting cytosine deaminase is the fusion protein of a cytosine deaminase, or a fragment or mutant thereof retaining enzyme activity, and a Cas enzyme with helicase activity and partial or no nuclease activity, or the fusion protein of a cytosine deaminase, or a fragment or mutant thereof retaining enzyme activity, and a Cpf enzyme with helicase activity and partial or no nuclease activity; the method includes expressing the targeting cytosine deaminase and an sgRNA in the cell, wherein the sgRNA is specifically recognized by the Cas enzyme or Cpf enzyme and binds to the sequence having a splice site of an intron of interest of the gene of interest, or binds to the complementary sequence of a polypyrimidine region of interest.

In one or more embodiments, the targeting cytosine deaminase is the fusion protein of a cytosine deaminase, or a fragment or mutant thereof retaining enzyme activity, and an Ago protein; the method includes a step of expressing in the cell the targeting cytosine deaminase and a gDNA recognized by the Ago protein.

In one or more embodiments, provided herein is a method of regulating RNA splicing of a gene of interest in a cell, the method comprising a step of expressing in the cell (1) a fusion protein of a Cas protein with helicase activity and partial or no nuclease activity, and cytosine deaminase AID or a mutant thereof, and (2) an sgRNA; wherein, the Cas protein recognition region of the sgRNA is specifically recognized by the Cas protein, and the sgRNA binds to the sequence having a splice site of an intron of interest of the gene of interest, or binds to the complementary sequence of a polypyrimidine region of interest.

In one or more embodiments, the sgRNA binds to the sequence having the 5' splice site of the intron of interest of the gene of interest, and the fusion protein mutates the GT at the 5' splice site to AT, thereby inducing exon skipping, activating alternative splice sites, inducing mutually exclusive exon switching or intron retention.

In one or more embodiments, the sgRNA binds to the sequence having the 3' splice site of the intron of interest of the gene of interest, and the fusion protein mutates the AG at the 3' splice site to AA, thereby inducing exon skipping, activating alternative splice sites, inducing mutually exclusive exon switching or intron retention.

In one or more embodiments, the sgRNA binds to the complementary sequence of the polypyrimidine region of interest, and induces the C at the polypyrimidine region to T, thereby enhancing exon inclusion.

In one or more embodiments, RNA splicing of the gene of interest in the cell is regulated by transferring expression vector(s) of the fusion protein and the sgRNA into the cell.

In one or more embodiments, the method further includes a step of simultaneously transferring an expression plasmid of Ugi.

In one or more embodiments, the method further includes a step of simultaneously transferring expression plasmid(s) of a fusion protein of a nuclease-deficient or nuclease-partially-deficient Cas9 protein, AID or a mutant thereof, and an Ugi.

In one or more embodiments, the fusion protein and AID, a fragment or a mutant thereof are as described in any part or any embodiment herein.

In one or more embodiments, the cell of interest and the gene of interest are as described in any part or any embodiment herein.

In certain embodiments, provided herein is a method for inducing exon skipping, the method comprising a step of expressing in the cell (1) a fusion protein of a Cas protein with helicase activity and partial or no nuclease activity, cytosine deaminase AID or a mutant thereof, and an optional Ugi fusion protein, and (2) an sgRNA; wherein, the Cas protein recognition region of the sgRNA is specifically recognized by the Cas protein, and the sgRNA binds to the sequence having a splice site of an intron of interest of the gene of interest.

In certain embodiments, provided herein is a method for activating alternative splice site(s), the method comprising a step of expressing in the cell (1) a fusion protein of a Cas protein with helicase activity and partial or no nuclease activity, cytosine deaminase AID or a mutant thereof, and an optional Ugi fusion protein, and (2) an sgRNA; wherein, the Cas protein recognition region of the sgRNA is specifically recognized by the Cas protein, and the sgRNA binds to the sequence having a splice site of an intron of interest of the gene of interest, wherein the intron of interest has alternative splice site(s) nearby.

In certain embodiments, provided herein is a method for inducing mutually exclusive exon switching, the method comprising a step of expressing in the cell (1) a fusion protein of a Cas protein with helicase activity and partial or no nuclease activity, cytosine deaminase AID or a mutant thereof, and optional an Ugi, and (2) an sgRNA; wherein, the Cas protein recognition region of the sgRNA is specifically recognized by the Cas protein, and the target binding region of the sgRNA comprises the sequence of a splice site of an intron of interest of the gene of interest, wherein the gene of interest is slected from a group consisting of PKMs.

In certain embodiments, provided herein is a method for inducing intron retention, the method comprising a step of expressing in the cell (1) a fusion protein of a Cas protein with helicase activity and partial or no nuclease activity, cytosine deaminase AID or a mutant thereof, and optional an Ugi fusion protein, and (2) an sgRNA; wherein, the Cas protein recognition region of the sgRNA is specifically recognized by the Cas protein, and the sgRNA comprises a splice site of the intron of interest, wherein the intron of interest is short in length (<150bp) and rich in G/C bases.

In certain embodiments, provided herein is a method for enhancing exon inclusion, the method comprising a step of expressing in the cell (1) a fusion protein of a Cas protein with helicase activity and partial or no nuclease activity, cytosine deaminase AID or a mutant thereof, and optional an Ugi, and (2) an sgRNA; wherein, the Cas protein recognition region of the sgRNA is specifically recognized by the Cas protein, and the sgRNA comprises the complementary sequence of the polypyrimidine region upstream of the exon of interest.

Also provided herein is a fusion protein that contains a Cas protein with helicase activity and partial or no nuclease activity and cytosine deaminase AID or a mutant thereof.

In one or more embodiments, the fusion protein herein also contains Ugi.

Also provided herein is a fusion protein for generating a point mutation in a cell, or for regulating RNA splicing of a gene of interest in a cell, or for inducing exon skipping, activating alternative splicing sites, inducing mutually exclusion exon switching, inducing intron retention, or enhancing exon inclusion in a cell of interest, wherein the fusion protein contains a Cas protein with helicase activity and partial or no nuclease activity and cytosine deaminase AID or a mutant thereof, and optional a linker sequence, a nuclear localization sequence, and Ugi.

Also provided herein is a method for treating a disease using the method for regulating RNA splicing described herein.

Also provided herein is use of the fusion protein described herein or its expression vector and the corresponding sgRNA or its expression vector in the preparation of a kit for regulating RNA splicing, as well as a kit comprising the fusion protein described herein or its expression and the corresponding sgRNA or its expression vector.

### BRIEF DESCRIPTION OF DRAWINGS

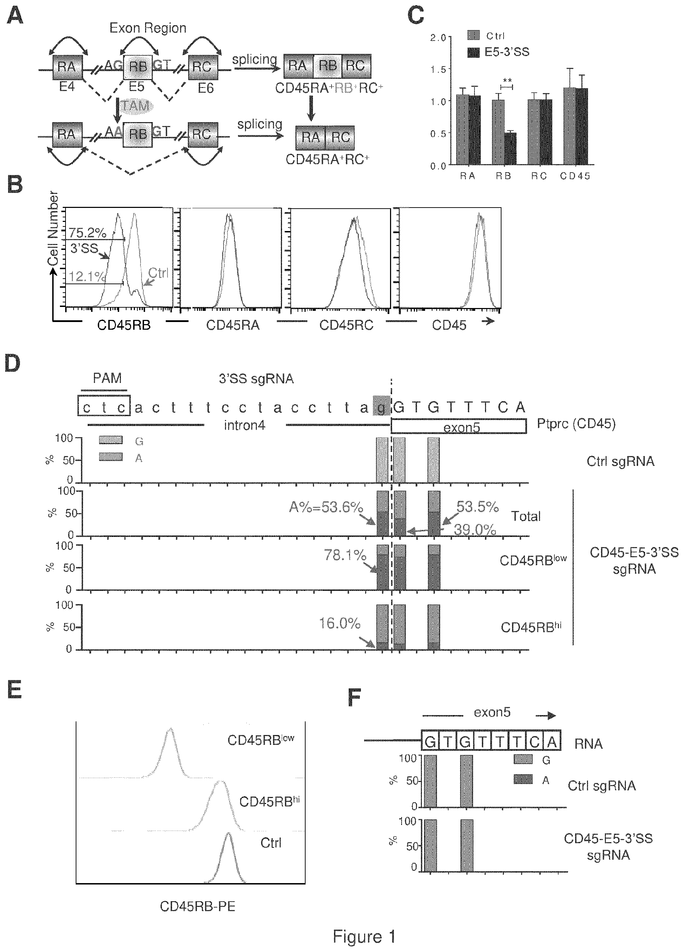

Figure 1: TAM induced exon 5 skipping in CD45 by converting the invariant guanine to adenine at the 3' splice site. (A) A schematic diagram of using TAM to convert guanine to adenine at the 3' splice site of CD45 RB exon and induce exon skipping. In WT Raji cells, combined splicing exon 5 of CD45 produced the longest CD45 isoform (CD45RA⁺RB⁺RC⁺, top panel); TAM converted the AG dinucleotide to AA at 3'SS of exon 5, thereby eliminating this splice site and disrupting exon recognition, leading exon 5 skipping and production of the CD45 isoform lacking the CD45RB (CD45RA⁺RC⁺, botton panel). (B, C) TAM caused CD45RB exon skipping. Raji cells were transfected with the expression plasmid(s) of AIDx-nCas9-Ugi and the targeting sgRNA (CD45-E5-3'SS) or a control sgRNA targeting the AAVS1 (Ctrl). Seven days after transfection, expression of the targeted exon (CD45RB), its upstream exon (exon 4, CD45RA), downstream exon (Exon 6, CD45RC) and total CD45 was determined by flow cytometry using exon-specific antibodies (B); or the expression of the corresponding exons was detected by exon-specific real-time PCR (C). The data are representative (B) or summary (C) of two independent experiments. **, p<0.01 in Student's t test. (D) In CD45RB^{low} cells, the G>A mutation at the 3'SS was enriched. Intron-exon junctions were amplified from the genomic DNA of the cells shown in B and the sorted CD45RB^{hi} and CD45RB^{low} cells from TAM-treated cells. The amplicons were analyzed by high-throughput sequencing with over 8000x coverage. The base composition of each nucleotide having a detectable mutation (mutant reading/WT reading >0.1%) is depicted, and the percentage of G>A conversion of the mutated Gs is marked. The locations of the sgRNA and PAM sequences are shown on the top of the intron-exon junction sequence. Intron/exon junctions are depicted using dashed lines. The data are representative of two independent experiments. (E) Flow cytometric analysis of CD45RB expression in control Raji cells or sorted CD45RB^{hi} and CD45RB^{low} cells from TAM-treated cells. (F) TAM induced CD45RB skipping without changing the coding sequence of CD45. As in D, the exon-intron junctions were amplified from cDNA and analyzed for base substitution by high-throughput sequencing. Note that the two exon mutations are not detectable in the cDNA of TAM-treated cells as compared with genomic DNA.

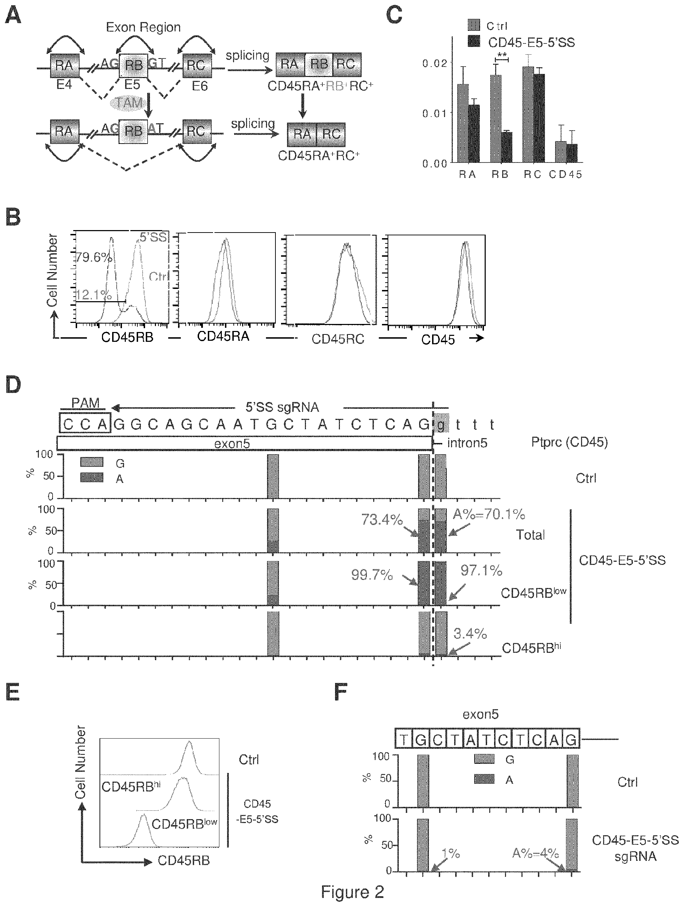

Figure 2: TAM induced CD45RB exon skipping by converting the invariant guanine at the 5' splice site to adenine. (A) A schematic diagram of directing TAM to convert the invariant guanine at the 5' SS of CD45 RB exon to adenine, and induce exon skipping. (B, C) TAM caused CD45RB exon skipping. Raji cells were transfected with the expression plasmid(s) of AIDx-nCas9-Ugi and targeting sgRNA (E5-5'SS) or control sgRNA against AAVS1 (Ctrl). Seven days after transfection, the expression of the targeted exon (CD45RB), its upstream exon (exon 4, CD45RA), downstream exon (Exon 6, CD45RC) and total CD45 was determined by flow cytometry using exon-specific antibodies (B), or by exon-specific real-time PCR (C). The data are representative (B) or summary (C) of two independent experiments. **, p<0.01 in Student's t test. (D) G>A mutation was enriched at the 5' site of CD45RB exon in CD45RB^{low} cells. Intron-exon junctions were amplified from the cells shown in B and the sorted CD45RB^{hi} and CD45RB^{low} cells from TAM-treated Raji cells. The amplicons were analyzed by high-throughput sequencing with over 8000x coverage. The base composition of each nucleotide having a detectable mutation (mutant reading/WT reading >0.1%) is depicted, and the percentage of G>A conversion of the target G is marked on the left. The locations of the sgRNA and PAM sequences are marked on the top of the intron-exon junction sequence. Intron/exon junctions are depicted using dashed lines. The data are representative of two independent experiments. (E) Flow cytometric analysis of CD45RB expression in control Raji cells or sorted CD45RB^{hi} and CD45RB^{low} cells from TAM-treated cells. (F) TAM induced CD45RB skipping and minimal changes in CD45 protein sequence. The exon-intron junctions were amplified from cDNA and analyzed for base substitution by high-throughput sequencing. Note that the two mutations in the cDNA of TAM-treated cells are significantly reduced as compared with genomic DNA.

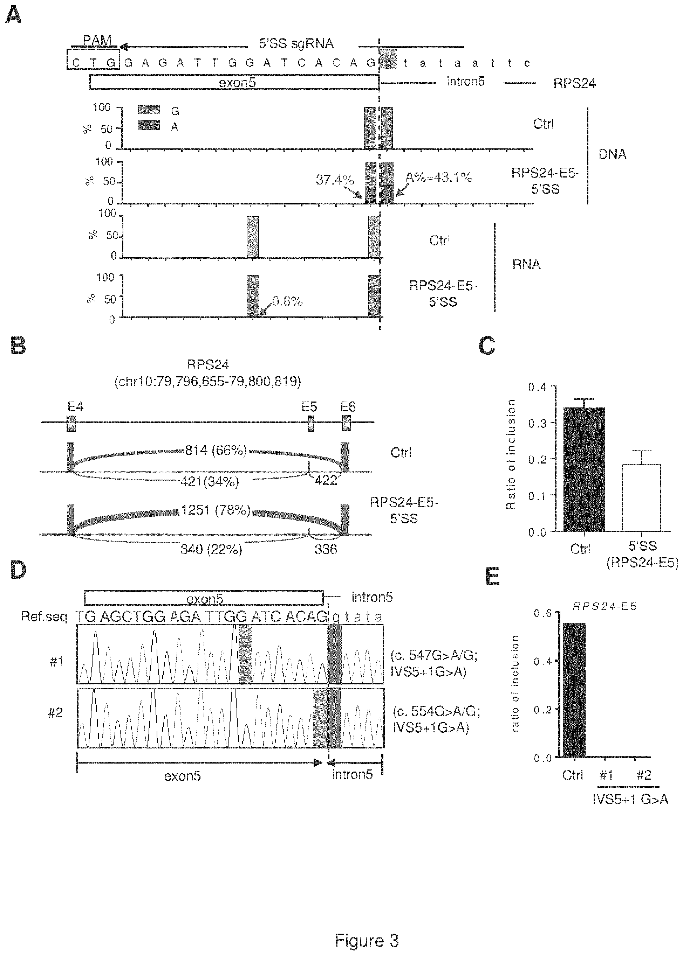

Figure 3: TAM promoted skipping of RPS24 exon 5 by converting the invariant guanine at the 5' SS to adenine. (A) The conversion of adenine at the 5' splice site of RPS24 exon 5 to adenine by TAM 293T cells were transfected with the expression plasmid(s) of nCas9-AIDx-Ugi and control sgRNA (Ctrl) or the sgRNA targeting the 5' SS of RPS24 exon 5 (5') (E5-5'SS). Six days after transfection, sgRNA targeted regions were amplified from genomic DNA (top 2 panels) or cDNA (bottom 2 panels) and analyzed by high-throughput sequencing with over 8000x coverage. The base composition of nucleotides having detectable mutations (>0.1%) is depicted. The locations of the sgRNA and PAM sequences are shown on the top of the exon/intron junction sequence from Refseq. Intron/exon junctions are depicted using dashed lines. The data are representative of two independent experiments. (B) TAM promoted the skipping of exon 5 in RPS24. As in A, the splicing junctions were amplified from cDNA and analyzed by high-throughput sequencing. The Figure shows the coverage and percentage of each splicing junction of the cells treated with control sgRNA (top panel) or E5-5'SS sgRNA (bottom panel). The count and percentage (in parentheses) of the junction readings are depicted on the top of each junction arc. For clarity, only the junction arcs representing more than 1% of the total transcripts are depicted. (C) The ratio of the RPS24 isoform to the included or skipped exon 5 was determined by isoform-specific real-time PCR. The data are the summary of three independent experiments. (D, E) The 5'SS G to A mutation caused a complete skipping of RPS24 exon 5. Two single-cell clones were obtained from TAM-treated cells and analyzed by Sanger sequencing. The right of (D) shows the genotype of the cells. The expression of the isoform including exon 5 was determined by real-time PCR (E). The data are the summary of three independent experiments.

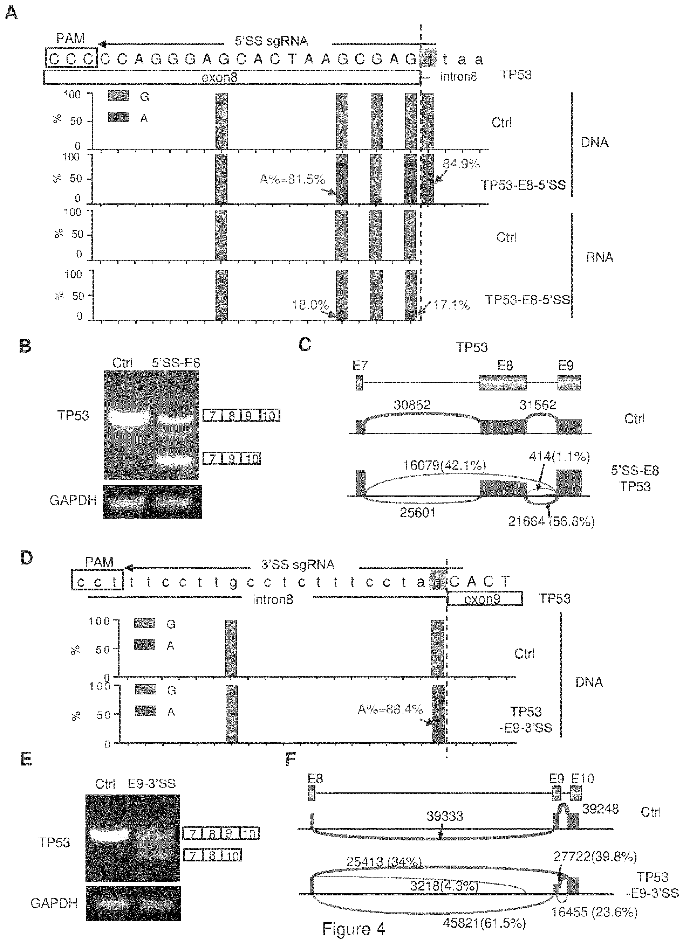

Figure 4: TAM induced skipping of exon 8 or exon 9 in TP53 by mutating guanine at their respective splice site. (A-C) TAM caused the skipping of exon 8 in TP53 by mutating its 5'SS. (A) As shown in Figure 1, 293T cells were transfected with the expression plasmid(s) of nCas9-AIDx-Ugi and control sgRNA against AAVS1 (Ctrl) or sgRNA targeting 5'SS of TP53 exon 8 (E8-5'SS). Six days after transfection, sgRNA targeted regions were amplified from genomic DNA (top 2 panels) or cDNA (bottom 2 panels) and analyzed by high-throughput sequencing. The base composition of nucleotides having detectable mutations (>0.1%) is depicted. The locations of the sgRNA and PAM sequences are shown on the top of the exon/intron junction sequence from Refseq. Intron/exon junctions are depicted using dashed lines. The data are representative of two independent experiments. (B) Analysis of splicing of TP53 exon 8 by RT-PCR. (C) As in A, the splicing junctions were amplified from cDNA and analyzed by high-throughput sequencing. The Figure shows the coverage and percentage of each splicing junction of the cells treated with control sgRNA (top panel) or E8-5'SS sgRNA (bottom panel). For clarity, only the junction arcs representing more than 1% of the total transcripts are depicted. The count and percentage (in parentheses) of the junction readings are depicted on the top of each junction arc. Note that in TAM-treated cells, 42.1% of the total transcript skiped exon 8, while 1.1% activated the cryptic splice site within exon 8. (D-F) TAM caused the skipping of exon 9 in TP53 by mutating its 3'SS. (D) As shown in (A), 293T cells were transfected using TAM and sgRNA targeting 3'SS of TP53 exon 9. Seven days after transfection, intron-exon junctions were amplified from genomic DNA and analyzed by high-throughput sequencing. (E) Analysis of TP53 splicing by RT-PCR. (F) As in D, the splicing junctions were amplified from cDNA and analyzed by high-throughput sequencing. Intersections that account for more than 1% of total transcripts are depicted. Note that 3'SS mutation caused exon skipping in 34% of the total transcripts and activatiton of the cryptic splice site in 23.6% of the mRNAs. TAM-treated cells also activated the neuronal exon within intron 8 (4.3% of the total transcripts). (A-F) Data represent two independent experiments.

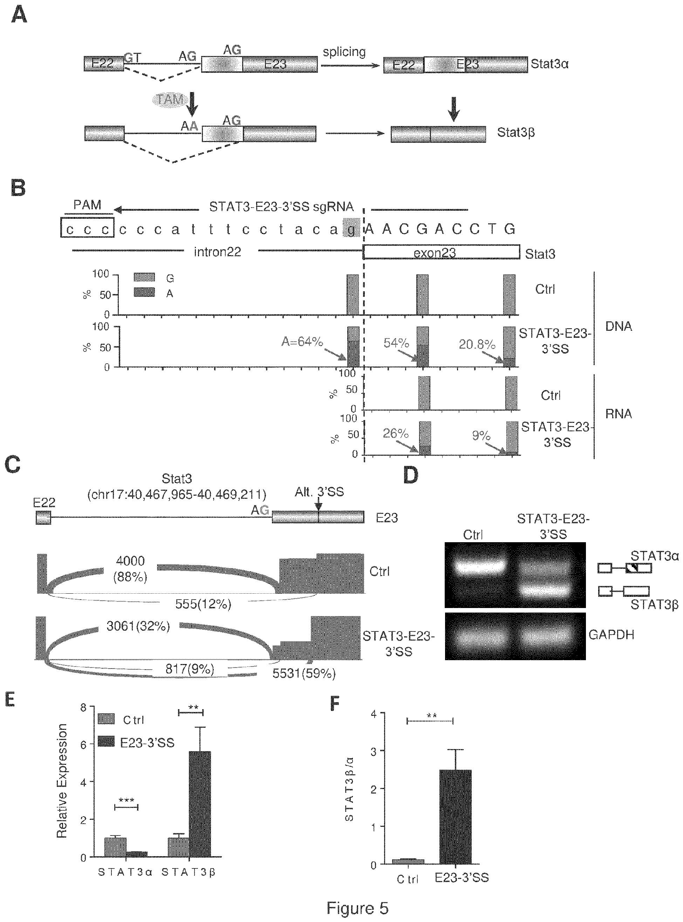

Figure 5: TAM activated alternative splice sites and converted Stat3α to Stat3β. (A) A schematic diagram of eliminating the typical 3'SS of Stat3 exon 23 (Stat3α) and promoting the use of downstream alternative 3'SS (Stat3β) by TAM. (B) Mutation of the invariant G at the typical 3'SS of Stat3 exon 23 by TAM. As shown in Figure 1, 293T cells were transfected with the expression plasmid(s) of AIDx-nCas9-Ugi and the sgRNA targeting Stat3 exon 23 (E23-3'SS-) or sgRNA targeting AAVS1 (Ctrl). Intron-exon junctions were amplified from DNA (top 2 panels) or cDNA (bottom 2 panels) and analyzed by high-throughput sequencing. The base composition of nucleotides having detectable mutations (>0.1%) is depicted. Note that TAM also induced two mutations in exon 23, which is much less than cDNA (26% and 6%) of cDNA (54% and 16%). The data are representative of two independent experiments. (C) TAM enhanced the use of the distal 3'SS in Stat3 exon 23. The splicing junctions were amplified from cDNA and analyzed by high-throughput sequencing. The Figure shows the coverage and percentage of each splicing junction of the cells treated with control sgRNA (top panel) or E23-3'SS sgRNA (bottom panel). Intersections that account for more than 1% of total transcripts are depicted. The count and percentage (in parentheses) of the junction readings are depicted on the top of each junction arc. Note that only in cells treated with Stat3-E23-3'SS, sgRNAs were cryptic splice sites activated in about 10% of the transcripts. The data are representative of two independent experiments. (E-F) TAM converted Stat3α to Stat3β. The expression of Stat3α and Stat3β in TAM treated cells was detected by RT-PCR (D) and isoform-specific real-time fluorescence quantitative PCR (E), and the ratio of Stat3α to Stat3β was determined (F).

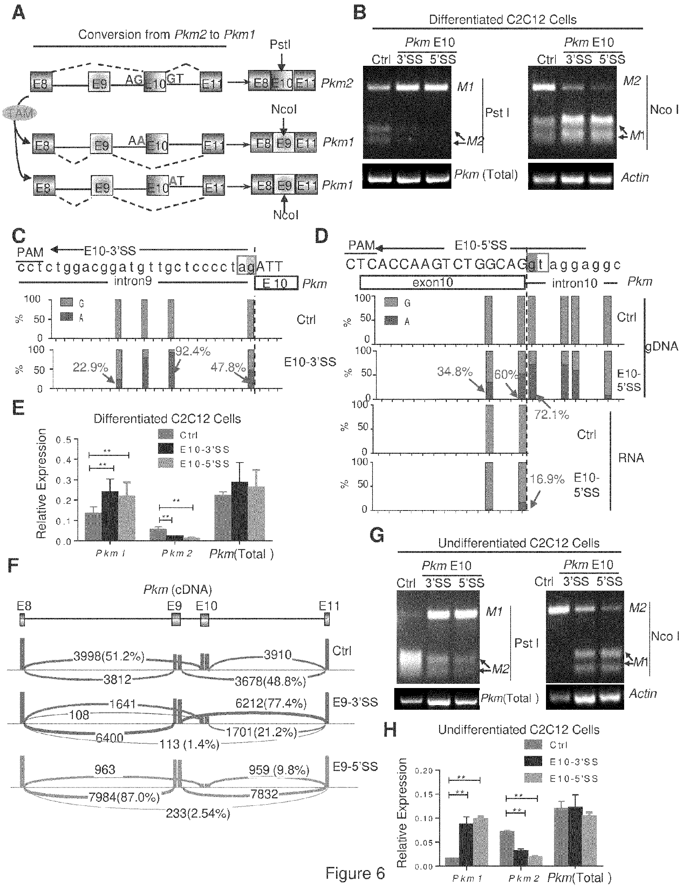

Figure 6: TAM switched PKM2 to PKM1 by eliminating the 5'SS or 3'SS of exon 10. (A) A schematic diagram showing switching of PKM2 to PKM1 in C2C12 cells by TAM. In the top panel, in WT C2C12 cells, exon 10, not exon 9 of PKM gene, was spliced to produce PKM2, whose cDNA was recognized by the restriction enzyme PstI (top panel); in the bottom panel, TAM converted the GT dinucleotide at the 5'SS of exon 10 to AT (or 3'SS AG to AA). Therefore, exon 9 instead of exon 10 was spliced to produce PKM1, whose cDNA was recognized by the restriction enzyme NcoI. (B) TAM increased PKM1 expression while inhibiting PKM2 expression. C2C12 cells were transfected with TAM and targeting sgRNA (PKM-E10-5'SS or PKM-E10-3'SS) or control sgRNA (Ctrl). Seven days after transfection, the cells were differentiated into muscle cells, then PKM was amplified from the cDNA, and the amplicon was digested with PstI or NcoI. The fragment corresponding to PKM1 or PKM2 is indicated, while GAPDH and total PKM (amplicon of exon 5 and exon 6) are included as vector controls. (C, D) TAM converted the invariant G to A at the 3'SS (C) or 5'SS (D) of PKM exon 10. Intron-exon junctions were amplified from genomic DNA (top 2 panels) or cDNA (bottom 2 panels) and analyzed by high-throughput sequencing. The base composition of each guanine and the percentage of A are described. The data are representative of two independent experiments. (E) Real-time PCR analysis of the ratio of PKM1 to PKM2. The data are representative (B, D, E) or summary of two independent experiments (C). (F) TAM converted PKM2 to PKM1. As in C, the splicing junctions were amplified from cDNA and analyzed by high-throughput sequencing. The Figure shows the coverage and percentage of each splicing junction of the cells treated with control sgRNA (top panel) or E10-5'SS sgRNA (bottom panel). The count and percentage (in parentheses) of the junction readings are depicted on the top of each junction arc. (G, H) Similar to the above, TAM can convert PKM2 to PKM1 in undifferentiated C2C12 cells.

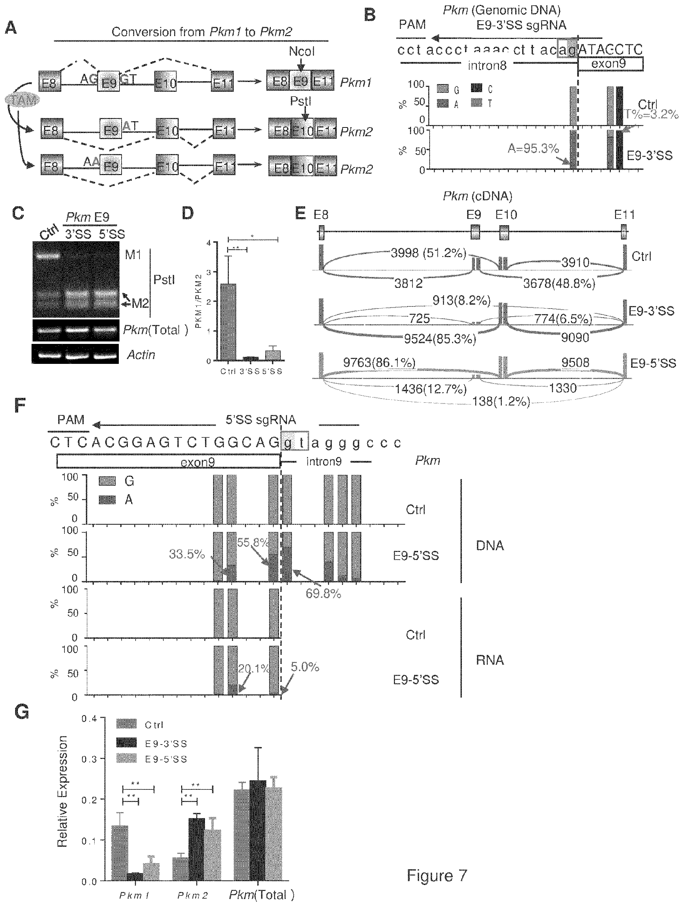

Figure 7: TAM suppressed the expression of PKM1 by eliminating the 3'SS or 5'SS of the exon 9 of PKM. (A) TAM converted the invariant G at 3'SS or 5'SS of PKM exon 9 to A. (B) Genomic DNA from control or TAM-treated cells (E9-3'SS) of muscle cells differentiated from C2C12 cells was analyzed by high-throughput sequencing. The percentage G or A of each guanine with a mutation frequency more than 1% is depicted. The data are representative of two independent experiments. Note that TAM also caused a C>T mutation in exon 9 at this position. (C, D, E) TAM inhibited PKM1 expression and meanwhile promoted PKM2 expression. (C) PKM was amplified from cDNA, and the amplicon was digested with NcoI. The fragment corresponding to PKM1 or PKM2 is indicated, while GAPDH and total PKM (amplicon of exon 5 and exon 6) are included as vector controls. (D) The expression of PKM1 and PKM2 was measured by real-time PCR, and the ratio of PKM1 to PKM2 was calculated. (E) The splicing junctions were amplified from cDNA and analyzed by high-throughput sequencing. The Figure shows the coverage and percentage of each splicing junction of the cells treated with control sgRNA (top panel) or E9-3'SS sgRNA (bottom panel). The count and percentage (in parentheses) of the junction readings are depicted on the top of each junction arc. The data are summary of two independent experiments. ***, p<0.0001 in student's t test. (F) As above, genomic DNA from control or TAM-treated cells (E9-5'SS) of muscle cells differentiated from C2C12 cells was analyzed by high-throughput sequencing. The percentage G or A of each guanine with a mutation frequency more than 1% is depicted. The data are representative of two independent experiments. (G) Real-time quantitative PCR analysis of PKM1 and PKM2 expression.

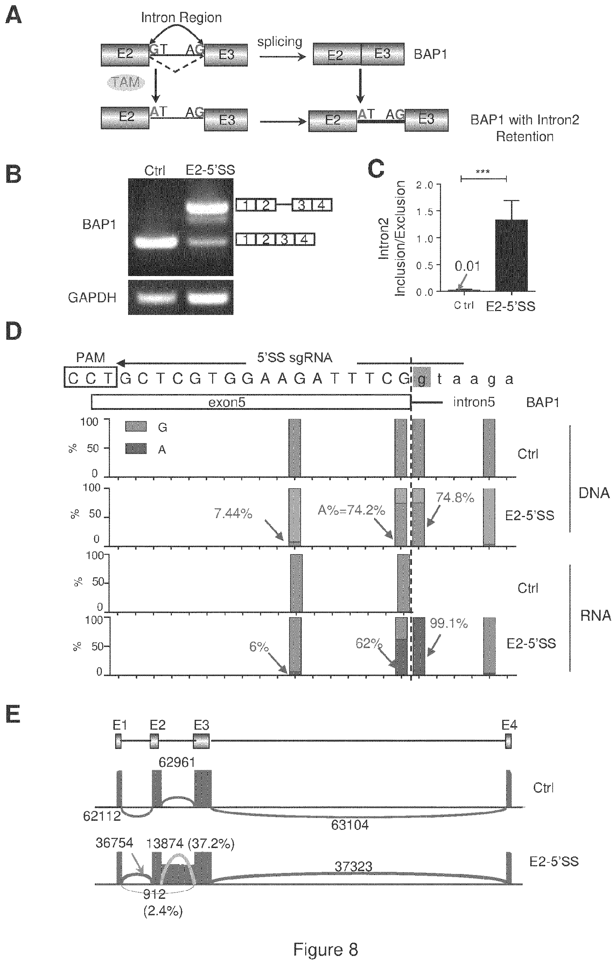

Figure 8: After TAM converted the invariant G to A on the 5'SS, intron 2 of BAP1 was retained. (A) A schematic diagram of directing TAM to mutate the invariant G at the 5' splice site of BAP1 exon 2 and showing its retention. The second intron of BAP1 may be spliced in an intron-defined manner, wherein the 5'SS is paired with the downstream 3'SS. The invariant G was converted to A, and U1 recognized U1 RNP at 5'SS and destroyed the intron definition, resulting in the inclusion of the intron. (B, C) TAM induced the retention of BAP1 intron 2. 293T cells were transfected with the expression plasmid(s) of AIDx-nCas9-Ugi and the sgRNA targeting AAVS1 (Ctrl) or sgRNA targeting 5'SS of BAP1 exon 2 (NAP1-E2-5'SS). Seven days after transfection, BAP1 mRNA splicing was analyzed by RT-PCR (B) or isoform-specific real-time PCR (C). (D) The retained intron contained a 5'SS G>A mutation. Intron-exon junctions were amplified from genomic DNA (top 2 panels) or cDNA (bottom 2 panels) of 293T cells treated with control sgRNA (ctrl) or targeting sgRNA (E2-5'SS). The base composition of each guanine with a detectable mutation is depicted. The locations of the sgRNA and PAM sequences are marked on the top of the intron-exon junction sequence. Intron/exon junctions are depicted using dashed lines. The data are representative of two independent experiments. Note that because intron 2 was effectively spliced in control cells, only cells receiving E2-5'SS sgRNA had readings that covered the intron, and 99% of them contained the G>A mutation. (E) Mutated 5'SS induced retention of the second intron, instead of skipping the second exon in BAP1. As in D, the splicing junctions were amplified from cDNA and analyzed by high-throughput sequencing. The Figure shows the coverage and percentage of each splicing junction of the cells treated with control sgRNA (top panel) or E2-5'SS sgRNA (bottom panel). The count and percentage (in parentheses) of the junction readings are depicted on the top of each junction arc. Note that, in sgRNA-treated cells, 2.4% of the mRNAs were spliced to skip the second exon, while more than 60% retained the second intron. The data are representative (B, D, E) or summary (C) of two independent experiments.

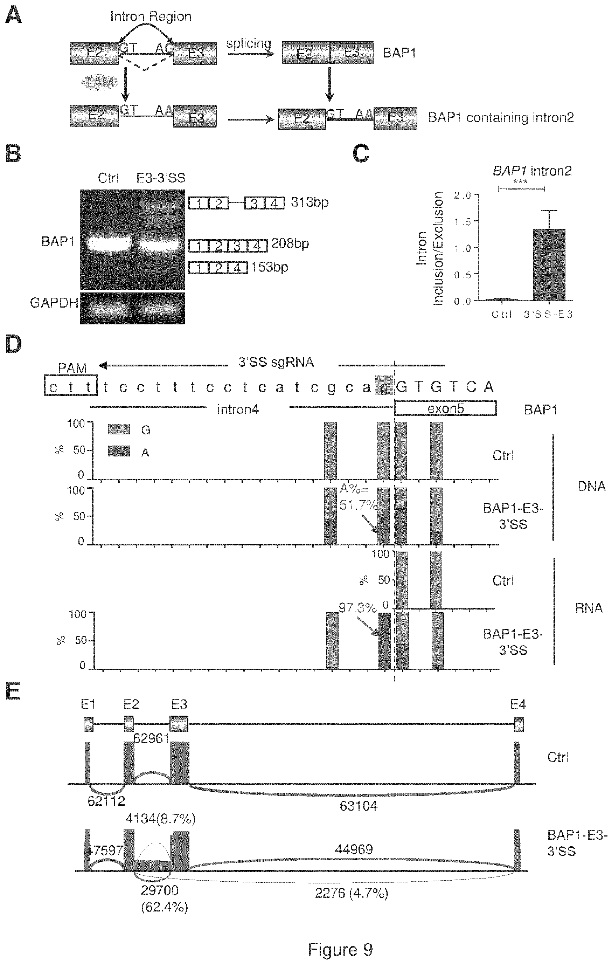

Figure 9: Conversion of invariant G to A at the 3'SS of exon 3 of BAP1 resulted in its retention. (A) A schematic diagram of directing TAM to mutate the invariant G at the 3'SS of BAP1 exon 3 and directing its retention. (B, C) TAM induced the retention of BAP1 intron 2. 293T cells were transfected with the expression plasmid(s) of AIDx-nCas9-Ugi and the sgRNA targeting AAVS1 (Ctrl) or 3'SS of BAP1 intron 2. Seven days after transfection, BAP1 mRNA splicing was analyzed by RT-PCR (B) and isoform-specific real-time PCR (C). (D) The retained second intron contained a G>A mutation at 3'SS. 5'SS was amplified from genomic DNA (top 2 panels) or cDNA (bottom 2 cells) of 293T cells treated with control sgRNA (Ctrl), or sgRNA targeting 3'ss (E3-3'SS). The base composition of each guanine with a detectable mutation is depicted (G>A conversion efficiency is more than 0.1%). The locations of the sgRNA and PAM sequences are shown on the top of the intron-exon junction sequence. Intron/exon junctions are depicted using dashed lines. The data are representative of two independent experiments. Note that because intron 2 was effectively spliced in Ctrl cells, only cells receiving E3-3'SS sgRNA had readings that covered the intron. (E) TAM mainly induced the retention of the second exon of BAP1. As in D, the splicing junctions were amplified from cDNA and analyzed by high-throughput sequencing. The Figure shows the coverage and percentage of each splicing junction of the cells treated with control sgRNA (top panel) or E3-3'SS sgRNA (bottom panel). The count and percentage (in parentheses) of the junction readings are depicted on the top of each junction arc. Note that, in sgRNA-treated cells, 4.7% of the mRNAs skipped the third exon, 8.7% used the downstream cryptic splice site, while more than 20% retained the second intron. The data are representative (B, D, E) or summary (C) of two independent experiments.

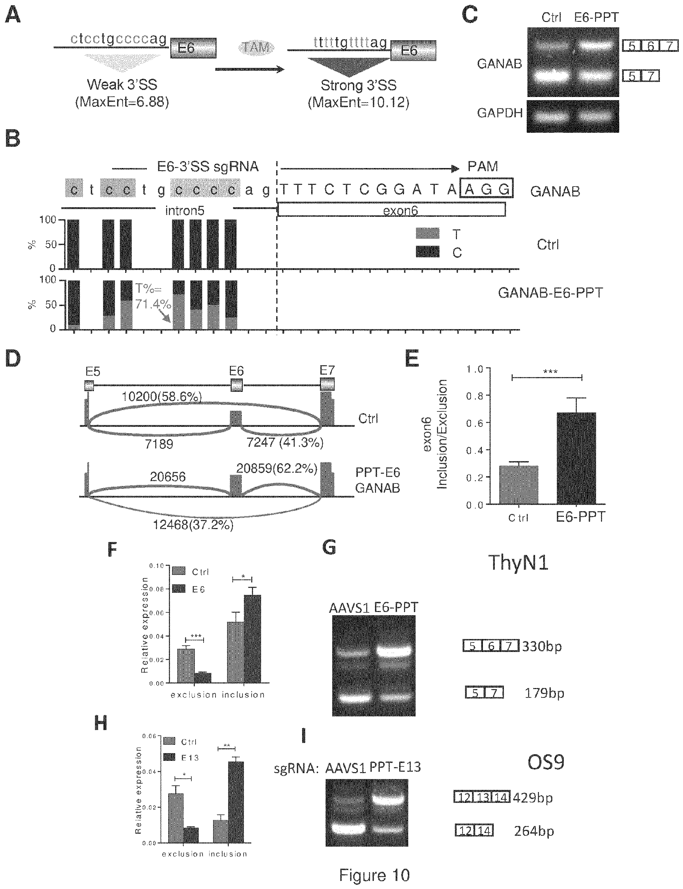

Figure 10: Polypyrimidine Tract (PPT) upstream of GANAB exon 6 converted Cs to Ts to enhance its inclusion. (A) A schematic diagram of directing TAM to convert Cs to Ts at the PPT of GANAB exon 6 to enhance the strength of 3'SS. The polypyrimidine polysaccharide of GANAB exon 6 contains multiple Cs (left) and converting these Cs to Ts (right) increased the strength of this 3'SS (from 6.88 to 10.12) and enhanced the inclusion of exon 6. (B) TAM converted the PPT of GnAB exon 6 to Ts. 293T cells were transfected with the expression plasmid of AIDx-nCas9-Ugi and control sgRNA (Ctrl) or the sgRNA targeting the PPT of GANAB exon 6 (PPT-E6 GANAB). Six days after transfection, sgRNA targeting regions were amplified from genomic DNA and analyzed by high-throughput sequencing with over 8000x coverage. The base composition of nucleotides having detectable mutations (>0.1%) is depicted. The locations of the sgRNA and PAM sequences are shown on the top of the junction sequence. Intron/exon junctions are depicted using dashed lines. The data are representative of two independent experiments. (C, D, E) TAM enhanced the inclusion of the sixth exon in GANAB. (C) As in B, the splicing junctions were amplified from cDNA and analyzed by high-throughput sequencing. The Figure shows the coverage and percentage of each splicing junction of the cells treated with control sgRNA (top panel) or PPT-E6 GANAB sgRNA (bottom panel). The count and percentage (in parentheses) of the junction readings are depicted on the top of each junction arc. (D, E) Analysis of GANAB mRNA splicing by RT-PCR (D) or isoform-specific real-time PCR (E). The data are representative (C, D) or summary (E) of two independent experiments. (F, G) TAM promoted the inclusion of the sixth exon in ThyN1. (H, I) TAM enhanced the inclusion of the 13th exon in OS9.

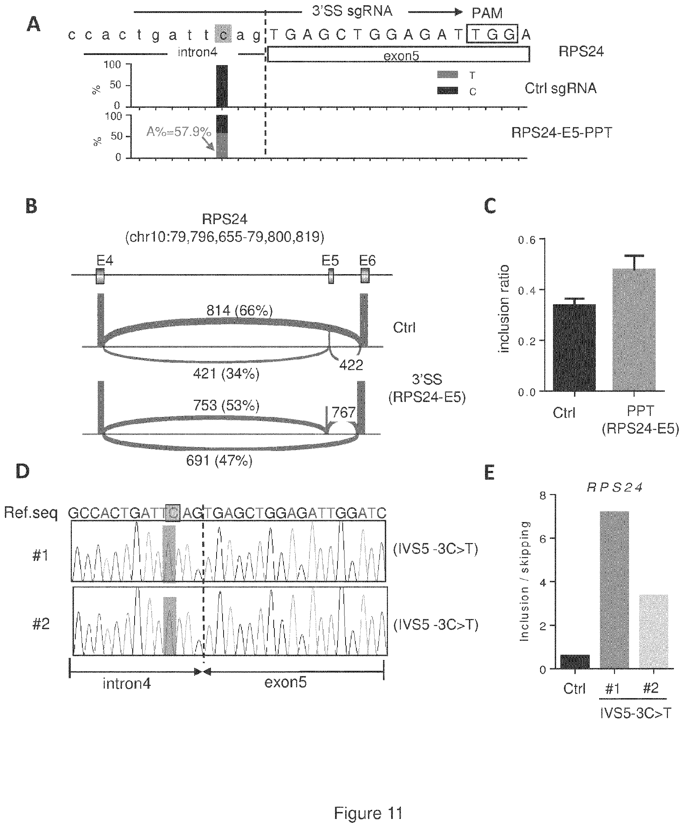

Figure 11: Polypyrimidine Tract (PPT) upstream of RPS24 exon 5 converted C to T to enhance its inclusion. (A) TAM converted C to T at the PPT of exon 5 of RPS24. 293T cells were transfected with expression plasmid(s) of AIDx-nCas9-Ugi and sgRNA targeting AAVS1 (Ctrl) or polypyrimidine nucleoside of the fifth exon in RPS24 (PPT- E5RPS25). Six days after transfection, sgRNA targeting regions were amplified from genomic DNA and analyzed by high-throughput sequencing with over 8000x coverage. The percentage of each cytosine having a detectable mutation (>0.1%) is depicted, and the data are representative of two independent experiments. (B, C) As in A, TAM enhanced the inclusion of the fifth exon of RPS24. RPS24 mRNA splicing was analyzed by high-throughput sequencing for junctions amplified from cDNA (B) or isoform-specific real-time PCR (C). (D, E) Conversion of PPT from C to T increased the content of exon 6 of RPS24. Two single-cell clones were derived from TAM-treated cells and analyzed by Sanger sequencing (D). The right shows the genotype of the cloned cells. (E) The content of RPS24 exon 6 was determined by isoform-specific real-time PCR. The data are representative (A, B, D) or summary (C, E) of two independent experiments.

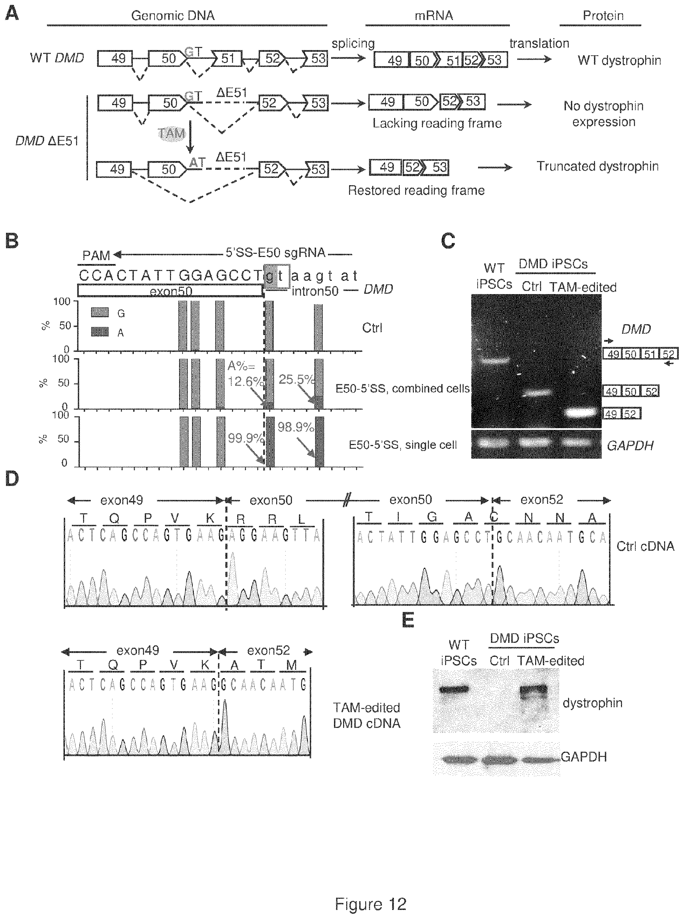

Figure 12: TAM was used to induce exon skipping, repair reading frame of the DMD gene, and restore expression of dystrophin (DMD) in cells of a Duchenne muscular dystrophy patient. (A) A schematic diagram of directing TAM to convert G at 5'SS of DMD exon 50 to A, and restore the expression of dystrophin protein in the patient's cells. Compared with WT cells (top panel), the patient lost exon 51 due to a genetic mutation, resulting in a damage to the reading frame of dystrophin and complete loss of dystrophin (middle panel); a GU>AU mutation at the 5'SS of exon 50 by TAM led to skipping of exon 50 in pateint's cells and restored the reading frame and expression of dystrophin. (B) After treating iPSC cells of the Duchenne muscular dystrophy patient with control sgRNA (ctrl) or targeting sgRNA (E50-5'SS), the corresponding DNA was amplified by PCR, and the induced mutations were analyzed by high-throughput sequencing. The data are representative of two independent experiments. (C, D) Normal human-derived iPSCs, patient-derived iPSCs, and repaired patient-derived iPSCs were differentiated into cardiomyocytes, and DMD gene expression was detected by RT-PCR (C) or western blot (D), respectively. (E) The repaired cells precisely spliced exons 49 and 52.

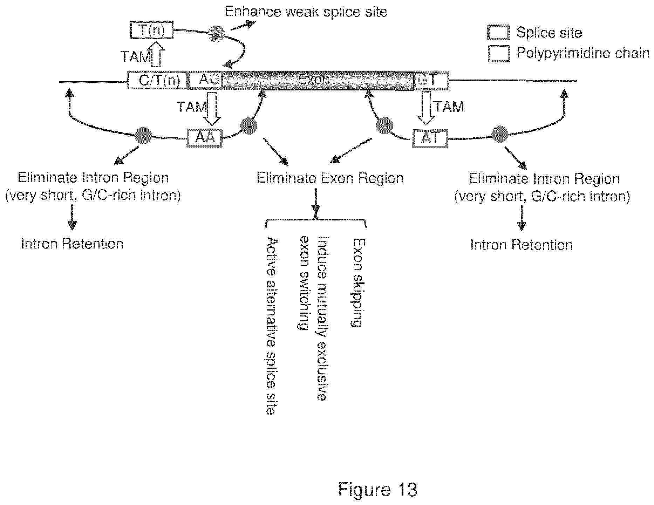

Figure 13: A schematic diagram of using TAM technology to regulate RNA splicing. Using TAM technology to mutate GT to AT at the 5' splice site of an intron can induce exon skipping, activate alternative splice sites, induce mutually exclusive exon switching or intron retention; to mutate AG to AA at the 3' splice site of an intron can also induce exon skipping, activate alternative splice sites, induce mutually exclusive exon switching or intron retention; to mutate C to T in the pyrimidine region at the 3' end of an intron can enhance weak splice sites, thereby enhancing exon inclusion.

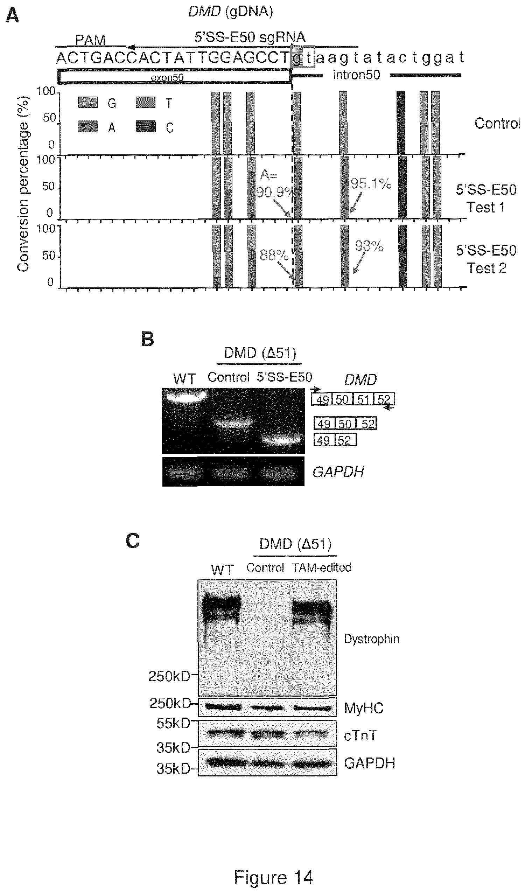

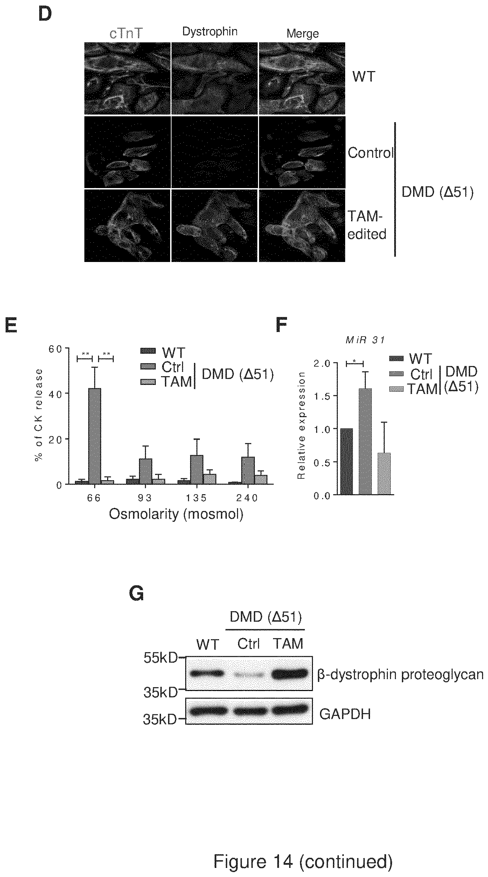

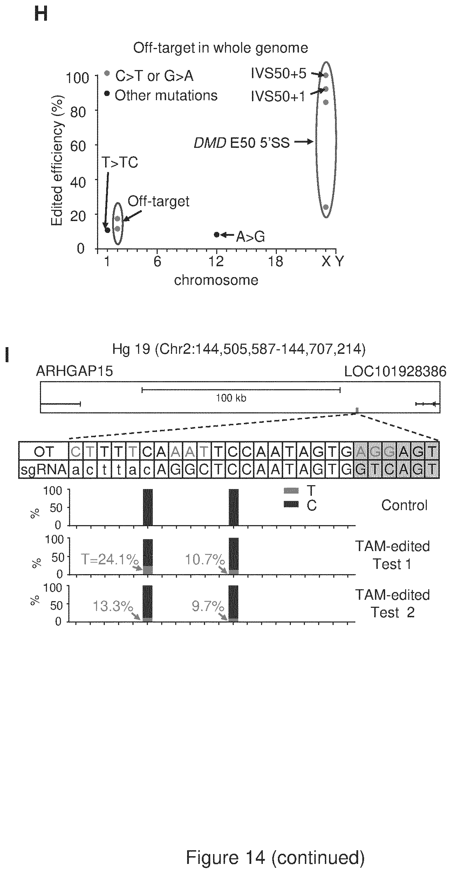

Figure 14: TAM was used to induce exon skipping, repair reading frame of the DMD gene, and restore expression of dystrophin (DMD) in cells of Duchenne muscular dystrophy patients.

### DETAILED DESCRIPTION

It should be understood that, within the scope of the present disclosure, the above technical features of the present disclosure and the technical features specifically described in the following (e.g., Examples) can be combined with each other, thereby forming preferred technical solution(s).

In this disclosure, by generating a point mutation in a cell, especially by mutating the 3' splice site AG of an intron of interest of a gene of interest in the cell to AA, or mutating the 5' splice site GT of an intron of interest of a gene of interest in the cell to AT, or mutating the multiple Cs (for example, 2-10) in the polypyrimidine region of an intron of interest of a gene of interest in the cell to Ts, RNA splicing of the gene of interest in the cell can be regulated, so that to induce exon skipping, activate alternative splice sites, induce mutually exclusive exon switching, induce intron retention or enhance exon inclusion. "Regulating" herein means to change the conventional splicing manner of the RNA.

The present disclosure can be implemented using targeting cytosine deaminase. In this disclosure, the targeting cytosine deaminase is constructed by fusing cytosine deaminase with a protein with a targeting effect.

As used herein, cytosine deaminase refers to various enzymes with cytosine deaminase activity, including but not limited to enzymes of the APOBEC family, such as APOBEC-2, AID, APOBEC-3A, APOBEC-3B, APOBEC-3C, APOBEC-3DE, APOBEC-3G, APOBEC-3F, APOBEC-3H, APOBEC4, APOBEC1 and pmCDA1. The cytosine deaminase suitable for use herein can be derived from any species, preferably mammalian, especially human cytosine deaminase. It is preferred that the cytosine deaminase suitable for use herein is an activated cytosine deaminase, such as a human-derived activated cytosine deaminase. The cytosine deaminases of the APOBEC family are RNA editing enzymes with a nuclear localization signal at the N-terminus and a nuclear export signal at the C-terminus. The catalytic domain of these enzymes is shared by the APOBEC family. Generally, the N-terminal structure is considered necessary for somatic hypermutation (SHM). The function of cytosine deaminases is to deaminate cytosine and transform cytosine into uracil, and then DNA repairing can transform uracil into other bases. It should be understood that the cytosine deaminases well known in the art or fragments or mutants thereof that retain the biological activity of deaminating cytosine and converting cytosine into uracil can be used herein.

In certain embodiments, AID is used herein as the cytosine deaminase in the targeting cytosine deaminase. Amino acid residues 9-26 of AID are nuclear localization (NLS) domain, especially amino acid residues 13-26, which are involved in DNA binding; amino acid residues 56-94 are catalytic domain; amino acid residues 109-182 are APOBEC-like domain; amino acid residues 193-198 are nuclear export (NES) domain; amino acid residues 39-42 interact with catenin-like protein 1 (CTNNBL1); and amino acid residues 113-123 are hotspot recognition loop.

The full-length AID (as shown in SEQ ID NO: 25, amino acids 1457-1654), or a fragment of AID can be used in this disclosure. Preferably, the fragment includes at least the NLS domain, the catalytic domain, and the APOBEC-like domain. Therefore, in certain embodiments, the fragment comprises at least amino acid residues 9-182 of AID (i.e., amino acid residues 1465-1638 of SEQ ID NO: 25). In other embodiments, the fragment comprises at least amino acid residues 1-182 of AID (i.e., amino acid residues 1457-1638 of SEQ ID NO: 25). For example, in certain embodiments, the AID fragment used herein consists of amino acid residues 1-182, amino acid residues 1-186, or amino acid residues 1-190. Therefore, in certain embodiments, the AID fragment used herein consists of amino acid residues 1457-1638 of SEQ ID NO: 25, amino acid residues 1457-1642 of SEQ ID NO: 25, or amino acid residues 1457-1646 of SEQ ID NO: 25.

A variant of AID that retains its cytosine deaminase activity (i.e., the biological activity of deaminating cytosine and converting cytosine into uracil) can also be used herein. For example, such variants may have 1-10, such as 1-8, 1-5, or 1-3 amino acid variations, including amino acid deletions, substitutions, and mutations, with respect to the sequence of the wild-type AID. Preferably, these amino acid variations do not present in the above-mentioned NLS domain, catalytic domain, or APOBEC-like domain, or even if they occur in these domains, they do not affect the original biological functions of these domains. For example, it is preferable that these variations do not occur at the amino acid residue 24, 27, 38, 56, 58, 87, 90, 112, 140 of the AID amino acid sequence. In certain embodiments, these variations also do not occur within amino acids 39-42, amino acids 113-123. Thus, for example, variations can occur in amino acids 1-8, amino acids 28-37, amino acids 43-55 and/or amino acids 183-198. In certain embodiments, variations occur at amino acids 10, 82, and 156. For example, substitutions occur at amino acids 10, 82, and 156, which may be K10E, T82I, and E156G In these embodiments, the amino acid sequence of the exemplary AID mutant contains or consists of the amino acid sequence shown as residues 1447-1629 of SEQ ID NO: 31. Examples of other AIDs, fragments or mutants thereof can refer to CN201710451424.3, the entire contents of which are incorporated herein by reference.

Herein, the protein with a targeting effect may be a protein known in the art that can target a gene of interest in the cell genome, including but not limited to a TALEN protein that specifically recognizes the target sequence, a zinc finger protein that recognizes the target sequence by mutation, an Ago protein, a Cpf enzyme and a Cas enzyme. This disclosure can be implemented using TALEN proteins, zinc finger proteins, Ago proteins, and Cpf enzymes and Cas enzymes, which are well known in the art.

Therefore, in certain embodiments, the targeting cytosine deaminase suitable for use herein may be selected from the group consisting of:
(1) a fusion protein of a cytosine deaminase, or a fragment or mutant thereof retaining enzyme activity, and a Cas enzyme with helicase activity and partial or no nuclease activity;
(2) a fusion protein of a cytosine deaminase, or a fragment or mutant thereof retaining enzyme activity, and a TALEN protein that specifically recognizes a target sequence;
(3) a fusion protein of a cytosine deaminase, or a fragment or mutant thereof retaining enzyme activity, and a zinc finger protein that specifically recognizes a target sequence;
(4) a fusion protein of a cytosine deaminase, or a fragment or mutant thereof retaining enzyme activity, and a Cpf enzyme with helicase activity and partial or no nuclease activity; and
(5) a fusion protein of a cytosine deaminase, or a fragment or mutant thereof retaining enzyme activity, and a Ago protein.

When Cpf enzymes are used, it is preferable to use a Cpf enzyme in which nuclease activity is partially or completely absent but helicase activity retains. The Cpf enzyme, under the guidance of its recognized sgRNA, binds to the specific DNA sequence, allowing the cytosine deaminase fused thereto to perform the mutations described herein. The Ago protein needs to bind to the specific DNA sequence under the guidance of its recognized gDNA.

In certain embodiments, the targeting cytosine deaminase AID-mediated gene mutation technology (TAM) is used herein to mutate guanine to adenine at the splice site of the intron, specifically block the exon recognition process, and regulate the alternative splicing process of endogenous mRNA. The TAM technique herein uses a fusion protein of a Cas protein lacking nuclease activity and cytosine deaminase AID, an active fragment or a mutant thereof. Under the guidance of sgRNA, the fusion protein is recruited to the specific DNA sequence, wherein AID, active fragments or mutants thereof mutates guanine (G) into adenine (A), or mutates cytosine (C) into thymine (T).

CRISPR (Clustered Regularly Interspaced Short Palindromic Repeats) is a gene editing system of bacteria to resist viruses or evade mammalian immune responses. The system has been modified and optimized, and has been widely used in *in vitro* biochemical reactions, gene editing of cells and individuals. Generally, the complex formed by the Cas protein with endonuclease activity (also called Cas enzyme) and its specifically recognized sgRNA is complementary paired with the template strand in the target DNA through the matching region (i.e., target binding region) of the sgRNA, and the double-stranded DNA is cut at a specific location by Cas. The above-mentioned characteristics of Cas/sgRNA are used in this disclosure, that is, the Cas is localized to the desired location through the specific binding of the sgRNA to the target, where the AID or its active fragment or mutant in the fusion protein mutates guanine (G) to adenine (A), or cytosine (C) to thymine (T).

The Cas protein suitable for this disclosure having helicase activity and partial (only having DNA single-strand break ability) or no nuclease activity (no DNA double-strand break ability), especially those having helicase activity and partial or no endonuclease activity, can be derived from various Cas proteins well known in the art and variants thereof, including but not limited to Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpf1, their homologues or modified variants.

In some embodiments, a Cas9 enzyme lacking nuclease activity toghether with its specifically recognized single-stranded sgRNA are used. Cas9 enzymes may be Cas9 enzymes from different species, including but not limited to Cas9 from *Streptococcus pyogenes* (SpCas9), Cas9 from *Staphylococcus aureus* (SaCas9), and Cas9 from *Streptococcus thermophilus* (St1Cas9), etc. Various variants of the Cas9 enzyme can be used, provided that the Cas9 enzyme can specifically recognize its sgRNA and lack nuclease activity.

Cas proteins lacking nuclease activity can be prepared by methods well known in the art. These methods include, but are not limited to, deleting the entire catalytic domain of the endonuclease in the Cas proteins, or mutating one or several amino acids in the catalytic domain, thereby producing Cas proteins lacking nuclease activity. The mutation may be deletion or substitution of one or several (for example, 2 or more, 3 or more, 4 or more, 5 or more, 10 or more to the entire catalytic domain) amino acid residues, or insertion of one or several (e.g., 1 or more, 2 or more, 3 or more, 4 or more, 5 or more, 10 or more, or 1-10, 1-15) new amino acids residues. Conventional methods in the art can be used to perform the above deletion of the domain or mutation of amino acid residue, and to detect whether the mutated Cas protein has nuclease activity. For example, for Cas9, the two endonuclease catalytic domains RuvC1 and HNH can be mutated separately, e.g., the amino acid 10 asparagine of the enzyme (in RuvC1 domain) is mutated to alanine or other amino acids, the amino acid 841 histidine (in HNH domain) is mutated to alanine or other amino acids. These two mutations make Cas9 lose endonuclease activity. Preferably, the Cas enzyme has no nuclease activity at all. In one or more embodiments, the amino acid sequence of the nuclease-activity-free Cas9 enzyme used herein is shown as residues 42-1452 of SEQ ID NO: 25. In other embodiments, the Cas enzyme used herein partially lacks nuclease activity, i.e., the Cas enzyme can cause DNA single-strand breaks. A representative example of such Cas enzymes can be shown as amino acid residues 42-1419 of SEQ ID NO: 33. In other embodiments, the amino acid sequence of the Cas enzyme used herein is shown as residues 199-1566 of SEQ ID NO: 23, or is shown as residues 199-1262 of SEQ ID NO: 50. Examples of other Cas enzymes can refer to CN201710451424.3, the entire contents of which are incorporated herein by reference.

The Cas/sgRNA complex's function requires a protospacer adjacent motif (PAM) in the non-template strand (3' to 5') of the DNA. The corresponding PAMs of different Cas enzymes are not exactly the same. For example, generally, the PAM for SpCas9 is NGG (SEQ ID NO: 34); the PAM for SaCas9 is NNGRR (SEQ ID NO: 35); the PAM for St1Cas9 is NNAGAA (SEQ ID NO: 36); wherein N is A, C, T or G, and R is G or A.

In certain preferred embodiments, the PAM for SaCas9 is NNGRRT (SEQ ID NO: 37). In certain preferred embodiments, the PAM for SpCas9 is TGG (SEQ ID NO: 38); in certain preferred embodiments, the PAM for SaCas9 enzyme KKH mutant is NNNRRT (SEQ ID NO: 39); wherein, N is A, C, T or G, and R is G or A.

Generally, sgRNA contains two parts: target binding region and protein recognition region (such as Cas enzyme recognition region or Cpf enzyme recognition region). The target binding region and the protein recognition region are usually connected in a 5' to 3' direction. The length of the target binding region is usually 15 to 25 bases, more usually 18 to 22 bases, such as 20 bases. The target binding region specifically binds to the template strand of DNA, thereby recruiting the fusion protein to a predetermined site. Generally, the opposite complementary region of the sgRNA binding region on the DNA template strand is immediately adjacent to PAM, or separated from PAM by several bases (for example, within 10, or within 8, or within 5 bases). Therefore, when designing sgRNA, the enzyme's PAM is determined according to the used splicing enzyme (such as Cas enzyme), and then the non-template strand of DNA is searched for a site that can be used as PAM, and then a fragment of 15bp-20bp in length, more usually 18bp-22bp in length, which is downstream from the PAM site of the non-template strand (3' to 5') and immediately adjacent to the PAM site or separated from the PAM site within 10bp (e.g., within 8bp or 5bp) serves as the sequence of the target binding region of sgRNA. The protein recognition region of sgRNA is determined according to the used splicing enzyme, which is known by those skilled in the art.

Therefore, the sequence of the target binding region of the sgRNA herein comprises the fragment of 15bp-20bp in length, more usually 18bp-22bp in length, downstream from the PAM site recognized by the selected splicing enzyme (such as Cas enzyme or Cpf enzyme) and immediately adjacent to the PAM site or separated from the PAM site within 10bp (e.g., within 8bp or 5bp); its protein recognition region is specifically recognized by the selected splicing enzyme.

Given that the purpose of this disclosure is to mutate guanine to adenine at the intron splice site, or mutate C to T in the polypyrimidine strand upstream of the 3' splice site, it should be considered whether a PAM sequence is present near the splice site, and the distance between the PAM sequence and the splice site(s), when designing an sgRNA for this disclosure. Therefore, in general, the sgRNA binds to the sequence containing the splice site(s) of the intron of interest of the gene of interest, or to the complementary sequence of the polypyrimidine region of interest. Alternatively, the target binding region of the sgRNA contains the complementary sequence of the splice site(s) of the intron of interest of the gene of interest, or contains the sequence of the polypyrimidine region of the intron of interest of the gene of interest.

The sgRNA can be prepared by conventional methods in the art, for example, synthesized by conventional chemical synthesis methods. The sgRNA can also be transferred into cells via an expression vector, and expressed in the cells; or it can be introduced into animals/humans via adeno-associated viruses. The expression vector of the sgRNA can be constructed using methods well known in the art.

In certain embodiments, sgRNA sequences or complementary sequences thereof are also provided herein, which include a target binding region and a protein recognition region, wherein the target binding region binds to a sequence containing a splice site of the intron of interest of the gene of interest, or to a complementary sequence of the polypyrimidine region of interest. Generally, the target binding region is 15-25bp in length, such as 18-22bp, preferably 20bp. In certain embodiments, the target binding region of the sgRNA binds to the sequence in DMD exon 50 having the 3' splice site; preferably, the target binding region of the sgRNA is as shown in SEQ ID NO: 17 or 51.

The targeting cytosine deaminase used herein is preferably a fusion protein of the aforementioned Cas enzyme and the aforementioned AID or fragments or mutants thereof. The Cas enzyme is usually at the N-terminus of the amino acid sequence of the fusion protein, and the AID or its fragment or mutant is at the C-terminus. Of course, the AID or its fragment or mutant can be at the N-terminus of the amino acid sequence of the fusion protein, and the Cas enzyme is at the C-terminus. In certain embodiments, provided herein are fusion proteins substantially formed by a Cas enzyme and AID or a fragment or mutant thereof. It should be understood that the fusion protein "substantially formed by ..." or similar references herein does not indicate that the fusion protein only contains Cas enzyme and AID or its fragment or mutant thereof. The phrase should be understood that the fusion protein can only contain Cas enzyme and AID or its fragment or mutant thereof, or the fusion protein can further contain other parts that do not affect the targeting effect of the Cas enzyme and the function to mutate target sequence(s) by AID or its fragment or mutant thereof in the fusion protein. Said other parts include but are not limited to various linker sequences, nuclear localization sequences, Ugi sequences, and amino acid sequences introduced into the fusion protein due to gene cloning process and/or to construct the fusion protein, to promote expression of the recombinant proteins, to obtain the recombinant proteins automatically secreted from the host cells, or to facilitate the detection and/or purification of the recombinant proteins, as described below.

Cas enzymes can be fused to AID or fragments or mutants thereof via linkers. The linker may be a peptide of 3 to 25 residues, for example, a peptide of 3 to 15, 5 to 15, 10 to 20 residues. Suitable examples of the peptide linkers are well known in the art. Generally, a linker contains one or more motifs that repeat in sequence, which usually contain Gly and/or Ser. For example, the motif may be SGGS (SEQ ID NO: 40), GSSGS (SEQ ID NO: 41), GGGS (SEQ ID NO: 42), GGGGS (SEQ ID NO: 43), SSSSG (SEQ ID NO: 44), GSGSA (SEQ ID NO: 45) and GGSGG (SEQ ID NO: 46). Preferably, the motifs are adjacent to each other in the linker sequence, with no amino acid residue inserted between the repeated motifs. The linker sequence may comprise or consist of 1, 2, 3, 4 or 5 repeated motifs. In certain embodiments, the linker sequence is a polyglycine linker sequence. The number of glycine in the linker sequence is not particularly limited, but is usually 2-20, such as 2-15, 2-10, 2-8. In addition to glycine and serine, the linker can also contain other known amino acid residues, such as alanine (A), leucine (L), threonine (T), glutamic acid (E), phenylalanine (F), arginine (R), glutamine (Q), etc. In certain embodiments, the linker sequence is XTEN, and its amino acid sequence is shown as amino acid residues 183-198 of SEQ ID NO:29. Other exemplary linker sequences can be the linker sequences described in CN201710451424.3, such as SEQ ID NO: 21-31 described therein.

It should be understood that it is often necessary to add appropriate restriction site(s) during the gene cloning process, which will inevitably introduce one or more irrelevant residues at the end(s) of the expressed amino acid sequence(s), while not affect the activity of the obtained sequence. In order to construct the fusion protein, promote the expression of the recombinant protein, obtain the recombinant proteins automatically secreted from the host cells, or facilitate the purification of the recombinant proteins, it is often necessary to add amino acid(s) to the N-terminus, C-terminus, or within other suitable regions of the recombinant protein, and the added amino acid(s) include but are not limited to suitable linker peptides, signal peptides, leader peptides, terminally extended amino acid(s), etc. Therefore, the N-terminus or C-terminus of the fusion protein herein may futher contains one or more polypeptide fragments as protein labels. Any suitable label can be used for this disclosure. For example, the labels may be FLAG, HA, HA1, c-Myc, Poly-His, Poly-Arg, Strep-TagII, AU1, EE, T7, 4A6, ε, B, gE, and Ty1. These labels can be used to purify proteins.

The fusion protein herein may also contain a nuclear localization sequence (NLS). Nucleus localization sequences known in the art derived from various sources and with various amino acid compositions can be used. Such nuclear localization sequences include, but are not limited to: NLS from SV40 virus large T antigen; NLS from nucleoplasmic proteins, for example, nucleoplasmic protein bipartite NLS; NLS from c-myc; NLS from hRNPA1M9; sequences from IBB domain of importin-a; sequences from myoma T protein; sequences from mouse c-ab1IV; sequences from influenza virus NS1; sequences from hepatitis virus δ antigen; sequences from mouse Mx1 protein; sequences from human poly(ADP-ribose) polymerase; and sequences from steroid hormone receptor (human) glucocorticoid; etc. The amino acid sequences of these NLS sequences can be found in CN201710451424.3 as SEQ ID NO: 33-47. In certain specific embodiments, the sequence shown by amino acid residues 26-33 of SEQ ID NO: 25 is used herein as NLS. The NLS can be located at the N-terminus, C-terminus of the fusion protein; it can also be located within the sequence of the fusion protein, such as located at the N-terminus and/or C-terminus of the Cas9 enzyme in the fusion protein, or located at the N-terminal and/or C-terminal of the AID or its fragment or mutant in the fusion protein.

The accumulation of the fusion protein disclosed herein in the nucleus can be detected by any suitable technique. For example, detection labels can be fused to the Cas enzyme so that the location of the fusion proteins within cells can be visualized when combined with methods of detecting nucleus location (e.g., a dye specific to the nucleus, such as DAPI). In some embodiments, 3*flag is used as a label herein, and the peptide sequence may be amino acid residues 1 to 23 of SEQ ID NO:25. It should be understood that, generally, if a label sequence is used, the label sequence is at the N-terminus of the fusion protein. The label sequence can be directly connected to NLS, or may be connected via an appropriate linker sequence. The NLS sequence may be directly connected to the Cas enzyme or AID or its fragment or mutant, or it may be connected to the Cas enzyme or AID or its fragment or mutant through an appropriate linker sequence.

Therefore, in certain embodiments, the fusion protein herein consists of a Cas enzyme and a AID or its fragment or mutant. In other embodiments, the fusion protein herein is formed by connection of a Cas enzyme to a AID or its fragment or mutant via a linker. In certain embodiments, the fusion protein herein consists of a NLS, a Cas enzyme, a AID or its fragment or mutant, and optionally a linker sequence between the Cas enzyme and the AID or its fragment or mutant. In certain embodiments, in addition to the NLS, Cas enzyme and AID or a fragment or mutate thereof, the fusion protein herein may also contain a phage protein, such as UGI as an UNG inhibitor. The amino acid sequence of an exemplary UGI may be amino acid residues 1576-1659 of SEQ ID NO: 23 of the present disclosure. Therefore, in certain embodiments, the fusion protein herein contains the Cas9 enzyme described herein, the AID or a fragment or mutant thereof, UGI and NLS described herein, or consists of these parts, optional linker(s) between them and optional amino acid sequence(s) for detection, isolation or purification. The Ugi sequence may be located at the N-terminus, C-terminus of the fusion protein, or within the fusion protein, for example, located between the NLS sequence and the Cas enzyme or between the Cas enzyme and the AID or a fragment or mutant thereof. In certain embodiments, the fusion protein herein contains or consists of, from the N-terminus to the C-terminus, AID or a fragment or mutant thereof, Cas enzyme, Ugi and NLS, or contains or consists of, from the N-terminus to the C-terminus, Cas enzyme, AID or a fragment or mutant thereof, Ugi and NLS; they can be connected by linker(s).

In certain embodiments, the fusion proteins disclosed in CN 201710451424.3 are used herein. More specifically, the amino acid sequence of the used fusion protein disclosed in this disclosure is SEQ ID NO: 25, 27, 29, 31, 33, 48, or 50, or amino acids 26-1654 of SEQ ID NO: 25, or amino acids 26-1638 of SEQ ID NO: 27, or amino acids 26-1629 of SEQ ID NO: 31, or amino acids 26-1638 of SEQ ID NO: 33, or amino acids 26-1629 of SEQ ID NO: 48. In certain embodiments, the fusion protein herein is shown by SEQ ID NO: 23 of the present disclosure.

An expression vector/plasmid expressing the above fusion protein and a vector/plasmid expressing the desired sgRNA can be constructed and transferred into cells of interest to regulate their RNA splicing by inducing mutations at the splice site(s) of the gene of interest.

The "expression vector" may be various bacterial plasmids, bacteriophages, yeast plasmids, plant cell viruses, mammalian cell viruses such as adenovirus, retrovirus, or other vectors well known in the art. Any plasmid or vector can be used, provided that it can replicate and be stable in the host. An important charecterastic of an expression vector is that it usually contains an origin of replication, a promoter, a marker gene and a translation control element. The expression vector may also include a ribosome binding site for translation initiation and a transcription terminator. The polynucleotide sequences described herein are operably linked to appropriate promoters in the expression vectors, so that mRNA synthesis is directed by the promoters. Representative examples of these promoters are: the lac or trp promoter of *E.coli;* the PL promoter of phage λ; eukaryotic promoters including the CMV immediate early promoter, the HSV thymidine kinase promoter, the early and late SV40 promoters, LTRs of retroviruses, and other known promoters that can control gene expression in prokaryotic or eukaryotic cells or their viruses. Marker genes can provide phenotypic traits for selection of transformed host cells, including but not limited to dihydrofolate reductase, neomycin resistance, and green fluorescent protein (GFP) for eukaryotic cell, or tetracycline or ampicillin resistance for *E.coli.* When the polynucleotides described herein are expressed in higher eukaryotic cells, transcription will be enhanced if an enhancer sequence is inserted into the vector. Enhancers are cis-acting factors of DNA, usually are about 10bp to 300bp, which act on the promoter to enhance gene transcription.

Those skilled in the art know how to select appropriate vectors, promoters, enhancers and host cells. Methods well known to those skilled in the art can be used to construct expression vectors containing the polynucleotide sequences described herein and appropriate transcription/translation control signals. These methods include *in vitro* recombinant DNA technology, DNA synthesis technology, *in vivo* recombinant technology and so on.

The fusion protein herein, its coding sequence or expression vector, and/or the sgRNA, its coding sequence or expression vector may be provided in the form of a composition. For example, the composition may contain the fusion protein herein and the sgRNA or the vector expressing the sgRNA, or may contain the vector expressing the fusion protein herein and the sgRNA or the vector expressing the sgRNA. In the composition, the fusion protein or its expression vector, or sgRNA or its expression vector may be provided as a mixture, or may be packaged separately. The composition may be in the form of a solution or a lyophilized form. Preferably, the fusion protein in the composition is a fusion protein of the AID or a fragment or mutant thereof described herein and the Cas enzyme described herein.

The composition may be provided in a kit. Accordingly, provided herein are kits containing the compositions described herein. Alternatively, provided herein is a kit containing the fusion protein herein and the sgRNA or the vector expressing the sgRNA, or containing the vector expressing the fusion protein herein and the sgRNA or the vector expressing the sgRNA. In the kit, the fusion protein or its expression vector, or sgRNA or its expression vector may be packaged separately, or may be provided as a mixture. The kit may further include, for example, reagents for transferring into cells the fusion protein or its expression vector and/or sgRNA or its expression vector, and instructions for the transfer. Alternatively, the kit may also include instructions for implementing the various methods and uses described herein using the ingredients contained in the kit. The kit also includes other reagents, such as reagents for PCR.

The fusion protein herein, its coding sequence or expression vector, and/or the sgRNA or its expression vector can be used to induce base mutations at a splice site of the gene of interest to regulate its RNA splicing. Therefore, provided herein is a method for inducing base mutation in a splice site of a gene of interest in a cell of interest, wherein the method comprises the step of expressing the fusion protein described herein in the cell, the method also comprises the step of expressing sgRNAs or gDNAs based on the expressed fusion protein. For example, in certain embodiments, the fusion protein described herein of the AID or a fragment or mutant thereof and the Cas enzyme, together with its recognized sgRNA, are expressed in cells. In certain embodiments, the fusion protein of a cytosine deaminase, or a fragment or mutant thereof retaining enzyme activity, and a TALEN protein that specifically recognizes a target sequence is expressed in cells. In certain embodiments, the fusion protein of a cytosine deaminase, or a fragment or mutant thereof retaining enzyme activity, and a zinc finger protein that specifically recognizes a target sequence is expressed in cells. In certain embodiments, the fusion protein of a cytosine deaminase or a fragment or mutant thereof retaining enzyme activity and a Cpf enzyme with helicase activity and partial or no nuclease activity, together with the sgRNA recognized by the Cpf enzyme, are expressed in cells. In other embodiments, the fusion protein of a cytosine deaminase or a fragment or mutant thereof retaining enzyme activity and an Ago protein, together with the gDNA recognized by the Ago protein, are expressed in cells.

In this disclosure, cells of interest especially also include those in which a splice site of a gene of interest needs to be mutated to regulate its RNA splicing. Such cells include prokaryotic cells and eukaryotic cells, such as plant cells, animal cells, microbial cells, and the like. Especially preferred are animal cells, such as mammalian cells, rodent cells, including cells of humans, horses, cattles, sheeps, mice, rabbits, and the like. Microbial cells include cells from various microbial species that are well known in the art, especially cells from microbial species valuable in medical research and production (e.g., production of fuel such as ethanol, protein, and oil such as DHA). The cells may also be cells from various organs, such as cells from human liver, kidney, or skin, etc, or may be blood cells. The cells may also be various mature cell lines that are commercially available, such as 293 cells, COS cells. In some embodiments, the cells are those from healthy individuals; in other embodiments, the cells are those from diseased tissues of diseased individuals, such as cells from inflammatory tissues, or tumor cells. In certain embodiments, the cells of interest are induced pluripotent stem cells. Cells can be those genetically engineered to have a specific function (e.g., to produce a protein of interest) or to generate a phenotype of interest. It should be understood that cells of interest include somatic cells and germ cells. In certain embodiments, the cells are specific cells in animals or humans.

The genes of interest may be any nucleic acid sequences of interest, especially various genes or nucleic acid sequences related to diseases, or related to the production of various proteins of interest, or related to biological functions of interest. Such genes or nucleic acid sequences of interest include, but are not limited to, nucleic acid sequences encoding various functional proteins. Herein, a functional protein refers to a protein capable of achieving the physiological function of an organism, including a catalytic protein, a transport protein, an immune protein, and a regulatory protein. In certain specific embodiments, the functional proteins include, but are not limited to: proteins involved in the occurrence, development and metastasis of diseases, proteins involved in cell differentiation, proliferation and apoptosis, proteins involved in metabolism, development-related proteins, and various medicinal targets, etc. For example, functional proteins may be antibodies, enzymes, lipoproteins, hormone-like proteins, transport and storage proteins, kinetic proteins, receptor proteins, membrane proteins, and the like.

As illustrative examples, genes of interest include but are not limited to RPS24, CD45, DMD, PKM, BAP1, TP53, STAT3, GANAB, ThyN1, OS9, SMN2, β-hemoglobin gene, LMNA, MDM4, Bcl2, and LRP8, etc.

In certain embodiments, the methods described herein include transferring the fusion protein or its expression vector and its recognized sgRNA or expression vector thereof or gDNA or expression vector thereof into the cell. In the case where the cell constitutively expresses the fusion protein described herein, the corresponding sgRNA or expression vector thereof or its recognized gDNA or expression vector thereof can be transferred into the cell alone. In the case where the cell inducibly expresses the fusion protein described herein, after being transfered with the sgRNA or gDNA, the cell can also be incubated with an inducing agent, or the cell can be subjected to corresponding induction means (such as lighting). Preferably, the method herein is implemented using the fusion protein of the AID or a fragment or mutant thereof described herein and the Cas enzyme described herein, together with its recognized sgRNA.

Conventional transfection methods can be used to transfer into cells the fusion protein or its expression vector and/or its recognized sgRNA or expression vector thereof or gDNA or expression vector thererof. For example, when the cell of interest is a prokaryotic organism such as *E.coli,* competent cells that can absorb DNAs can be harvested after the exponential growth phase and treated with the CaCl₂ method, which is well known in the art. Another method is to use MgCl₂. If necessary, transformation can also be carried out by electroporation. When the host is a eukaryote, the following DNA transfection methods can be used: the calcium phosphate co-precipitation method, conventional mechanical methods such as microinjection, electroporation, liposome packaging, etc. For example, during transfection, the plasmid DNA-liposome complex is prepared and co-transfected into the cell together with the corresponding sgRNA or gDNA. Commercially available transfection kits or reagents can be used to transfer the vectors or plasmids described herein into cells of interest, such reagents include but are not limited to Lipofectamine® 2000 reagents. After transforming the cells, the obtained transformants can be cultured by conventional methods to express the fusion proteins described herein. According to the used cells, the culture medium can be selected from various conventional culture media.

Generally, for different cells, expression vectors expressing the fusion protein and sgRNA or gDNA of the present disclosure can be designed using known techniques, so that these expression vectors are suitable for expression in the cells. For example, a promoter and other related regulatory sequences that facilitate starting expression in the cell can be provided in the expression vector. These can be selected and implemented by technicians according to actual practice.

For the sgRNA used in this disclosure, the site that suitable as a PAM can be found near the splice site of interest of the gene of interest, and the Cas enzyme that recognizes the PAM can be selected based on the PAM, and then the fusion protein herein containing the Cas enzyme together with its corresponding sgRNA can be designed and prepared as described herein. Therefore, the target recognition region of the sgRNA used herein usually contains the complementary sequence of the splice site(s) of the intron of interest of the gene of interest.

The splice site described herein has a well-known meaning in the art, including 5' splice site and 3' splice site. Herein, both the 5' splice site and the 3'splice site are relative to an intron. Generally, the site that can serve as a PAM is selected near the splice site of the exon/intron of interest of the gene of interest. For example, the exon or intron of interest of the gene of interest may be exon 5 of RPS, exon 5 of CD45, exon 8 or 9 of TP53 gene, exon 9 or 10 of PKM, intron 2 of BAP1 and intron 8 of TP53, etc. Alternatively, in certain embodiments, the site that can serve as a PAM is selected near the polypyrimidine chain present within the intron upstream of the 3' splice site of the gene of interest. Therefore, the target binding region of such sgRNA contains the sequence of the polypyrimidine region of the intron of interest of the gene of interest.

The method herein may be a method *in vitro* or a method *in vivo;* in addition, the method herein includes a method for therapeutic purposes and a method for non-therapeutic purposes. When implemented *in vivo,* the fusion protein herein or its expression vector and its recognized sgRNA or expression vector thereof or gDNA or expression vector thereof can be transferred into the body of the subject, such as corresponding tissue cells, by methods well known in the art. It should be understood that when implemented *in vivo,* the subjects may be humans or various non-human animals, including various non-human model organisms commonly used in the art. Experiments *in vivo* should meet ethical requirements.

The method described herein for inducing base mutations at the splice site of a gene of interest in a cell of interest is a general RNA splicing regulation method that can be used for gene therapy. Accordingly, provided herein is a method for gene therapy, comprising administering to a subject in need a therapeutically effective amount of a vector expressing the fusion protein described herein and a vector expressing the corresponding sgRNA or gDNA. The therapeutically effective amount can be determined according to the age, sex, nature and severity of the disease, etc. Generally, administration of a therapeutically effective amount of the vector should be sufficient to alleviate the symptoms of the disease or cure the disease. The gene therapy can be used for the treatment of diseases caused by genetic mutations, and can also be used for the treatment of diseases in which symptoms of the diseases can be relieved or the diseases can be cured by regulating different splicing isoforms. For example, diseases caused by genetic mutations include but are not limited to: Duchenne myasthenia caused by mutations in the DMD gene, SMN, thalassemia caused by 647G>A mutation of β hemoglobin IVS2, familial hypercholesterolemia and premature aging caused by LMNA mutation, etc. Diseases in which symptoms of the diseases can be relieved or the diseases can be cured by regulating ratio of different splicing isoforms include tumors, the splicing isoforms including but not limited to conversion of Stat3α to Stat3β, conversion of PKM2 to PKM1, MDM4 exon 6 skipping, selection of Bcl2 alternative splice sites, LRP8 exon 8 skipping.

In certain embodiments, provided herein is a method for tumor therapy, comprising administering to a subject in need a therapeutically effective amount of a vector expressing the fusion protein described in any embodiment herein and a vector expressing corresponding sgRNA. In certain embodiments, the target binding region of the sgRNA comprises the complementary sequence of the 3' splice site of Stat3 intron 22. In certain embodiments, the target binding region of sgRNA suitable for the method is shown as SEQ ID NO: 3. Alternatively, the target binding region of the sgRNA comprises the complementary sequence of the 5' or 3' splice site of PKM intron 10. In certain embodiments, the target binding region of sgRNA suitable for the method is shown as SEQ ID NO: 15 or 16.

In certain embodiments, provided herein is a method of treating Duchenne myasthenia due to a DMD gene mutation, the method comprising the step of administering to a subject in need a therapeutically effective amount of a vector expressing the fusion protein described herein and a vector expressing corresponding sgRNA, wherein the target binding region of the sgRNA comprises the complementary sequence of the 5' splice site of DMD exon 50. In certain embodiments, the target binding region of sgRNA suitable for the method is shown as SEQ ID NO: 17 or 51. In certain embodiments, tthe amino acid sequence of the fusion protein suitable for the method is shown as SEQ ID NO: 23 or 50.

The methods for gene therapy described herein can be implemented by means well known in the art. Generally, the routes of administration for gene therapy include routes *ex vivo* and routes *in vivo.* For example, suitable backbone vectors (such as adeno-associated virus vectors) can be used to construct expression vectors expressing the fusion protein described herein and vectors expressing the sgRNA or gDNA, which can be administered to the patient in a general route, such as injection. Alternatively, in the case of blood diseases, blood cells having a gene variation of the subject may be obtained, treated *in vitro* using the method described herein, proliferated *in vitro* after the the variation is eliminated, and then reinfused into the subject. In addition, the methods described herein can also be used to modify pluripotent stem cells of the subject, which are reinfused into the subject to achieve therapeutic purposes.

In yet another aspect of the present disclosure, provided herein is use of the fusion protein, its coding sequence and/or expression vector, and/or sgRNA and/or its expression vector according to any of the embodiments herein in the preparation of a reagent or a kit for regulating RNA splicing, in the preparation of a reagent for gene therapy, or in the preparation of a medicament for the treatment of diseases caused by genetic mutations or tumors that benefit from changes in the proportion of different splicing isoforms of functional proteins. This disclosure is also directed to the fusion protein, its coding sequence and/or expression vector, and the sgRNA and/or its expression vector, according to any of the embodiments described herein, for regulating RNA splicing, gene therapy (especially for the treatment of diseases caused by genetic mutations or tumors benefiting from changes in the proportion of different splicing isoforms of functional proteins).

The methods described herein can effectively induce exon skipping (e.g., RPS24 exon 5, CD45 exon 5, DMD gene exon 50, 23, 51, etc.), regulate the selection of mutually exclusive exons (PKM1/PKM2, etc.), induce intron retention/inclusion (BAP1 and TP53, etc.) and induce the use of alternative splice sites (STAT3α/β, etc.), and the like. At the same time, by mutating the C upstream of the 3' splice site to T, the inclusion ratio of selective exons can be promoted (RPS24 exon 5, GANAB exon 5, ThyN1 exon 6, OS9 exon 13 and SMN2 exon 7). In addition, this disclosure also proves that this method can effectively correct the genetic splicing defects caused by human genetic mutations. Therefore, the method disclosed herein is a general RNA splicing regulation method, which can be used for treatment of diseases, especially for gene therapy of the following diseases: Duchenne myasthenia caused by mutations in the DMD gene, SMN, thalassemia caused by 647G>A mutation of β hemoglobin IVS2, familial hypercholesterolemia and premature aging caused by LMNA mutation. At the same time, the method described herein can also achieve the treatment of tumors and other diseases by regulating the ratio of different splicing isoforms, including but not limited to inducing conversion of Stat3α to Stat3β, conversion of PKM2 to PKM1, MDM4 exon 6 skipping, selection of Bcl2 alternative splice sites, LRP8 exon 8 skipping, etc.

The present disclosure will be illustrated by way of specific examples below. It should be understood that these examples are merely exemplary and do not limit the scope of the present disclosure. The experimental methods without specifying the specific conditions in the following examples generally used the conventional conditions, such as those described in Sambrook & Russell, Molecular Cloning: A Laboratory Manual (3rd ed.) or followed the manufacturer's recommendation. Unless defined otherwise, technical and scientific terms used herein have the same meaning as commonly understood by one of ordinary skill in the art to which this disclosure is related. In addition, any methods and materials similar or equivalent to those described herein can be applied to the present disclosure. The preferable implementation methods and materials described herein are for illustration purposes only.

### I. Materials and methods

### (1) Construction of plasmids expressing AIDX-Cas9 or Cas9-AIDX fusion protein

With reference to the method disclosed in the examples of CN 201710451424.3 (the entire contents of which are incorporated herein by reference), a plasmid expressing AIDX-Cas9 or Cas9-AIDX fusion protein used herein was constructed.

In the following experiments, the AIDX-nCas9-Ugi fusion protein was used, and its expression plasmid, namely M091-AIDX- XTEN-nCas9-Ugi, was constructed according to the methods of Examples 1-3 and 14 of CN 201710451424.3, which expressed the fusion protein of SEQ ID NO: 23, wherein, residues 1-182 is the amino acid sequence of AIDX, residues 183-198 is the amino acid sequence of linker XTEN, residues 199-1566 is the amino acid sequence of nCas9, and residues 1567-1570 and 1654-1657 are linker sequences, residues 1571-1653 is the amino acid sequence of Ugi, and residues 1658-1664 is the amino acid sequence of SV40 NLS. The coding sequence of the fusion protein is shown as SEQ ID NO: 22.

### (2) Preparation of gRNA

1. Searching for 20bp target sequence. If the starting base of the 20 bp target sequence is not G, a G should be added to its 5' end to enable efficiently transcription by the RNA polymerase III U6 promoter. It should be n oted that the target sequence cannot contain XhoI or NheI recognition site.
2. The sgRNA was cloned into pLX (Addgene) to obtain pLX sgRNA. The following 4 primers were required, wherein R1 and F2 were sgRNA specific:
   F1: AAA*CTCGAG*TGTACAAAAAAGCAGGCTTTAAAG (SEQ ID NO: 18)
   R1: rc(GN₁₉)GGTGTTTCGTCCTTTCC (SEQ ID NO: 19)
   F2: GN₁₉GTTTTAGAGCTAGAAATAGCAA (SEQ ID NO: 20)
   R2: AAA*GCTAGC*TAATGCCAACTTTGTACAAGAAAGCTG (SEQ ID NO: 21) wherein, GN₁₉=new target binding sequence, rc(GN₁₉)=reverse complementary sequence of the new target binding sequence.
3. F1+R1 and F2+R2 were used to amplify pLX sgRNA respectively;
4. The two amplified products were purified by gel purification, combined and used for the third PCR with F1+R2;
5. NheI and XhoI were used to digest the products obtained from the PCR in Step 4; and
6. SgRNA expression vectors were prepared by ligation and transformation.

### (3) Cell Transfection

293T Cells were grown to 70-90% confluence before transfection. For transfection, plasmid DNA-liposome complexes were prepared by diluting four-folds amount of Lipofectamine® 2000 reagent in Opti-MEM® medium, and separately diluting the plasmid expressing the fusion protein described herein and the plasmid for the corresponding sgRNA in Opti-MEM® medium, then adding the diluted plasmids to the diluted Lipofectamine® 2000 reagent (1:1) and incubating for 30 minutes. The plasmid DNA-liposome complex was then transfected into 293T cells. As a control, only the plasmid DNA-liposome complex was transfected into reporter cells obtained according to Example 4 of CN201710451424.3, 2ug/ml puromycin and 20ug/ml blasticidin were added, and cells were screened for 3 days; on day 7 after transfection, gene expression, splicing and mutation were analyzed by high-throughput sequencing, respectively.

### (4) Quantitative PCR and high-throughput sequencing, etc.

Unless defined otherwise, biological methods such as quantitative PCR and high-throughput sequencing in this disclosure were implemented using the methods and reagents commonly used in the art.

### II. Result

### 1. Mutation of G to A at the splice site(s) led to exon skipping.

RPS24 is a constituent protein of ribosomes, and its mutation will cause congenital aplastic anemia. Exon 5 of RPS24 can be alternatively spliced to produce two isoforms with different 3' UTRs, in which liver cancer cells tend to express the isoform containing exon 5. However, its physiological function is not clear.

In this experiment, TAM technology was used to design the sgRNA (RPS24-E5-5'SS, the sequence of its target binding region is shown in SEQ ID NO: 9), and the G of the 5' splice site or 3' splice site of RPS24 exon 5 was mutated to A, regulating alternative splicing of exon 5. 293T cells were transfected as described above, and gene expression, splicing and mutation were analyzed by high-throughput sequencing on the 7th day after transfection.

In 293T cells, the fusion protein targeted to the 5' splice site of RPS24 exon 5 by use of the UNG inhibitor UGI in the AIDX-nCas9-Ugi fusion protein and the sgRNA. According to the results of the sequencing, the first base of intron 5 (IVS5+1) had more than 40% of G to A mutations, and the last base of exon 5 had 30% of G to A mutations, while there were other two sites on exon 5 having less than 10% of G to A mutations (Figure 3, A). Sequencing of exon splice sites revealed that the inclusion ratio of exon 5 in the cells transfected with RPS24 sgRNA was decreased compared to the control group (Figure 3, B); quantitative PCR results also provided the consistent conclusion (Figure 3, C); no exon mutation was found in mature RNA (Figure 3, A).

At the same time, two monoclonal cell lines with identical genotype were obtained, in which 5' splice sites were completely mutated to A, while a G to A mutation in the exon was also found (Figure 3, D). In these two clones, the isoform containing RPS24 exon 5 was completely undetectable, indicating that the G to A mutation at the 5' splice site caused skipping of RPS24 exon 5 (Figure 3, E).

The above results show that the TAM technique could effectively mutate G to A at the splice site(s), resulting in exon skipping (mutation at 5' splice site of RPS exon 5).

### 2. Mutation of G to A at the splice site(s) of CD45 exon 5 led to exon skipping.

To further verify whether the splice site(s) can be effectively destroyed and exon skipping can be regulated, three selective exons of the CD45 gene were selected as target genes. CD45 is a receptor tyrosine phosphatase, which can regulate the development and function of T lymphocytes and B lymphocytes by regulating the signaling of antigen receptors (such as TCR or BCR). The CD45 gene consists of approximately 33 exons, in which exons 4, 5, and 6 encoding the extracellular regions A, B, and C of the CD45 protein can be alternatively spliced. The expression pattern of the CD45 isoforms depends on the developmental stage of T cells and B cells. The longest CD45 isoform (B220) containing the three selective exons is expressed on the surface of B cells.

The sgRNAs (CD45-E5-5'SS and CD45-E5-3'SS, the sequences of their target binding region were SEQ ID NO: 1 and 2) for the Gs at 5' splice site and 3' splice site of exon 5 in CD45 gene were designed. The editing of exon 5 splice sites was performed in Raji cells, a germinal center B cell line expressing the unspliced CD45 isoform. 400 ng expression plasmid of AIDx-nCas9-Ugi, 300 ng expression plasmid of sgRNA and 50 ng expression plasmid of Ugi were electrotransfected into 1×10⁵ Raji cells with Neon (Life Technologies) with 1,100V voltage and a pulse of 40 ms. 24h after transfection, 2µg/ml puromycin was added to select transfected cells for 3 days.

It was found that the two sgRNAs could induce G>A mutations at the splice sites in 53.6% and 73.4% of the DNAs, respectively (Figures 1 and 2). When the splice site(s) of exon 5 were destroyed, CD45RB expression was significantly down-regulated, and the expression of CD45RA and CD45RC did not change significantly, indicating that the splice sites were independent when inducing exon skipping, and mutations in either 5'SS or 3'SS could cause exon skipping.

### 4. Mutation of G to A at the splice site(s) of TP53 exon 8 led to exon skipping.

In this experiment, TAM technology was used to design sgRNA (TP53-E8-5'SS, the sequence of which is shown in SEQ ID NO: 7), and the G of the 3' splice site of TP53 exon 8 was mutated to A, regulating alternative splicing of exon 8 (Figure 4). 293T cells were transfected as described above, and gene expression, splicing and mutation were analyzed by high-throughput sequencing on the 7th day after transfection.

According to the results of the sequencing, the first base of intron 8 (IVS8+1) had more than 80% of G to A mutations (Figure 4, A). Sequencing of exon splice sites revealed that more than 40% of TP53s in sgRNA-transfected cells skipped exon 8; quantitative PCR results (Figure 4, B, C) also provided the consistent conclusion; no exon mutation was found in mature RNA. The control group had no detectable skipping of exon 8.

### 5. Mutation of G to A at the splice site(s) of TP53 exon 9 led to exon skipping.

It was verifred that skipping of exon 9 in TP53 gene can be achieved by the same method. Specifically, 293T cells were transfected using TAM and with the sgRNA targeting 3'SS of TP53 exon 9 (TP53-E9-3'SS, its target binding sequence is shown in SEQ ID NO: 8). Seven days after transfection, intron-exon junctions were amplified from genomic DNA and analyzed by high-throughput sequencing. TP53 splicing was analyzed by RT-PCR. The splicing junctions were amplified from cDNA and analyzed by high-throughput sequencing. 3'SS mutation caused exon skipping in 34% of the total transcripts and activatiton of the cryptic splice site in 23.6% of the mRNAs. TAM-treated cells also activated the neuronal exon within intron 8 (4.3% of the total transcripts) (Figure 4, D-F).

### 6. Accurate editing of splice sites can change the selection of alternative splice sites

In addition to exon skipping, the selection of alternative splice sites may occur during RNA splicing, and new protein isoforms with different physiological functions may be formed. For example, the selection of an alternative splice site on exon 23 of Stat3 will result in a truncated STAT3β isoform lacking the C-terminal transactivation domain. The full-length STAT3α can promote tumorigenesis, while STAT3β has dominant negative effect, inhibiting STAT3α function and promoting tumor cell apoptosis. Especially in breast cancer cells, inducing STAT3β expression can inhibit cell survival more effectively compared to knocking out STAT3 expression, indicating that inducing STAT3β expression can be used as a tumor therapy. Because there is only 50bp between the conventional splice site and alternative splice site of STAT3, it is difficult to induce STAT3β expression using the conventional double sgRNA splicing method, while TAM technology can provide a more accurate gene editing method. In this experiment, with the sgRNA designed to destroy conventional splice sites, TAM eliminated the typical 3'SS of Stat3 exon 23 (Stat3α), and promoted the use of downstream alternative 3'SS (Stat3β), the schematic diagram of which is shown in Figure 5(A). 293T cells were transfected with AIDx-nCas9-Ugi and the sgRNA targeting Stat3 exon 23 (STAT3-E23-3'SS, its target binding region is shown in SEQ ID NO: 3) or the sgRNA targeting AAVS1 (Ctrl). Intron-exon junctions were amplified from DNA (top 2 panels) or cDNA (bottom 2 panels) and analyzed by high-throughput sequencing. TAM and sgRNA were expressed in 293T cells using the method described above, and more than 50% of the Gs at 3' splice site were mutated to As (Figure 5, B). Results show that TAM enhanced the use of the distal 3'SS in Stat3 exon 23 (Figure 5, C). Quantitative PCR and immunoblotting analysis revealed that STAT3β expression level was up-regulated and STAT3α expression level was down-regulated (Figure 5, E-F). As expected, proliferation rate of the TAM-edited cells was more significantly suppressed compared to cells with STAT3 expression knocking out.

The above results show that, in the case of extremely close alternative splice sites, TAM technology can overcome the defects of conventional double sgRNA splicing methods, accurately destroy selective splice sites, and regulate the selection of alternative splice sites.

### 7. Mutually exclusive exon

Mutually exclusive exon is another major type of alternative splicing, in which mutually exclusive exons can be selectively included in different transcripts to produce proteins with different functions. Pyruvate kinase (PKM) is the rate-limiting enzyme of the glycolysis process. During splicing, exons 9 and 10 of PKM can be selectively included to produce two isoforms PKM1 and PKM2, wherein PKM1 containing exon 9 but not exon 10 is mainly expressed in adult tissues, while PKM2 containing exon 10 but not exon 9 is mainly expressed in embryonic stem cells and tumor cells. Because PKM2 is related to tumorigenesis, it is hoped that TAM technology can switch the PKM splicing mode of tumor cells from PKM2 to PKM1.

Figure 6(A) shows a schematic diagram of TAM switching PKM2 to PKM1 in C2C12 cells. In the top panel, exon 10 of the PKM gene rather than exon 9 was spliced to produce PKM2, whose cDNA was recognized by the restriction enzyme PstI; in the bottom panel, TAM converted the GT dinucleotide to AT at the 5'SS of exon 10. Therefore, exon 9 instead of exon 10 was spliced to produce PKM1, whose cDNA was recognized by the restriction enzyme NcoI.

SgRNA (PKM-3'SS-E10 or PKM-5'SS-E10, the sequence of their target binding region is SEQ ID NO: 15 or 16, respectively) for the 3' or 5' splice site of intron 10 were designed and transferred into C2C12 cells to mutate the G to A (Figure 6, C, D). It was found that in the muscle cells differentiated from C2C12, PKM2 expression was significantly down-regulated and PKM1 expression was up-regulated (Figure 6, B, E, F). Similarly, in undifferentiated C2C12 cells, PKM2 expression was significantly down-regulated and PKM1 expression was up- regulated (Figure 6, G, H).

By the sgRNA (PKM-3'SS-E9, PKM-5'SS-E9, their target binding region is shown in SEQ ID NO: 13 or 14, respectively) targeting the 5' or 3' splice site of intron 9, the G could be mutated to A, while PKM1 expression level was down-regulated (Figure 7) and PKM2 expression was up-regulated. This further proved that the mutation of the splice site(s) can change the selection of the splice site(s) of mutually exclusive exons.

### 8. Inducing intron retention

Intron retention is another type of alternative splicing, and recent studies have shown that intron retention occurs in many human diseases including tumors. We demonstrated that the use of TAM and sgRNA to disrupt the splice site(s) of a corresponding intron can specifically induce intron retention.

BAP1 is a histone deubiquitinase, and its second intron is retained in some tumors, causing a decrease in BAP1 expression. The second intron of BAP1 may be spliced in an intron-defined manner, wherein the 5'SS is paired with the downstream 3'SS. The G is converted to A, and U1 recognizes U1 RNP at 5'SS and destroys the intron definition, resulting in the inclusion of the intron. This experiment used TAM to mutate G at the 5' splice site of intron 2 of BAP1, the schematic diagram of which is shown in Figure 8(A).

SgRNA (BAP1-E2-5'SS, its target binding region is shown in SEQ ID NO: 5) targeting the 5' splice site of intron 2 was designed. 293T cells were transfected with the expression plasmid of AIDx-nCas9-Ugi and the expression plasmid of the sgRNA targeting AAVS1 (Ctrl) or BAP1 intron 2. Seven days after transfection, BAP1 mRNA splicing was analyzed by RT-PCR (Figure 8, B) or isoform-specific real-time PCR (Figure 8, C). The results show that more than 70% of Gs were mutated to As (Figure 8, D). After mutation, the retention of intron 2 was induced, and more than 60% of the BAP1 mRNAs contained intron 2; similarly, mutation of the 3' splice site of the intron 2 (sgRNA sequence is shown as SEQ ID NO: 6 (BAP1-E3-3'SS)) also induced BAP1 intron retention (Figure 9, B-E).

### 9. C to T mutation at 3' splice site-3 postion can promote exon inclusion

In addition to splice sites, other cis-acting elements on mRNA can also change the splicing process of pre-mRNA, therefore TAM technology can also be used to edit other splicing regulatory elements. Because changes in introns do not affect the sequences for gene expression, we focused on the editing of splicing regulatory elements of intron. A polypyrimidine chain consisting of cytosine (C) and thymine (T) is present upstream of the 3' splice site. This experiment proved that the C in the polypyrimidine chain can be mutated to T by TAM and the corresponding sgRNA, therefore enhancing the strength of the 3' splice site and promoting the inclusion of downstream exons.

293T cells were transfected with the expression plasmid of AIDx-nCas9-Ugi and the expression plasmid of sgRNA targeting AAVS1 (Ctrl) or sgRNA targeting polypyrimidine nucleosides of the fifth exon in RPS24 (RPS24-E5-PPT, its target binding region is shown as SEQ ID NO: 10). Six days after transfection, sgRNA targeting regions were amplified from genomic DNA and analyzed by high-throughput sequencing with over 8000x coverage. The results show that more than 50% of the Cs in the polypyrimidine chain were mutated to Ts. It was found that the inclusion rate of exon 5 increased (Figure 11, B, C). After sorting, two single-cell clones containing complete C to T mutations were obtained, and their inclusion rate of exon 5 was increased by 8-fold and 5-fold, respectively (Figure 11, E).

In addition, 293T cells were transfected with the expression plasmid of AIDx-nCas9-Ugi and the expression plasmid of control sgRNA (Ctrl) or sgRNA targeting PPT of exon 6 in GANAB (GANAB-E6-PPT, its target binding region is shown as SEQ ID NO: 4). Six days after transfection, sgRNA targeting regions were amplified from genomic DNA and analyzed by high-throughput sequencing with over 8000x coverage. The results are shown in Figure 10 (B-E), wherein multiple Cs were induced to mutate to Ts, with the highest being IVS5-6C, in which more than 70% of the Cs were mutated to Ts. High-throughput sequencing proved that the inclusion of exon 6 was increased by 50%. Similar methods could also cause the increase of the inclusion of ThyN1 exon 6 (the target binding region of the sgRNA is shown in SEQ ID NO: 12, THYN1-E6-PPT) (Figure 10, F-G) and the increase of the inclusion of OS9 exon 13 (the target binding region of the sgRNA is shown in SEQ ID NO: 11, OS9-E13-PPT) (Figure 10, H-I).

### 10. TAM technology can restore DMD protein expression in human iPS cells and mdx mouse models (C2C12 and iPS)

Duchenne muscular dystrophy (DMD) is a muscular dystrophy disease. There is one case for every 4,000 men in the United States. The heritable mutation of the patient's DMD gene leads to the change of the gene's open reading frame or the formation of immature codons, resulting in dystrophin defects in skeletal muscle and the occurrence of the disease. Compared with the mutated DMD gene, the truncated dystrophin retains partial function, resulting in Becker muscular dystrophy with mild symptom. Therefore, some studies have used antisense oligonucleotides or double sgRNA-mediated CRISPR technology to skip some exons, so that to restore the open reading frame of DMD and promote the expression of dystrophin. This method of partially restoring the expression of dystrophin by skipping the non-essential regions of the DMD gene is expected to benefit 80% of DMD patients. However, treatment by antisense oligonucleotides requires continuous administration, which is extremely time-consuming and expensive. It is necessary to develop a new DMD gene therapy.

In order to find out whether TAM technology can regulate exon skipping of the DMD gene, iPS cells of a DMD patient lacking exon 51 is used in this experiment. According to the results of sequence analysis, after skipping of exon 50 by the sgRNA (the sequence of its target binding region is shown as SEQ ID NO: 17, DMD EXON50 5'SS), the open reading frame of dystrophin protein was restored (Figure 12). The iPSCs from the patient were transfected with the expression plasmid of sgRNA (the sequence of the target binding region is shown in SEQ ID NO: 17) and the expression plasmid of AIDx-nCas9-Ugi. High-throughput sequencing shows that it can induce more than 12% of G>A mutations (Figure 12, B), and then a monoclonal cell having complete G>A mutations were obtained (Figure 12B). Then the iPSCs were differentiated into cardiomyocytes and it was found that the TAM-edited cells had exon 50 skipping (Figure 12C, D). Further, western bloting shows that the expression of the dystrophin protein was restored in the TAM-repaired cells (Figure 12, E).

Using the same experiment, skipping of DMD exon 50 was induced by AIDx-saCas9 (KKH, nickase)-Ugi (coding sequence: SEQ ID NO: 49, amino acid sequence: SEQ ID NO: 50) and the corresponding sgRNA sequence (the sequence is shown in SEQ ID NO: 51, and its backbone sequence is shown in SEQ ID NO: 52). Specifically, after treating iPSC cells of the Duchenne myasthenia patient with control sgRNA (ctrl) or targeting sgRNA (E50-5'SS) together with AIDx-saCas9 (KKH, nickase)-Ugi, the corresponding DNA was amplified by PCR, and the induced mutations were analyzed by high-throughput sequencing. The data are representative of two independent experiments. The results are shown in Figure 14(A). Normal human-derived iPSCs, patient-derived iPSCs, and repaired patient-derived iPSCs were differentiated into cardiomyocytes, and the expression of the DMD gene and dystrophin was detected by RT-PCR or western blot or immunofluorescence staining, as shown in Figure 14, B, C and D, respectively. Figure 14, E, F, and G shows that the repaired cardiomyocytes reversed the amyasthenia phenotype. Creatine kinase release induced by hypotonicity (E), miR31 expression (F), and the expression of β-dystrophin proteoglycan protein (G) proved that the repaired cardiomyocytes reversed the phenotype of amyasthenia. In addition, whole-genome sequencing proved the high specificity of the gene editing, with only one off-target site found in two whole-genome sequencing (Figure 14, H and I).

The sequence involved in this disclosure is as follows:

| Sequence No. | Name | Sequence No. | Name |
|---|---|---|---|
| 1 | CD45-E5-5'SS | 27 | dcas9-aidm |
| 2 | CD45-E5-3'SS | 28 | AIDx-XTEN-dCas9 |
| 3 | STAT3-E23-3'SS | 29 | AIDx-XTEN-dCas9 |
| 4 | GANAB-E6-PPT | 30 | dCas9-XTEN-AID P182X K10E T82I E156G |
| 5 | BAP1-E2-5'SS | 31 | dCas9-XTEN-AID P182X K10E T82I E156G |
| 6 | BAP1-E3-3'SS | 32 | ncas9-P182x |
| 7 | TP53-E8-5'SS | 33 | ncas9-P182x |
| 8 | TP53-E9-3'SS | 34 | PAM sequence |
| 9 | RPS24-E5-5'SS | 35 | |
| 10 | RPS24-E5-PPT | 36 | |
| 11 | OS9-E13-PPT | 37 | |
| 12 | THYN1-E6-PPT | 38 | |
| 13 | PKM-3'SS-E9 | 39 | |
| 14 | PKM-5'SS-E9 | 40 | linker sequence |
| 15 | PKM-3'SS-E10 | 41 | |
| 16 | PKM-5'SS-E10 | 42 | |
| 17 | DMD EXON50 5'SS | 43 | |
| 18 | primer | 44 | |
| 19 | | 45 | |
| 20 | | 46 | |
| 21 | | 47 | dCas9-XTEN-AID P182X |
| 22 | AIDX-XTEN-nC AS9 | 48 | dCas9-XTEN-AID P182X |
| 23 | AIDX-XTEN-nC AS9 | 49 | AIDx-saCas9(KKH nickase)-Ugi |
| 24 | dcas9-AID | 50 | AIDx-saCas9(KKH nickase)-Ugi |
| 25 | dcas9-AID | 51 | DMD EXON50 5'SS |
| 26 | dcas9-aidm | 52 | sgRNA backbone sequecne |

## Claims

1. A method for regulating RNA splicing of a gene of interest in a cell, wherein the method includes expressing targeting cytosine deaminase in the cell to induce mutation of the 3' splice site AG to AA of an intron of interest of the gene of interest in the cell, or mutation of the 5' splice site GT to AT of an intron of interest of the gene of interest in the cell, or mutation of the multiple Cs to Ts in the polypyrimidine region of an intron of interest of the gene of interest in the cell.

2. The method according to claim 1, wherein the targeting cytosine deaminase is selected from the group consisting of:
(1) a fusion protein of a cytosine deaminase, or a fragment or mutant thereof retaining enzyme activity, and a Cas enzyme with helicase activity and partial or no nuclease activity;
(2) a fusion protein of a cytosine deaminase, or a fragment or mutant thereof retaining enzyme activity, and a TALEN protein that specifically recognizes a target sequence;
(3) a fusion protein of a cytosine deaminase, or a fragment or mutant thereof retaining enzyme activity, and a zinc finger protein that specifically recognizes a target sequence;
(4) a fusion protein of a cytosine deaminase, or a fragment or mutant thereof retaining enzyme activity, and a Cpf enzyme with helicase activity and partial or no nuclease activity; and
(5) a fusion protein of a cytosine deaminase, or a fragment or mutant thereof retaining enzyme activity, and an Ago protein.

3. The method according to claim 2, wherein the targeting cytosine deaminase is the fusion protein of a cytosine deaminase, or a fragment or mutant thereof retaining enzyme activity, and a Cas enzyme with helicase activity and partial or no nuclease activity, or the fusion protein of a cytosine deaminase, or a fragment or mutant thereof retaining enzyme activity, and a Cpf enzyme with helicase activity and partial or no nuclease activity; the method includes expressing the targeting cytosine deaminase and an sgRNA in the cell, wherein the sgRNA is specifically recognized by the Cas enzyme or Cpf enzyme and binds to the sequence having the splice site of the intron of interest of the gene of interest, or binds to the complementary sequence of the polypyrimidine region of interest.

4. The method according to claim 3, wherein,
the sgRNA binds to the sequence having the 5' splice site of the intron of interest of the gene of interest, and the fusion protein mutates the GT to AT at the 5' splice site, thereby inducing exon skipping, activating alternative splice sites, inducing mutually exclusive exon switching or intron retention; or
the sgRNA binds to the sequence having the 3' splice site of the intron of interest of the gene of interest, and the fusion protein mutates the AG to AA at the 3' splice site, thereby inducing exon skipping, activating alternative splice sites, inducing mutually exclusive exon switching or intron retention; or
the sgRNA binds to the complementary sequence of the polypyrimidine region of interest, and induces the C to T at the polypyrimidine region, thereby enhancing exon inclusion.

5. The method according to claim 2, wherein the targeting cytosine deaminase is the fusion protein of a cytosine deaminase, or a fragment or mutant thereof retaining enzyme activity, and an Ago protein; the method includes the step of expressing in the cell the targeting cytosine deaminase and a gDNA recognized by the Ago protein.

6. The method according to claim 3, wherein, the fusion protein further contains Ugi, or the method further includes the step of simultaneously transferring an expression plasmid of Ugi;
or, the method comprises the step of directly introducing the fusion protein and the sgRNA.

7. The method according to any of claims 2-3, wherein,
the Cas enzyme has no nuclease activity, with no DNA double-strand break ability, or partial nuclease activity, with only DNA single-strand break ability; and/or
the Cas enzyme is selected from the group consisting of: Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, their homologues or modified variants; preferably, the Cas enzyme is a Cas9 enzyme, preferably selected from the group consisting of: Cas9 from *Streptococcus pyogenes,* Cas9 from *Staphylococcus aureus,* and Cas9 from *Streptococcus thermophilus;* and/or
the cytosine deaminase is full-length human-derived activated cytosine deaminase (hAID), or a fragment or mutant that retains enzyme activity, wherein the fragment includes at least the NLS domain, catalytic domain and APOBEC-like domain of the cytosine deaminase; and/or the fusion protein further comprises one or more of the following sequences: linker sequences, nuclear localization sequences, Ugi, and amino acid residues or sequences introduced to construct the fusion protein, promote expression of the recombinant proteins, obtain the recombinant proteins automatically secreted from the host cells, or facilitate the purification of the recombinant proteins.

8. The method according to claim 7, wherein,
the Cas enzyme is a Cas9 enzyme, and the two endonuclease catalytic domains RuvC1 and/or HNH of the enzyme are mutated, resulting in lacking of nuclease activity and retention of helicase activity; preferably, both the RuvC1 and HNH of the Cas9 enzyme are mutated, resulting in lacking of nuclease activity and retention of helicase activity; more preferably, the amino acid 10 asparagine of the Cas9 enzyme is mutated to alanine or other amino acids, the amino acid 841 histidine is mutated to alanine or other amino acids; more preferably, the amino acid sequence of the Cas9 enzyme is amino acid residues 199-1566 of SEQ ID NO: 23, or amino acid residues 42-1452 of SEQ ID NO: 25, or amino acid residues 42-1419 of SEQ ID NO: 33, or amino acid residues 199-1262 of SEQ ID NO: 50; and/or
the fragment of the cytosine deaminase comprises at least amino acid residues 9-182 of the cytosine deaminase, for example, at least amino acids residues 1-182; preferably, the fragment consists of amino acid residues 1-182, amino acid residues 1-186, or amino acid residues 1-190; or, the amino acid sequence of the cytosine deaminase is amino acid residues 1457-1654 of SEQ ID NO: 25, the fragment contains at least amino acid residues 1465-1638 of SEQ ID NO: 25, for example, at least amino acid residues 1457-1638 of SEQ ID NO: 25; preferably, the fragment consists of amino acid residues 1457-1638 of SEQ ID NO: 25, amino acid residues 1457-1642 of SEQ ID NO: 25, or amino acid residues 1457-1646 of SEQ ID NO: 25; the mutant comprises substitution mutations at amino acid residues 10, 82, and 156, preferably, the substitution mutations are K10E, T82I, and E156G, more preferably, the mutant comprises amino acid residues 1447-1629 of SEQ ID NO: 31, or consists of amino acid residues 1447-1629 of SEQ ID NO: 31.

9. The method according to claim 8, wherein the amino acid sequence of the fusion protein is SEQ ID NO: 23, 25, 27, 29, 31, 33, 48, or 50, or amino acids 26-1654 of SEQ ID NO: 25, or amino acids 26-1638 of SEQ ID NO: 27, or amino acids 26-1629 of SEQ ID NO: 31, or amino acids 26-1638 of SEQ ID NO: 33, or amino acids 26-1629 of SEQ ID NO: 48.

10. A fusion protein comprising a Cas protein with helicase activity and partial or no nuclease activity, a cytosine deaminase or a fragment or mutant thereof that retains enzyme activity, and Ugi, and an optional nuclear localization sequence and linker sequence.

11. The fusion protein according to claim 10, wherein,
the Cas enzyme is according to claim 7 or 8;
the cytosine deaminase or a fragment or mutant thereof that retains enzyme activity is according to claim 7 or 8;
the amino acid sequence of the Ugi is amino acid residues 1576-1659 of SEQ ID NO:23.

12. A composition or a kit comprising the composition, wherein, the composition comprises the fusion protein according to claim 10 or 11 or an expression vector thereof;
the kit further optionally comprises an sgRNA recognized by the fusion protein in the composition or its expression vector;
preferably, the kit comprises a virus particle that enable the expression of the fusion protein in the composition and sgRNA.

13. An sgRNA comprising a protein recognition region and a target recognition region, wherein the target binding region binds to the sequence comprising a splice site of an intron of interest of a gene of interest, or binds to the complementary sequence of a polypyrimidine region of a gene of interest.

14. The sgRNA according to claim 13, wherein the target binding region of the sgRNA binds to the sequence in DMD exon 50 having the 5' splice site; preferably, the target binding region of the sgRNA is SEQ ID NO: 17 or 51.

15. Use of the fusion protein according to claim 10 or 11 or its expression vector and the sgRNA recognized by the fusion protein or its expression vector in the preparation of a reagent or a kit for regulating RNA splicing, a reagent for gene therapy, or a medicament for the treatment of a disease caused by genetic mutations or a tumor that benefits from changes in the proportion of different splicing isoforms of functional proteins;
preferably, the disease caused by genetic mutations is selected from the group consisting of: Duchenne myasthenia caused by mutations in the DMD gene, SMN, thalassemia caused by 647G>A mutation of β hemoglobin IVS2, familial hypercholesterolemia and premature aging caused by LMNA mutation; the splicing isoform is selected from the group consisting of: conversion of Stat3α to Stat3β, conversion of PKM2 to PKM1, MDM4 exon 6 skipping, Bcl2 alternative splice sites selection, and LRP8 exon 8 skipping.


Sequence listing

<110>  SHANGHAI INSTITUTES FOR BIOLOGICAL SCIENCES, CAS

<120>  METHOD FOR MODULATING RNA SPLICING BY INDUCING BASE MUTATION AT
SPLICE SITE OR BASE SUBSTITUTION IN POLYPYRIMIDINE REGION

<130>  172473PCWO

<150>  CN 201710611651.8
<151>  2017-07-25

<160>  52

<170>  SIPOSequenceListing 1.0

<210>  1
<211>  20
<212>  DNA
<213>  Artificial Sequence

<220>
<223>  The target binding region of sgRNA CD45-E5-5'SS

<400>  1
cctgagatag cattgctgcc                                                    20

<210>  2
<211>  20
<212>  DNA
<213>  Artificial Sequence

<220>
<223>  The target binding region of sgRNA CD45-E5-3'SS

<400>  2
aacacctaag gtaggaaagt                                                    20

<210>  3
<211>  20
<212>  DNA
<213>  Artificial Sequence

<220>
<223>  The target binding region of sgRNA STAT3-E23-3'SS

<400>  3
gtcgttctgt aggaaatggg                                                    20

<210>  4
<211>  20
<212>  DNA
<213>  Artificial Sequence

<220>
<223>  The target binding region of sgRNA GANAB-E6-PPT

<400>  4
ctgccccagt ttctcggata                                                    20

<210>  5
<211>  20
<212>  DNA

<213>  Artificial Sequence

<220>
<223>  The target binding region of sgRNA BAP1-E2-5′SS

<400>  5
taccgaaatc ttccacgagc                                                   20

<210>  6
<211>  20
<212>  DNA
<213>  Artificial Sequence

<220>
<223>  The sequence of sgRNA BAP1-E3-3′SS

<400>  6
cacctgcgat gaggaaagga                                                   20

<210>  7
<211>  20
<212>  DNA
<213>  Artificial Sequence

<220>
<223>  The sequence of sgRNA TP53-E8-5′ SS

<400>  7
cctcgcttag tgctccctgg                                                   20

<210>  8
<211>  20
<212>  DNA
<213>  Artificial Sequence

<220>
<223>  The target binding region of sgRNA TP53-E9-3′SS

<400>  8
gctaggaaag aggcaaggaa                                                   20

<210>  9
<211>  20
<212>  DNA
<213>  Artificial Sequence

<220>
<223>  The target binding region of sgRNA RPS24-E5-5′SS

<400>  9
tatacctgtg atccaatctc                                                   20

<210>  10
<211>  20
<212>  DNA
<213>  Artificial Sequence

<220>
<223>  The target binding region of sgRNA RPS24-E5-PPT

<400>  10
tgattcagtg agctggagat                                                   20

<210>  11
<211>  20
<212>  DNA
<213>  Artificial Sequence

<220>
<223>  The target binding region of sgRNA OS9-E13-PPT

<400>  11
cccctctaag aggaggatcc                                                   20

<210>  12
<211>  20
<212>  DNA
<213>  Artificial Sequence

<220>
<223>  The target binding region of sgRNA THYN1-E6-PPT

<400>  12
gtacactgtt gtcacatagg                                                   20

<210>  13
<211>  20
<212>  DNA
<213>  Artificial Sequence

<220>
<223>  The target binding region of sgRNA PKM-3'SS-E9

<400>  13
ctatctgtaa ggtttagggt                                                   20

<210>  14
<211>  20
<212>  DNA
<213>  Artificial Sequence

<220>
<223>  The target binding region of sgRNA PKM-5'SS-E9

<400>  14
ccctacctgc cagactccgt                                                   20

<210>  15
<211>  23
<212>  DNA
<213>  Artificial Sequence

<220>
<223>  The target binding region of sgRNA PKM-3' SS-E10

<400>  15
ctaggggagc aacatccgtc cag                                               23

<210>  16
<211>  20
<212>  DNA
<213>  Artificial Sequence

<220>
<223>  The target binding region of sgRNA PKM-5'SS-E10

<400>  16
tcctacctgc cagacttggt                                                   20

<210>  17
<211>  20
<212>  DNA
<213>  Artificial Sequence

<220>
<223>  The target binding region of sgRNA DMD EXON50 5'SS

<400>  17
atacttacag gctccaatag                                                   20

<210>  18
<211>  33
<212>  DNA
<213>  Artificial Sequence

<220>
<223>  Primer

<400>  18
aaactcgagt gtacaaaaaa gcaggcttta aag                                    33

<210>  19
<211>  37
<212>  DNA
<213>  Artificial Sequence

<220>
<223>  Primer

<220>
<221>  misc_feature
<222>  (2)..(20)
<223>  n is a, c, g or t

<400>  19
gnnnnnnnnn nnnnnnnnnn ggtgtttcgt cctttcc                                37

<210>  20
<211>  42
<212>  DNA
<213>  Artificial Sequence

<220>
<223>  Primer

<220>
<221>  misc_feature
<222>  (2)..(20)
<223>  n is a, c, g or t

<400>  20
gnnnnnnnnn nnnnnnnnnn gttttagagc tagaaatagc aa                          42

<210>  21
<211>  36
<212>  DNA
<213>  Artificial Sequence

<220>

<223>  Primer

<400>  21
aaagctagct aatgccaact ttgtacaaga aagctg                                36

<210>  22
<211>  5013
<212>  DNA
<213>  Artificial Sequence

<220>
<223>  Coding sequence of AIDX-XTEN-nCAS9

<400>  22
atggacagcc tcttgatgaa ccggaggaag tttctttacc aattcaaaaa tgtccgctgg      60
gctaagggtc ggcgtgagac ctacctgtgc tacgtagtga agaggcgtga cagtgctaca     120
tccttttcac tggactttgg ttatcttcgc aataagaacg gctgccacgt ggaattgctc     180
ttcctccgct acatctcgga ctgggaccta gaccctggcc gctgctaccg cgtcacctgg     240
ttcacctcct ggagcccctg ctacgactgt gcccgacatg tggccgactt tctgcgaggg     300
aaccccaacc tcagtctgag gatcttcacc gcgcgcctct acttctgtga ggaccgcaag     360
gctgagcccg aggggctgcg gcggctgcac cgcgccgggg tgcaaatagc catcatgacc     420
ttcaaagatt atttttactg ctggaatact tttgtagaaa accatgaaag aactttcaaa     480
gcctgggaag ggctgcatga aaattcagtt cgtctctcca gacagcttcg gcgcatcctt     540
ttgcccagcg gcagcgagac tcccggaccc tcagagtccg ccacacccga aagtatggat     600
aagaaatact caataggctt agctatcggc acaaatagcg tcggatgggc ggtgatcact     660
gatgaatata aggttccgtc taaaaagttc aaggttctgg gaaatacaga ccgccacagt     720
atcaaaaaaa atcttatagg ggctctttta tttgacagtg gagagacagc ggaagcgact     780
cgtctcaaac ggacagctcg tagaaggtat acacgtcgga agaatcgtat ttgttatcta     840
caggagattt tttcaaatga gatggcgaaa gtagatgata gtttctttca tcgacttgaa     900
gagtcttttt tggtggaaga agacaagaag catgaacgtc atcctatttt tggaaatata     960
gtagatgaag ttgcttatca tgagaaatat ccaactatct atcatctgcg aaaaaaattg    1020
gtagattcta ctgataaagc ggatttgcgc ttaatctatt tggccttagc gcatatgatt    1080
aagtttcgtg gtcatttttt gattgaggga gatttaaatc ctgataatag tgatgtggac    1140
aaactattta tccagttggt acaaacctac aatcaattat ttgaagaaaa ccctattaac    1200
gcaagtggag tagatgctaa agcgattctt tctgcacgat tgagtaaatc aagacgatta    1260
gaaaatctca ttgctcagct ccccggtgag aagaaaaatg gcttatttgg gaatctcatt    1320
gctttgtcat tgggtttgac ccctaatttt aaatcaaatt ttgatttggc agaagatgct    1380
aaattacagc tttcaaaaga tacttacgat gatgatttag ataatttatt ggcgcaaatt    1440
ggagatcaat atgctgattt gtttttggca gctaagaatt tatcagatgc tattttactt    1500
tcagatatcc taagagtaaa tactgaaata actaaggctc ccctatcagc ttcaatgatt    1560
aaacgctacg atgaacatca tcaagacttg actcttttaa aagctttagt tcgacaacaa    1620
cttccagaaa agtataaaga aatctttttt gatcaatcaa aaaacggata tgcaggttat    1680
attgatgggg gagctagcca agaagaattt tataaattta tcaaaccaat tttagaaaaa    1740
atggatggta ctgaggaatt attggtgaaa ctaaatcgtg aagatttgct gcgcaagcaa    1800
cggacctttg acaacggctc tattccccat caaattcact tgggtgagct gcatgctatt    1860
ttgagaagac aagaagactt ttatccattt ttaaaagaca atcgtgagaa gattgaaaaa    1920
atcttgactt ttcgaattcc ttattatgtt ggtccattgg cgcgtggcaa tagtcgtttt    1980
gcatggatga ctcggaagtc tgaagaaaca attaccccat ggaattttga agaagttgtc    2040
gataaaggtg cttcagctca atcatttatt gaacgcatga caaactttga taaaaatctt    2100
ccaaatgaaa aagtactacc aaaacatagt ttgctttatg agtattttac ggtttataac    2160
gaattgacaa aggtcaaata tgttactgaa ggaatgcgaa aaccagcatt tctttcaggt    2220
gaacagaaga aagccattgt tgatttactc ttcaaaacaa atcgaaaagt aaccgttaag    2280
caattaaaag aagattattt caaaaaaata gaatgttttg atagtgttga aatttcagga    2340
gttgaagata gatttaatgc ttcattaggt acctaccatg atttgctaaa aattattaaa    2400
gataaagatt ttttggataa tgaagaaaat gaagatatct tagaggatat tgttttaaca    2460
ttgaccttat ttgaagatag ggagatgatt gaggaaagac ttaaaacata tgctcacctc    2520
tttgatgata aggtgatgaa acagcttaaa cgtcgccgtt atactggttg gggacgtttg    2580
tctcgaaaat tgattaatgg tattagggat aagcaatctg gcaaaacaat attagatttt    2640
ttgaaatcag atggttttgc caatcgcaat tttatgcagc tgatccatga tgatagtttg    2700
acatttaaag aagacattca aaaagcacaa gtgtctggac aaggcgatag tttacatgaa    2760
catattgcaa atttagctgg tagccctgct attaaaaaag gtattttaca gactgtaaaa    2820
gttgttgatg aattggtcaa agtaatgggg cggcataagc cagaaaatat cgttattgaa    2880
atggcacgtg aaaatcagac aactcaaaag ggccagaaaa attcgcgaga gcgtatgaaa    2940

cgaatcgaag aaggtatcaa agaattagga agtcagattc ttaaagagca tcctgttgaa 3000
aatactcaat tgcaaaatga aaagctctat ctctattatc tccaaaatgg aagagacatg 3060
tatgtggacc aagaattaga tattaatcgt ttaagtgatt atgatgtcga tcacattgtt 3120
ccacaaagtt tccttaaaga cgattcaata gacaataagg tcttaacgcg ttctgataaa 3180
aatcgtggta aatcggataa cgttccaagt gaagaagtag tcaaaaagat gaaaaactat 3240
tggagacaac ttctaaacgc caagttaatc actcaacgta agtttgataa tttaacgaaa 3300
gctgaacgtg gaggtttgag tgaacttgat aaagctggtt ttatcaaacg ccaattggtt 3360
gaaactcgcc aaatcactaa gcatgtggca caaattttgg atagtcgcat gaatactaaa 3420
tacgatgaaa atgataaact tattcgagag gttaaagtga ttaccttaaa atctaaatta 3480
gtttctgact tccgaaaaga tttccaattc tataaagtac gtgagattaa caattaccat 3540
catgcccatg atgcgtatct aaatgccgtc gttggaactg ctttgattaa gaaatatcca 3600
aaacttgaat cggagtttgt ctatggtgat tataaagttt atgatgttcg taaaatgatt 3660
gctaagtctg agcaagaaat aggcaaagca accgcaaaat atttctttta ctctaatatc 3720
atgaacttct tcaaaacaga aattacactt gcaaatggag agattcgcaa acgccctcta 3780
atcgaaacta atggggaaac tggagaaatt gtctgggata aagggcgaga ttttgccaca 3840
gtgcgcaaag tattgtccat gccccaagtc aatattgtca agaaaacaga agtacagaca 3900
ggcggattct ccaaggagtc aattttacca aaaagaaatt cggacaagct tattgctcgt 3960
aaaaaagact gggatccaaa aaaatatggt ggttttgata gtccaacggt agcttattca 4020
gtcctagtgg ttgctaaggt ggaaaaaggg aaatcgaaga agttaaaatc cgttaaagag 4080
ttactaggga tcacaattat ggaaagaagt tcctttgaaa aaaatccgat tgacttttta 4140
gaagctaaag gatataagga agttaaaaaa gacttaatca ttaaactacc taaatatagt 4200
ctttttgagt tagaaaacgg tcgtaaacgg atgctggcta gtgccggaga attacaaaaa 4260
ggaaatgagc tggctctgcc aagcaaatat gtgaattttt tatatttagc tagtcattat 4320
gaaaagttga agggtagtcc agaagataac gaacaaaaac aattgtttgt tgagcagcat 4380
aagcattatt tagatgagat tattgagcaa atcagtgaat tttctaagcg tgttatttta 4440
gcagatgcca atttagataa agttcttagt gcatataaca aacatagaga caaaccaata 4500
cgtgaacaag cagaaaatat tattcattta tttacgttga cgaatcttgg agctcccgct 4560
gcttttaaat attttgatac aacaattgat cgtaaacgat atacgtctac aaaagaagtt 4620
ttagatgcca ctcttatcca tcaatccatc actggtcttt atgaaacacg cattgatttg 4680
agtcagctag gaggtgactc tggtggttct actaatctgt cagatattat tgaaaaggag 4740
accggtaagc aactggttat ccaggaatcc atcctcatgc tcccagagga ggtggaagaa 4800
gtcattggga acaagccgga aagcgatata ctcgtgcaca ccgcctacga cgagagcacc 4860
gacgagaatg tcatgcttct gactagcgac gcccctgaat acaagccttg ggctctggtc 4920
atacaggata gcaacggtga gaacaagatt aagatgctct ctggtggttc tcccaagaag 4980
aagaggaaag tccatcacca ccaccatcac taa 5013

<210> 23
<211> 1670
<212> PRT
<213> Artificial Sequence

<220>
<223> Amino acid sequence of AIDX-XTEN-nCAS9

<400> 23
Met Asp Ser Leu Leu Met Asn Arg Arg Lys Phe Leu Tyr Gln Phe Lys
1               5                   10                  15
Asn Val Arg Trp Ala Lys Gly Arg Arg Glu Thr Tyr Leu Cys Tyr Val
            20                  25                  30
Val Lys Arg Arg Asp Ser Ala Thr Ser Phe Ser Leu Asp Phe Gly Tyr
        35                  40                  45
Leu Arg Asn Lys Asn Gly Cys His Val Glu Leu Leu Phe Leu Arg Tyr
    50                  55                  60
Ile Ser Asp Trp Asp Leu Asp Pro Gly Arg Cys Tyr Arg Val Thr Trp
65                  70                  75                  80
Phe Thr Ser Trp Ser Pro Cys Tyr Asp Cys Ala Arg His Val Ala Asp
                85                  90                  95
Phe Leu Arg Gly Asn Pro Asn Leu Ser Leu Arg Ile Phe Thr Ala Arg
            100                 105                 110
Leu Tyr Phe Cys Glu Asp Arg Lys Ala Glu Pro Glu Gly Leu Arg Arg
        115                 120                 125
Leu His Arg Ala Gly Val Gln Ile Ala Ile Met Thr Phe Lys Asp Tyr
    130                 135                 140

Phe Tyr Cys Trp Asn Thr Phe Val Glu Asn His Glu Arg Thr Phe Lys
145                 150                 155                 160
Ala Trp Glu Gly Leu His Glu Asn Ser Val Arg Leu Ser Arg Gln Leu
                165                 170                 175
Arg Arg Ile Leu Leu Pro Ser Gly Ser Glu Thr Pro Gly Thr Ser Glu
            180                 185                 190
Ser Ala Thr Pro Glu Ser Met Asp Lys Lys Tyr Ser Ile Gly Leu Ala
        195                 200                 205
Ile Gly Thr Asn Ser Val Gly Trp Ala Val Ile Thr Asp Glu Tyr Lys
    210                 215                 220
Val Pro Ser Lys Lys Phe Lys Val Leu Gly Asn Thr Asp Arg His Ser
225                 230                 235                 240
Ile Lys Lys Asn Leu Ile Gly Ala Leu Leu Phe Asp Ser Gly Glu Thr
                245                 250                 255
Ala Glu Ala Thr Arg Leu Lys Arg Thr Ala Arg Arg Arg Tyr Thr Arg
            260                 265                 270
Arg Lys Asn Arg Ile Cys Tyr Leu Gln Glu Ile Phe Ser Asn Glu Met
        275                 280                 285
Ala Lys Val Asp Asp Ser Phe Phe His Arg Leu Glu Glu Ser Phe Leu
    290                 295                 300
Val Glu Glu Asp Lys Lys His Glu Arg His Pro Ile Phe Gly Asn Ile
305                 310                 315                 320
Val Asp Glu Val Ala Tyr His Glu Lys Tyr Pro Thr Ile Tyr His Leu
                325                 330                 335
Arg Lys Lys Leu Val Asp Ser Thr Asp Lys Ala Asp Leu Arg Leu Ile
            340                 345                 350
Tyr Leu Ala Leu Ala His Met Ile Lys Phe Arg Gly His Phe Leu Ile
        355                 360                 365
Glu Gly Asp Leu Asn Pro Asp Asn Ser Asp Val Asp Lys Leu Phe Ile
    370                 375                 380
Gln Leu Val Gln Thr Tyr Asn Gln Leu Phe Glu Glu Asn Pro Ile Asn
385                 390                 395                 400
Ala Ser Gly Val Asp Ala Lys Ala Ile Leu Ser Ala Arg Leu Ser Lys
                405                 410                 415
Ser Arg Arg Leu Glu Asn Leu Ile Ala Gln Leu Pro Gly Glu Lys Lys
            420                 425                 430
Asn Gly Leu Phe Gly Asn Leu Ile Ala Leu Ser Leu Gly Leu Thr Pro
        435                 440                 445
Asn Phe Lys Ser Asn Phe Asp Leu Ala Glu Asp Ala Lys Leu Gln Leu
    450                 455                 460
Ser Lys Asp Thr Tyr Asp Asp Asp Leu Asp Asn Leu Leu Ala Gln Ile
465                 470                 475                 480
Gly Asp Gln Tyr Ala Asp Leu Phe Leu Ala Ala Lys Asn Leu Ser Asp
                485                 490                 495
Ala Ile Leu Leu Ser Asp Ile Leu Arg Val Asn Thr Glu Ile Thr Lys
            500                 505                 510
Ala Pro Leu Ser Ala Ser Met Ile Lys Arg Tyr Asp Glu His His Gln
        515                 520                 525
Asp Leu Thr Leu Leu Lys Ala Leu Val Arg Gln Gln Leu Pro Glu Lys
    530                 535                 540
Tyr Lys Glu Ile Phe Phe Asp Gln Ser Lys Asn Gly Tyr Ala Gly Tyr
545                 550                 555                 560
Ile Asp Gly Gly Ala Ser Gln Glu Glu Phe Tyr Lys Phe Ile Lys Pro
                565                 570                 575
Ile Leu Glu Lys Met Asp Gly Thr Glu Glu Leu Leu Val Lys Leu Asn
            580                 585                 590
Arg Glu Asp Leu Leu Arg Lys Gln Arg Thr Phe Asp Asn Gly Ser Ile
        595                 600                 605
Pro His Gln Ile His Leu Gly Glu Leu His Ala Ile Leu Arg Arg Gln
    610                 615                 620
Glu Asp Phe Tyr Pro Phe Leu Lys Asp Asn Arg Glu Lys Ile Glu Lys
625                 630                 635                 640
Ile Leu Thr Phe Arg Ile Pro Tyr Tyr Val Gly Pro Leu Ala Arg Gly

                645                 650                 655
Asn Ser Arg Phe Ala Trp Met Thr Arg Lys Ser Glu Glu Thr Ile Thr
            660                 665                 670
Pro Trp Asn Phe Glu Glu Val Val Asp Lys Gly Ala Ser Ala Gln Ser
        675                 680                 685
Phe Ile Glu Arg Met Thr Asn Phe Asp Lys Asn Leu Pro Asn Glu Lys
    690                 695                 700
Val Leu Pro Lys His Ser Leu Leu Tyr Glu Tyr Phe Thr Val Tyr Asn
705                 710                 715                 720
Glu Leu Thr Lys Val Lys Tyr Val Thr Glu Gly Met Arg Lys Pro Ala
                725                 730                 735
Phe Leu Ser Gly Glu Gln Lys Lys Ala Ile Val Asp Leu Leu Phe Lys
            740                 745                 750
Thr Asn Arg Lys Val Thr Val Lys Gln Leu Lys Glu Asp Tyr Phe Lys
        755                 760                 765
Lys Ile Glu Cys Phe Asp Ser Val Glu Ile Ser Gly Val Glu Asp Arg
    770                 775                 780
Phe Asn Ala Ser Leu Gly Thr Tyr His Asp Leu Leu Lys Ile Ile Lys
785                 790                 795                 800
Asp Lys Asp Phe Leu Asp Asn Glu Glu Asn Glu Asp Ile Leu Glu Asp
                805                 810                 815
Ile Val Leu Thr Leu Thr Leu Phe Glu Asp Arg Glu Met Ile Glu Glu
            820                 825                 830
Arg Leu Lys Thr Tyr Ala His Leu Phe Asp Asp Lys Val Met Lys Gln
        835                 840                 845
Leu Lys Arg Arg Arg Tyr Thr Gly Trp Gly Arg Leu Ser Arg Lys Leu
    850                 855                 860
Ile Asn Gly Ile Arg Asp Lys Gln Ser Gly Lys Thr Ile Leu Asp Phe
865                 870                 875                 880
Leu Lys Ser Asp Gly Phe Ala Asn Arg Asn Phe Met Gln Leu Ile His
                885                 890                 895
Asp Asp Ser Leu Thr Phe Lys Glu Asp Ile Gln Lys Ala Gln Val Ser
            900                 905                 910
Gly Gln Gly Asp Ser Leu His Glu His Ile Ala Asn Leu Ala Gly Ser
        915                 920                 925
Pro Ala Ile Lys Lys Gly Ile Leu Gln Thr Val Lys Val Val Asp Glu
    930                 935                 940
Leu Val Lys Val Met Gly Arg His Lys Pro Glu Asn Ile Val Ile Glu
945                 950                 955                 960
Met Ala Arg Glu Asn Gln Thr Thr Gln Lys Gly Gln Lys Asn Ser Arg
                965                 970                 975
Glu Arg Met Lys Arg Ile Glu Glu Gly Ile Lys Glu Leu Gly Ser Gln
            980                 985                 990
Ile Leu Lys Glu His Pro Val Glu Asn Thr Gln Leu Gln Asn Glu Lys
        995                 1000                1005
Leu Tyr Leu Tyr Tyr Leu Gln Asn Gly Arg Asp Met Tyr Val Asp Gln
    1010                1015                1020
Glu Leu Asp Ile Asn Arg Leu Ser Asp Tyr Asp Val Asp His Ile Val
1025                1030                1035                1040
Pro Gln Ser Phe Leu Lys Asp Asp Ser Ile Asp Asn Lys Val Leu Thr
                1045                1050                1055
Arg Ser Asp Lys Asn Arg Gly Lys Ser Asp Asn Val Pro Ser Glu Glu
            1060                1065                1070
Val Val Lys Lys Met Lys Asn Tyr Trp Arg Gln Leu Leu Asn Ala Lys
        1075                1080                1085
Leu Ile Thr Gln Arg Lys Phe Asp Asn Leu Thr Lys Ala Glu Arg Gly
    1090                1095                1100
Gly Leu Ser Glu Leu Asp Lys Ala Gly Phe Ile Lys Arg Gln Leu Val
1105                1110                1115                1120
Glu Thr Arg Gln Ile Thr Lys His Val Ala Gln Ile Leu Asp Ser Arg
                1125                1130                1135
Met Asn Thr Lys Tyr Asp Glu Asn Asp Lys Leu Ile Arg Glu Val Lys
            1140                1145                1150

Val Ile Thr Leu Lys Ser Lys Leu Val Ser Asp Phe Arg Lys Asp Phe
        1155                1160                1165
Gln Phe Tyr Lys Val Arg Glu Ile Asn Asn Tyr His His Ala His Asp
    1170                1175                1180
Ala Tyr Leu Asn Ala Val Val Gly Thr Ala Leu Ile Lys Lys Tyr Pro
1185                1190                1195                1200
Lys Leu Glu Ser Glu Phe Val Tyr Gly Asp Tyr Lys Val Tyr Asp Val
                1205                1210                1215
Arg Lys Met Ile Ala Lys Ser Glu Gln Glu Ile Gly Lys Ala Thr Ala
            1220                1225                1230
Lys Tyr Phe Phe Tyr Ser Asn Ile Met Asn Phe Phe Lys Thr Glu Ile
        1235                1240                1245
Thr Leu Ala Asn Gly Glu Ile Arg Lys Arg Pro Leu Ile Glu Thr Asn
    1250                1255                1260
Gly Glu Thr Gly Glu Ile Val Trp Asp Lys Gly Arg Asp Phe Ala Thr
1265                1270                1275                1280
Val Arg Lys Val Leu Ser Met Pro Gln Val Asn Ile Val Lys Lys Thr
                1285                1290                1295
Glu Val Gln Thr Gly Gly Phe Ser Lys Glu Ser Ile Leu Pro Lys Arg
            1300                1305                1310
Asn Ser Asp Lys Leu Ile Ala Arg Lys Lys Asp Trp Asp Pro Lys Lys
        1315                1320                1325
Tyr Gly Gly Phe Asp Ser Pro Thr Val Ala Tyr Ser Val Leu Val Val
    1330                1335                1340
Ala Lys Val Glu Lys Gly Lys Ser Lys Lys Leu Lys Ser Val Lys Glu
1345                1350                1355                1360
Leu Leu Gly Ile Thr Ile Met Glu Arg Ser Ser Phe Glu Lys Asn Pro
                1365                1370                1375
Ile Asp Phe Leu Glu Ala Lys Gly Tyr Lys Glu Val Lys Lys Asp Leu
            1380                1385                1390
Ile Ile Lys Leu Pro Lys Tyr Ser Leu Phe Glu Leu Glu Asn Gly Arg
        1395                1400                1405
Lys Arg Met Leu Ala Ser Ala Gly Glu Leu Gln Lys Gly Asn Glu Leu
    1410                1415                1420
Ala Leu Pro Ser Lys Tyr Val Asn Phe Leu Tyr Leu Ala Ser His Tyr
1425                1430                1435                1440
Glu Lys Leu Lys Gly Ser Pro Glu Asp Asn Glu Gln Lys Gln Leu Phe
                1445                1450                1455
Val Glu Gln His Lys His Tyr Leu Asp Glu Ile Ile Glu Gln Ile Ser
            1460                1465                1470
Glu Phe Ser Lys Arg Val Ile Leu Ala Asp Ala Asn Leu Asp Lys Val
        1475                1480                1485
Leu Ser Ala Tyr Asn Lys His Arg Asp Lys Pro Ile Arg Glu Gln Ala
    1490                1495                1500
Glu Asn Ile Ile His Leu Phe Thr Leu Thr Asn Leu Gly Ala Pro Ala
1505                1510                1515                1520
Ala Phe Lys Tyr Phe Asp Thr Thr Ile Asp Arg Lys Arg Tyr Thr Ser
                1525                1530                1535
Thr Lys Glu Val Leu Asp Ala Thr Leu Ile His Gln Ser Ile Thr Gly
            1540                1545                1550
Leu Tyr Glu Thr Arg Ile Asp Leu Ser Gln Leu Gly Gly Asp Ser Gly
        1555                1560                1565
Gly Ser Thr Asn Leu Ser Asp Ile Ile Glu Lys Glu Thr Gly Lys Gln
    1570                1575                1580
Leu Val Ile Gln Glu Ser Ile Leu Met Leu Pro Glu Glu Val Glu Glu
1585                1590                1595                1600
Val Ile Gly Asn Lys Pro Glu Ser Asp Ile Leu Val His Thr Ala Tyr
                1605                1610                1615
Asp Glu Ser Thr Asp Glu Asn Val Met Leu Leu Thr Ser Asp Ala Pro
            1620                1625                1630
Glu Tyr Lys Pro Trp Ala Leu Val Ile Gln Asp Ser Asn Gly Glu Asn
        1635                1640                1645
Lys Ile Lys Met Leu Ser Gly Gly Ser Pro Lys Lys Lys Arg Lys Val

1650                1655                1660
His  His His His His His
1665                1670

<210>  24
<211>  4989
<212>  DNA
<213>  Artificial Sequence

<220>
<223>  Coding sequence of dcas9-AID

<400>  24
atggactata aggaccacga cggagactac aaggatcatg atattgatta caaagacgat      60
gacgataaga tggccccaaa gaagaagcgg aaggtcggta tccacggagt cccagcagct     120
accatggaca agaagtattc tatcggactg gccatcggga ctaatagcgt cgggtgggcc     180
gtgatcactg acgagtacaa ggtgccctct aagaagttca aggtgctcgg gaacaccgac     240
cggcattcca tcaagaaaaa tctgatcgga gctctcctct ttgattcagg ggagaccgct     300
gaagcaaccc gcctcaagcg gactgctaga cggcggtaca ccaggaggaa gaaccggatt     360
tgttaccttc aagagatatt ctccaacgaa atggcaaagg tcgacgacag cttcttccat     420
aggctggaag aatcattcct cgtggaagag gataagaagc atgaacggca tcccatcttc     480
ggtaatatcg tcgacgaggt ggcctatcac gagaaatacc caaccatcta ccatcttcgc     540
aaaaagctgg tggactcaac cgacaaggca gacctccggc ttatctacct ggccctggcc     600
cacatgatca agttcagagg ccacttcctg atcgagggcg acctcaatcc tgacaatagc     660
gatgtggata aactgttcat ccagctggtg cagacttaca accagctctt tgaagagaac     720
cccatcaatg caagcggagt cgatgccaag gccattctgt cagcccggct gtcaaagagc     780
cgcagacttg agaatcttat cgctcagctg ccgggtgaaa agaaaaatgg actgttcggg     840
aacctgattg ctctttcact tgggctgact cccaatttca agtctaattt cgacctggca     900
gaggatgcca agctgcaact gtccaaggac acctatgatg acgatctcga caacctcctg     960
gcccagatcg gtgaccaata cgccgacctt ttccttgctg ctaagaatct ttctgacgcc    1020
atcctgctgt ctgacattct ccgcgtgaac actgaaatca ccaaggcccc tctttcagct    1080
tcaatgatta agcggtatga tgagcaccac caggacctga ccctgcttaa ggcactcgtc    1140
cggcagcagc ttccggagaa gtacaaggaa atcttctttg accagtcaaa gaatggatac    1200
gccggctaca tcgacggagg tgcctcccaa gaggaatttt ataagtttat caaacctatc    1260
cttgagaaga tggacggcac cgaagagctc ctcgtgaaac tgaatcggga ggatctgctg    1320
cggaagcagc gcactttcga caatgggagc attccccacc agatccatct tggggagctt    1380
cacgccatcc ttcggcgcca agaggacttc tacccctttc ttaaggacaa cagggagaag    1440
attgagaaaa ttctcacttt ccgcatcccc tactacgtgg gacccctcgc cagaggaaat    1500
agccggtttg cttggatgac cagaaagtca gaagaaacta tcactccctg gaacttcgaa    1560
gaggtggtgg acaagggagc cagcgctcag tcattcatcg aacggatgac taacttcgat    1620
aagaacctcc ccaatgagaa ggtcctgccg aaacattccc tgctctacga gtactttacc    1680
gtgtacaacg agctgaccaa ggtgaaatat gtcaccgaag ggatgaggaa gcccgcattc    1740
ctgtcaggcg aacaaaagaa ggcaattgtg gaccttctgt tcaagaccaa tagaaaggtg    1800
accgtgaagc agctgaagga ggactatttc aagaaaattg aatgcttcga ctctgtggag    1860
attagcgggg tcgaagatcg gttcaacgca agcctgggta cctaccatga tctgcttaag    1920
atcatcaagg acaaggattt tctggacaat gaggagaacg aggacatcct tgaggacatt    1980
gtcctgactc tcactctgtt cgaggaccgg gaaatgatcg aggagaggct taagacctac    2040
gcccatctgt tcgacgataa agtgatgaag caacttaaac ggagaagata taccggatgg    2100
ggacgcctta gccgcaaact catcaacgga atccgggaca aacagagcgg aaagaccatt    2160
cttgatttcc ttaagagcga cggattcgct aatcgcaact tcatgcaact tatccatgat    2220
gattccctga cctttaagga ggacatccag aaggcccaag tgtctggaca aggtgactca    2280
ctgcacgagc atatcgcaaa tctggctggt tcacccgcta ttaagaaggg tattctccag    2340
accgtgaaag tcgtggacga gctggtcaag gtgatgggtc gccataaacc agagaacatt    2400
gtcatcgaga tggccaggga aaaccagact acccagaagg gacagaagaa cagcagggag    2460
cggatgaaaa gaattgagga agggattaag gagctcgggt cacagatcct taaagagcac    2520
ccggtggaaa acacccagct tcagaatgag aagctctatc tgtactacct tcaaaatgga    2580
cgcgatatgt atgtggacca agagcttgat atcaacaggc tctcagacta cgacgtggac    2640
gccatcgtcc ctcagagctt cctcaaagac gactcaattg acaataaggt gctgactcgc    2700
tcagacaaga accggggaaa gtcagataac gtgccctcag aggaagtcgt gaaaaagatg    2760
aagaactatt ggcgccagct tctgaacgca aagctgatca ctcagcggaa gttcgacaat    2820
ctcactaagg ctgagagggg cggactgagc gaactggaca aagcaggatt cattaaacgg    2880
caacttgtgg agactcggca gattactaaa catgtcgccc aaatccttga ctcacgcatg    2940
aataccaagt acgacgaaaa cgacaaactt atccgcgagg tgaaggtgat taccctgaag    3000

tccaagctgg tcagcgattt cagaaaggac tttcaattct acaaagtgcg ggagatcaat 3060
aactatcatc atgctcatga cgcatatctg aatgccgtgg tgggaaccgc cctgatcaag 3120
aagtacccaa agctggaaag cgagttcgtg tacggagact acaaggtcta cgacgtgcgc 3180
aagatgattg ccaaatctga gcaggagatc ggaaaggcca ccgcaaagta cttcttctac 3240
agcaacatca tgaatttctt caagaccgaa atcacccttg caaacggtga gatccggaag 3300
aggccgctca tcgagactaa tggggagact ggcgaaatcg tgtgggacaa gggcagagat 3360
ttcgctaccg tgcgcaaagt gctttctatg cctcaagtga acatcgtgaa gaaaaccgag 3420
gtgcaaaccg gaggcttttc taaggaatca atcctcccca agcgcaactc cgacaagctc 3480
attgcaagga agaaggattg ggaccctaag aagtacggcg gattcgattc accaactgtg 3540
gcttattctg tcctggtcgt ggctaaggtg gaaaaaggaa agtctaagaa gctcaagagc 3600
gtgaaggaac tgctgggtat caccattatg gagcgcagct ccttcgagaa gaacccaatt 3660
gactttctcg aagccaaagg ttacaaggaa gtcaagaagg accttatcat caagctccca 3720
aagtatagcc tgttcgaact ggagaatggg cggaagcgga tgctcgcctc cgctggcgaa 3780
cttcagaagg gtaatgagct ggctctcccc tccaagtacg tgaatttcct ctaccttgca 3840
agccattacg agaagctgaa ggggagcccc gaggacaacg agcaaaagca actgtttgtg 3900
gagcagcata agcattatct ggacgagatc attgagcaga tttccgagtt ttctaaacgc 3960
gtcattctcg ctgatgccaa cctcgataaa gtccttagcg catacaataa gcacagagac 4020
aaaccaattc gggagcaggc tgagaatatc atccacctgt tcaccctcac caatcttggt 4080
gcccctgccg cattcaagta cttcgacacc accatcgacc ggaaacgcta tacctccacc 4140
aaagaagtgc tggacgccac cctcatccac cagagcatca ccggacttta cgaaactcgg 4200
attgacctct cacagctcgg aggggatgag ggagctccca agaaaaagcg caaggtaggt 4260
agttccggat ctccgaaaaa gaaacgcaaa gttggtagtg atgctttaga cgattttgac 4320
ttagatatgc ttggttcaga cgcgttagac gacttcggtg gaggatccat ggacagcctc 4380
ttgatgaacc ggaggaagtt tctttaccaa ttcaaaaatg tccgctgggc taagggtcgg 4440
cgtgagacct acctgtgcta cgtagtgaag aggcgtgaca gtgctacatc cttttcactg 4500
gactttggtt atcttcgcaa taagaacggc tgccacgtgg aattgctctt cctccgctac 4560
atctcggact gggacctaga ccctggccgc tgctaccgcg tcacctggtt cacctcctgg 4620
agcccctgct acgactgtgc ccgacatgtg gccgactttc tgcgagggaa ccccaacctc 4680
agtctgagga tcttcaccgc gcgcctctac ttctgtgagg accgcaaggc tgagcccgag 4740
gggctgcggc ggctgcaccg cgccggggtg caaatagcca tcatgacctt caaagattat 4800
ttttactgct ggaatacttt tgtagaaaac catgaaagaa ctttcaaagc ctgggaaggg 4860
ctgcatgaaa attcagttcg tctctccaga cagcttcggc gcatcctttt gcccctgtat 4920
gaggttgatg acttacgaga cgcatttcgt acttggggac gtgattacaa agacgatgac 4980
gataagtga 4989

<210> 25
<211> 1662
<212> PRT
<213> Artificial Sequence

<220>
<223> Amino acid sequence of dcas9-AID

<400> 25
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Gly Val Pro Ala Ala Thr Met Asp Lys Lys Tyr Ser Ile
        35                  40                  45
Gly Leu Ala Ile Gly Thr Asn Ser Val Gly Trp Ala Val Ile Thr Asp
    50                  55                  60
Glu Tyr Lys Val Pro Ser Lys Lys Phe Lys Val Leu Gly Asn Thr Asp
65                  70                  75                  80
Arg His Ser Ile Lys Lys Asn Leu Ile Gly Ala Leu Leu Phe Asp Ser
                85                  90                  95
Gly Glu Thr Ala Glu Ala Thr Arg Leu Lys Arg Thr Ala Arg Arg Arg
            100                 105                 110
Tyr Thr Arg Arg Lys Asn Arg Ile Cys Tyr Leu Gln Glu Ile Phe Ser
        115                 120                 125
Asn Glu Met Ala Lys Val Asp Asp Ser Phe Phe His Arg Leu Glu Glu
    130                 135                 140
Ser Phe Leu Val Glu Glu Asp Lys Lys His Glu Arg His Pro Ile Phe

145                 150                 155                 160
Gly Asn Ile Val Asp Glu Val Ala Tyr His Glu Lys Tyr Pro Thr Ile
                165                 170                 175
Tyr His Leu Arg Lys Lys Leu Val Asp Ser Thr Asp Lys Ala Asp Leu
            180                 185                 190
Arg Leu Ile Tyr Leu Ala Leu Ala His Met Ile Lys Phe Arg Gly His
        195                 200                 205
Phe Leu Ile Glu Gly Asp Leu Asn Pro Asp Asn Ser Asp Val Asp Lys
    210                 215                 220
Leu Phe Ile Gln Leu Val Gln Thr Tyr Asn Gln Leu Phe Glu Glu Asn
225                 230                 235                 240
Pro Ile Asn Ala Ser Gly Val Asp Ala Lys Ala Ile Leu Ser Ala Arg
                245                 250                 255
Leu Ser Lys Ser Arg Arg Leu Glu Asn Leu Ile Ala Gln Leu Pro Gly
            260                 265                 270
Glu Lys Lys Asn Gly Leu Phe Gly Asn Leu Ile Ala Leu Ser Leu Gly
        275                 280                 285
Leu Thr Pro Asn Phe Lys Ser Asn Phe Asp Leu Ala Glu Asp Ala Lys
    290                 295                 300
Leu Gln Leu Ser Lys Asp Thr Tyr Asp Asp Asp Leu Asp Asn Leu Leu
305                 310                 315                 320
Ala Gln Ile Gly Asp Gln Tyr Ala Asp Leu Phe Leu Ala Ala Lys Asn
                325                 330                 335
Leu Ser Asp Ala Ile Leu Leu Ser Asp Ile Leu Arg Val Asn Thr Glu
            340                 345                 350
Ile Thr Lys Ala Pro Leu Ser Ala Ser Met Ile Lys Arg Tyr Asp Glu
        355                 360                 365
His His Gln Asp Leu Thr Leu Leu Lys Ala Leu Val Arg Gln Gln Leu
    370                 375                 380
Pro Glu Lys Tyr Lys Glu Ile Phe Phe Asp Gln Ser Lys Asn Gly Tyr
385                 390                 395                 400
Ala Gly Tyr Ile Asp Gly Gly Ala Ser Gln Glu Glu Phe Tyr Lys Phe
                405                 410                 415
Ile Lys Pro Ile Leu Glu Lys Met Asp Gly Thr Glu Glu Leu Leu Val
            420                 425                 430
Lys Leu Asn Arg Glu Asp Leu Leu Arg Lys Gln Arg Thr Phe Asp Asn
        435                 440                 445
Gly Ser Ile Pro His Gln Ile His Leu Gly Glu Leu His Ala Ile Leu
    450                 455                 460
Arg Arg Gln Glu Asp Phe Tyr Pro Phe Leu Lys Asp Asn Arg Glu Lys
465                 470                 475                 480
Ile Glu Lys Ile Leu Thr Phe Arg Ile Pro Tyr Tyr Val Gly Pro Leu
                485                 490                 495
Ala Arg Gly Asn Ser Arg Phe Ala Trp Met Thr Arg Lys Ser Glu Glu
            500                 505                 510
Thr Ile Thr Pro Trp Asn Phe Glu Glu Val Val Asp Lys Gly Ala Ser
        515                 520                 525
Ala Gln Ser Phe Ile Glu Arg Met Thr Asn Phe Asp Lys Asn Leu Pro
    530                 535                 540
Asn Glu Lys Val Leu Pro Lys His Ser Leu Leu Tyr Glu Tyr Phe Thr
545                 550                 555                 560
Val Tyr Asn Glu Leu Thr Lys Val Lys Tyr Val Thr Glu Gly Met Arg
                565                 570                 575
Lys Pro Ala Phe Leu Ser Gly Glu Gln Lys Lys Ala Ile Val Asp Leu
            580                 585                 590
Leu Phe Lys Thr Asn Arg Lys Val Thr Val Lys Gln Leu Lys Glu Asp
        595                 600                 605
Tyr Phe Lys Lys Ile Glu Cys Phe Asp Ser Val Glu Ile Ser Gly Val
    610                 615                 620
Glu Asp Arg Phe Asn Ala Ser Leu Gly Thr Tyr His Asp Leu Leu Lys
625                 630                 635                 640
Ile Ile Lys Asp Lys Asp Phe Leu Asp Asn Glu Glu Asn Glu Asp Ile
                645                 650                 655

Leu Glu Asp Ile Val Leu Thr Leu Thr Leu Phe Glu Asp Arg Glu Met
            660                 665                 670
Ile Glu Glu Arg Leu Lys Thr Tyr Ala His Leu Phe Asp Asp Lys Val
        675                 680                 685
Met Lys Gln Leu Lys Arg Arg Arg Tyr Thr Gly Trp Gly Arg Leu Ser
    690                 695                 700
Arg Lys Leu Ile Asn Gly Ile Arg Asp Lys Gln Ser Gly Lys Thr Ile
705                 710                 715                 720
Leu Asp Phe Leu Lys Ser Asp Gly Phe Ala Asn Arg Asn Phe Met Gln
                725                 730                 735
Leu Ile His Asp Asp Ser Leu Thr Phe Lys Glu Asp Ile Gln Lys Ala
            740                 745                 750
Gln Val Ser Gly Gln Gly Asp Ser Leu His Glu His Ile Ala Asn Leu
        755                 760                 765
Ala Gly Ser Pro Ala Ile Lys Lys Gly Ile Leu Gln Thr Val Lys Val
    770                 775                 780
Val Asp Glu Leu Val Lys Val Met Gly Arg His Lys Pro Glu Asn Ile
785                 790                 795                 800
Val Ile Glu Met Ala Arg Glu Asn Gln Thr Thr Gln Lys Gly Gln Lys
                805                 810                 815
Asn Ser Arg Glu Arg Met Lys Arg Ile Glu Glu Gly Ile Lys Glu Leu
            820                 825                 830
Gly Ser Gln Ile Leu Lys Glu His Pro Val Glu Asn Thr Gln Leu Gln
        835                 840                 845
Asn Glu Lys Leu Tyr Leu Tyr Tyr Leu Gln Asn Gly Arg Asp Met Tyr
    850                 855                 860
Val Asp Gln Glu Leu Asp Ile Asn Arg Leu Ser Asp Tyr Asp Val Asp
865                 870                 875                 880
Ala Ile Val Pro Gln Ser Phe Leu Lys Asp Asp Ser Ile Asp Asn Lys
                885                 890                 895
Val Leu Thr Arg Ser Asp Lys Asn Arg Gly Lys Ser Asp Asn Val Pro
            900                 905                 910
Ser Glu Glu Val Val Lys Lys Met Lys Asn Tyr Trp Arg Gln Leu Leu
        915                 920                 925
Asn Ala Lys Leu Ile Thr Gln Arg Lys Phe Asp Asn Leu Thr Lys Ala
    930                 935                 940
Glu Arg Gly Gly Leu Ser Glu Leu Asp Lys Ala Gly Phe Ile Lys Arg
945                 950                 955                 960
Gln Leu Val Glu Thr Arg Gln Ile Thr Lys His Val Ala Gln Ile Leu
                965                 970                 975
Asp Ser Arg Met Asn Thr Lys Tyr Asp Glu Asn Asp Lys Leu Ile Arg
            980                 985                 990
Glu Val Lys Val Ile Thr Leu Lys Ser Lys Leu Val Ser Asp Phe Arg
        995                 1000                1005
Lys Asp Phe Gln Phe Tyr Lys Val Arg Glu Ile Asn Asn Tyr His His
    1010                1015                1020
Ala His Asp Ala Tyr Leu Asn Ala Val Val Gly Thr Ala Leu Ile Lys
1025                1030                1035                1040
Lys Tyr Pro Lys Leu Glu Ser Glu Phe Val Tyr Gly Asp Tyr Lys Val
                1045                1050                1055
Tyr Asp Val Arg Lys Met Ile Ala Lys Ser Glu Gln Glu Ile Gly Lys
            1060                1065                1070
Ala Thr Ala Lys Tyr Phe Phe Tyr Ser Asn Ile Met Asn Phe Phe Lys
        1075                1080                1085
Thr Glu Ile Thr Leu Ala Asn Gly Glu Ile Arg Lys Arg Pro Leu Ile
    1090                1095                1100
Glu Thr Asn Gly Glu Thr Gly Glu Ile Val Trp Asp Lys Gly Arg Asp
1105                1110                1115                1120
Phe Ala Thr Val Arg Lys Val Leu Ser Met Pro Gln Val Asn Ile Val
                1125                1130                1135
Lys Lys Thr Glu Val Gln Thr Gly Gly Phe Ser Lys Glu Ser Ile Leu
            1140                1145                1150
Pro Lys Arg Asn Ser Asp Lys Leu Ile Ala Arg Lys Lys Asp Trp Asp

1155                1160                1165
Pro Lys Lys Tyr Gly Gly Phe Asp Ser Pro Thr Val Ala Tyr Ser Val
    1170                1175                1180
Leu Val Val Ala Lys Val Glu Lys Gly Lys Ser Lys Lys Leu Lys Ser
1185                1190                1195                1200
Val Lys Glu Leu Leu Gly Ile Thr Ile Met Glu Arg Ser Ser Phe Glu
                1205                1210                1215
Lys Asn Pro Ile Asp Phe Leu Glu Ala Lys Gly Tyr Lys Glu Val Lys
            1220                1225                1230
Lys Asp Leu Ile Ile Lys Leu Pro Lys Tyr Ser Leu Phe Glu Leu Glu
        1235                1240                1245
Asn Gly Arg Lys Arg Met Leu Ala Ser Ala Gly Glu Leu Gln Lys Gly
    1250                1255                1260
Asn Glu Leu Ala Leu Pro Ser Lys Tyr Val Asn Phe Leu Tyr Leu Ala
1265                1270                1275                1280
Ser His Tyr Glu Lys Leu Lys Gly Ser Pro Glu Asp Asn Glu Gln Lys
                1285                1290                1295
Gln Leu Phe Val Glu Gln His Lys His Tyr Leu Asp Glu Ile Ile Glu
            1300                1305                1310
Gln Ile Ser Glu Phe Ser Lys Arg Val Ile Leu Ala Asp Ala Asn Leu
        1315                1320                1325
Asp Lys Val Leu Ser Ala Tyr Asn Lys His Arg Asp Lys Pro Ile Arg
    1330                1335                1340
Glu Gln Ala Glu Asn Ile Ile His Leu Phe Thr Leu Thr Asn Leu Gly
1345                1350                1355                1360
Ala Pro Ala Ala Phe Lys Tyr Phe Asp Thr Thr Ile Asp Arg Lys Arg
                1365                1370                1375
Tyr Thr Ser Thr Lys Glu Val Leu Asp Ala Thr Leu Ile His Gln Ser
            1380                1385                1390
Ile Thr Gly Leu Tyr Glu Thr Arg Ile Asp Leu Ser Gln Leu Gly Gly
        1395                1400                1405
Asp Glu Gly Ala Pro Lys Lys Lys Arg Lys Val Gly Ser Ser Gly Ser
    1410                1415                1420
Pro Lys Lys Lys Arg Lys Val Gly Ser Asp Ala Leu Asp Asp Phe Asp
1425                1430                1435                1440
Leu Asp Met Leu Gly Ser Asp Ala Leu Asp Asp Phe Gly Gly Gly Ser
                1445                1450                1455
Met Asp Ser Leu Leu Met Asn Arg Arg Lys Phe Leu Tyr Gln Phe Lys
            1460                1465                1470
Asn Val Arg Trp Ala Lys Gly Arg Arg Glu Thr Tyr Leu Cys Tyr Val
        1475                1480                1485
Val Lys Arg Arg Asp Ser Ala Thr Ser Phe Ser Leu Asp Phe Gly Tyr
    1490                1495                1500
Leu Arg Asn Lys Asn Gly Cys His Val Glu Leu Leu Phe Leu Arg Tyr
1505                1510                1515                1520
Ile Ser Asp Trp Asp Leu Asp Pro Gly Arg Cys Tyr Arg Val Thr Trp
                1525                1530                1535
Phe Thr Ser Trp Ser Pro Cys Tyr Asp Cys Ala Arg His Val Ala Asp
            1540                1545                1550
Phe Leu Arg Gly Asn Pro Asn Leu Ser Leu Arg Ile Phe Thr Ala Arg
        1555                1560                1565
Leu Tyr Phe Cys Glu Asp Arg Lys Ala Glu Pro Glu Gly Leu Arg Arg
    1570                1575                1580
Leu His Arg Ala Gly Val Gln Ile Ala Ile Met Thr Phe Lys Asp Tyr
1585                1590                1595                1600
Phe Tyr Cys Trp Asn Thr Phe Val Glu Asn His Glu Arg Thr Phe Lys
                1605                1610                1615
Ala Trp Glu Gly Leu His Glu Asn Ser Val Arg Leu Ser Arg Gln Leu
            1620                1625                1630
Arg Arg Ile Leu Leu Pro Leu Tyr Glu Val Asp Asp Leu Arg Asp Ala
        1635                1640                1645
Phe Arg Thr Trp Gly Arg Asp Tyr Lys Asp Asp Asp Asp Lys
    1650                1655                1660

<210>  26
<211>  4941
<212>  DNA
<213>  Artificial Sequence

<220>
<223>  Coding sequence of dcas9-aidm

<400>  26
atggactata aggaccacga cggagactac aaggatcatg atattgatta caaagacgat      60
gacgataaga tggccccaaa gaagaagcgg aaggtcggta tccacggagt cccagcagct     120
accatggaca agaagtattc tatcggactg gccatcggga ctaatagcgt cgggtgggcc     180
gtgatcactg acgagtacaa ggtgccctct aagaagttca aggtgctcgg gaacaccgac     240
cggcattcca tcaagaaaaa tctgatcgga gctctcctct ttgattcagg ggagaccgct     300
gaagcaaccc gcctcaagcg gactgctaga cggcggtaca ccaggaggaa gaaccggatt     360
tgttaccttc aagagatatt ctccaacgaa atggcaaagg tcgacgacag cttcttccat     420
aggctggaag aatcattcct cgtggaagag gataagaagc atgaacggca tcccatcttc     480
ggtaatatcg tcgacgaggt ggcctatcac gagaaatacc caaccatcta ccatcttcgc     540
aaaaagctgg tggactcaac cgacaaggca gacctccggc ttatctacct ggccctggcc     600
cacatgatca agttcagagg ccacttcctg atcgagggcg acctcaatcc tgacaatagc     660
gatgtggata aactgttcat ccagctggtg cagacttaca accagctctt tgaagagaac     720
cccatcaatg caagcggagt cgatgccaag gccattctgt cagcccggct gtcaaagagc     780
cgcagacttg agaatcttat cgctcagctg ccgggtgaaa agaaaaatgg actgttcggg     840
aacctgattg ctctttcact tgggctgact cccaatttca agtctaattt cgacctggca     900
gaggatgcca agctgcaact gtccaaggac acctatgatg acgatctcga caacctcctg     960
gcccagatcg gtgaccaata cgccgacctt ttccttgctg ctaagaatct ttctgacgcc    1020
atcctgctgt ctgacattct ccgcgtgaac actgaaatca ccaaggcccc tctttcagct    1080
tcaatgatta agcggtatga tgagcaccac caggacctga ccctgcttaa ggcactcgtc    1140
cggcagcagc ttccggagaa gtacaaggaa atcttctttg accagtcaaa gaatggatac    1200
gccggctaca tcgacggagg tgcctcccaa gaggaatttt ataagtttat caaacctatc    1260
cttgagaaga tggacggcac cgaagagctc ctcgtgaaac tgaatcggga ggatctgctg    1320
cggaagcagc gcactttcga caatgggagc attccccacc agatccatct tggggagctt    1380
cacgccatcc ttcggcgcca agaggacttc tacccctttc ttaaggacaa cagggagaag    1440
attgagaaaa ttctcacttt ccgcatcccc tactacgtgg gacccctcgc cagaggaaat    1500
agccggtttg cttggatgac cagaaagtca gaagaaacta tcactccctg gaacttcgaa    1560
gaggtggtgg acaagggagc cagcgctcag tcattcatcg aacggatgac taacttcgat    1620
aagaacctcc ccaatgagaa ggtcctgccg aaacattccc tgctctacga gtactttacc    1680
gtgtacaacg agctgaccaa ggtgaaatat gtcaccgaag ggatgaggaa gcccgcattc    1740
ctgtcaggcg aacaaaagaa ggcaattgtg gaccttctgt tcaagaccaa tagaaaggtg    1800
accgtgaagc agctgaagga ggactatttc aagaaaattg aatgcttcga ctctgtggag    1860
attagcgggg tcgaagatcg gttcaacgca agcctgggta cctaccatga tctgcttaag    1920
atcatcaagg acaaggattt tctggacaat gaggagaacg aggacatcct tgaggacatt    1980
gtcctgactc tcactctgtt cgaggaccgg gaaatgatcg aggagaggct taagacctac    2040
gcccatctgt tcgacgataa agtgatgaag caacttaaac ggagaagata taccggatgg    2100
ggacgcctta gccgcaaact catcaacgga atccgggaca aacagagcgg aaagaccatt    2160
cttgatttcc ttaagagcga cggattcgct aatcgcaact tcatgcaact tatccatgat    2220
gattccctga cctttaagga ggacatccag aaggcccaag tgtctggaca aggtgactca    2280
ctgcacgagc atatcgcaaa tctggctggt tcacccgcta ttaagaaggg tattctccag    2340
accgtgaaag tcgtggacga gctggtcaag gtgatgggtc gccataaacc agagaacatt    2400
gtcatcgaga tggccaggga aaaccagact acccagaagg gacagaagaa cagcagggag    2460
cggatgaaaa gaattgagga agggattaag gagctcgggt cacagatcct taaagagcac    2520
ccggtggaaa acacccagct tcagaatgag aagctctatc tgtactacct tcaaaatgga    2580
cgcgatatgt atgtggacca agagcttgat atcaacaggc tctcagacta cgacgtggac    2640
gccatcgtcc ctcagagctt cctcaaagac gactcaattg acaataaggt gctgactcgc    2700
tcagacaaga accggggaaa gtcagataac gtgccctcag aggaagtcgt gaaaaagatg    2760
aagaactatt ggcgccagct tctgaacgca aagctgatca ctcagcggaa gttcgacaat    2820
ctcactaagg ctgagagggg cggactgagc gaactggaca aagcaggatt cattaaacgg    2880
caacttgtgg agactcggca gattactaaa catgtcgccc aaatccttga ctcacgcatg    2940
aataccaagt acgacgaaaa cgacaaactt atccgcgagg tgaaggtgat taccctgaag    3000
tccaagctgg tcagcgattt cagaaaggac tttcaattct acaaagtgcg ggagatcaat    3060
aactatcatc atgctcatga cgcatatctg aatgccgtgg tgggaaccgc cctgatcaag    3120
aagtacccaa agctggaaag cgagttcgtg tacggagact acaaggtcta cgacgtgcgc    3180

aagatgattg ccaaatctga gcaggagatc ggaaaggcca ccgcaaagta cttcttctac 3240
agcaacatca tgaatttctt caagaccgaa atcacccttg caaacggtga gatccggaag 3300
aggccgctca tcgagactaa tggggagact ggcgaaatcg tgtgggacaa gggcagagat 3360
ttcgctaccg tgcgcaaagt gctttctatg cctcaagtga acatcgtgaa gaaaaccgag 3420
gtgcaaaccg gaggcttttc taaggaatca atcctcccca agcgcaactc cgacaagctc 3480
attgcaagga agaaggattg ggaccctaag aagtacggcg gattcgattc accaactgtg 3540
gcttattctg tcctggtcgt ggctaaggtg gaaaaaggaa agtctaagaa gctcaagagc 3600
gtgaaggaac tgctgggtat caccattatg gagcgcagct ccttcgagaa gaacccaatt 3660
gactttctcg aagccaaagg ttacaaggaa gtcaagaagg accttatcat caagctccca 3720
aagtatagcc tgttcgaact ggagaatggg cggaagcgga tgctcgcctc cgctggcgaa 3780
cttcagaagg gtaatgagct ggctctcccc tccaagtacg tgaatttcct ctaccttgca 3840
agccattacg agaagctgaa ggggagcccc gaggacaacg agcaaaagca actgtttgtg 3900
gagcagcata agcattatct ggacgagatc attgagcaga tttccgagtt ttctaaacgc 3960
gtcattctcg ctgatgccaa cctcgataaa gtccttagcg catacaataa gcacagagac 4020
aaaccaattc gggagcaggc tgagaatatc atccacctgt tcaccctcac caatcttggt 4080
gcccctgccg cattcaagta cttcgacacc accatcgacc ggaaacgcta tacctccacc 4140
aaagaagtgc tggacgccac cctcatccac cagagcatca ccggacttta cgaaactcgg 4200
attgacctct cacagctcgg aggggatgag ggagctccca agaaaaagcg caaggtaggt 4260
agttccggat ctccgaaaaa gaaacgcaaa gttggtagtg atgctttaga cgattttgac 4320
ttagatatgc ttggttcaga cgcgttagac gacttcggtg gaggatccat ggacagcctc 4380
ttgatgaacc ggaggaagtt tctttaccaa ttcaaaaatg tccgctgggc taagggtcgg 4440
cgtgagacct acctgtgcta cgtagtgaag aggcgtgaca gtgctacatc cttttcactg 4500
gactttggtt atcttcgcaa taagaacggc tgccacgtgg aattgctctt cctccgctac 4560
atctcggact gggacctaga ccctggccgc tgctaccgcg tcacctggtt cacctcctgg 4620
agcccctgct acgactgtgc ccgacatgtg gccgactttc tgcgagggaa ccccaacctc 4680
agtctgagga tcttcaccgc gcgcctctac ttctgtgagg accgcaaggc tgagcccgag 4740
gggctgcggc ggctgcaccg cgccggggtg caaatagcca tcatgacctt caaagattat 4800
ttttactgct ggaatacttt tgtagaaaac catgaaagaa ctttcaaagc ctgggaaggg 4860
ctgcatgaaa attcagttcg tctctccaga cagcttcggc gcatcctttt gcccgattac 4920
aaagacgatg acgataagtg a 4941

<210> 27
<211> 1646
<212> PRT
<213> Artificial Sequence

<220>
<223> Amino acid sequence of dcas9-aidm

<400> 27
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Gly Val Pro Ala Ala Thr Met Asp Lys Lys Tyr Ser Ile
        35                  40                  45
Gly Leu Ala Ile Gly Thr Asn Ser Val Gly Trp Ala Val Ile Thr Asp
    50                  55                  60
Glu Tyr Lys Val Pro Ser Lys Lys Phe Lys Val Leu Gly Asn Thr Asp
65                  70                  75                  80
Arg His Ser Ile Lys Lys Asn Leu Ile Gly Ala Leu Leu Phe Asp Ser
                85                  90                  95
Gly Glu Thr Ala Glu Ala Thr Arg Leu Lys Arg Thr Ala Arg Arg Arg
            100                 105                 110
Tyr Thr Arg Arg Lys Asn Arg Ile Cys Tyr Leu Gln Glu Ile Phe Ser
        115                 120                 125
Asn Glu Met Ala Lys Val Asp Asp Ser Phe Phe His Arg Leu Glu Glu
    130                 135                 140
Ser Phe Leu Val Glu Glu Asp Lys Lys His Glu Arg His Pro Ile Phe
145                 150                 155                 160
Gly Asn Ile Val Asp Glu Val Ala Tyr His Glu Lys Tyr Pro Thr Ile
                165                 170                 175
Tyr His Leu Arg Lys Lys Leu Val Asp Ser Thr Asp Lys Ala Asp Leu

            180                 185                 190
Arg Leu Ile Tyr Leu Ala Leu Ala His Met Ile Lys Phe Arg Gly His
        195                 200                 205
Phe Leu Ile Glu Gly Asp Leu Asn Pro Asp Asn Ser Asp Val Asp Lys
    210                 215                 220
Leu Phe Ile Gln Leu Val Gln Thr Tyr Asn Gln Leu Phe Glu Glu Asn
225                 230                 235                 240
Pro Ile Asn Ala Ser Gly Val Asp Ala Lys Ala Ile Leu Ser Ala Arg
                245                 250                 255
Leu Ser Lys Ser Arg Arg Leu Glu Asn Leu Ile Ala Gln Leu Pro Gly
            260                 265                 270
Glu Lys Lys Asn Gly Leu Phe Gly Asn Leu Ile Ala Leu Ser Leu Gly
        275                 280                 285
Leu Thr Pro Asn Phe Lys Ser Asn Phe Asp Leu Ala Glu Asp Ala Lys
    290                 295                 300
Leu Gln Leu Ser Lys Asp Thr Tyr Asp Asp Asp Leu Asp Asn Leu Leu
305                 310                 315                 320
Ala Gln Ile Gly Asp Gln Tyr Ala Asp Leu Phe Leu Ala Ala Lys Asn
                325                 330                 335
Leu Ser Asp Ala Ile Leu Leu Ser Asp Ile Leu Arg Val Asn Thr Glu
            340                 345                 350
Ile Thr Lys Ala Pro Leu Ser Ala Ser Met Ile Lys Arg Tyr Asp Glu
        355                 360                 365
His His Gln Asp Leu Thr Leu Leu Lys Ala Leu Val Arg Gln Gln Leu
    370                 375                 380
Pro Glu Lys Tyr Lys Glu Ile Phe Phe Asp Gln Ser Lys Asn Gly Tyr
385                 390                 395                 400
Ala Gly Tyr Ile Asp Gly Gly Ala Ser Gln Glu Glu Phe Tyr Lys Phe
                405                 410                 415
Ile Lys Pro Ile Leu Glu Lys Met Asp Gly Thr Glu Glu Leu Leu Val
            420                 425                 430
Lys Leu Asn Arg Glu Asp Leu Leu Arg Lys Gln Arg Thr Phe Asp Asn
        435                 440                 445
Gly Ser Ile Pro His Gln Ile His Leu Gly Glu Leu His Ala Ile Leu
    450                 455                 460
Arg Arg Gln Glu Asp Phe Tyr Pro Phe Leu Lys Asp Asn Arg Glu Lys
465                 470                 475                 480
Ile Glu Lys Ile Leu Thr Phe Arg Ile Pro Tyr Tyr Val Gly Pro Leu
                485                 490                 495
Ala Arg Gly Asn Ser Arg Phe Ala Trp Met Thr Arg Lys Ser Glu Glu
            500                 505                 510
Thr Ile Thr Pro Trp Asn Phe Glu Glu Val Val Asp Lys Gly Ala Ser
        515                 520                 525
Ala Gln Ser Phe Ile Glu Arg Met Thr Asn Phe Asp Lys Asn Leu Pro
    530                 535                 540
Asn Glu Lys Val Leu Pro Lys His Ser Leu Leu Tyr Glu Tyr Phe Thr
545                 550                 555                 560
Val Tyr Asn Glu Leu Thr Lys Val Lys Tyr Val Thr Glu Gly Met Arg
                565                 570                 575
Lys Pro Ala Phe Leu Ser Gly Glu Gln Lys Lys Ala Ile Val Asp Leu
            580                 585                 590
Leu Phe Lys Thr Asn Arg Lys Val Thr Val Lys Gln Leu Lys Glu Asp
        595                 600                 605
Tyr Phe Lys Lys Ile Glu Cys Phe Asp Ser Val Glu Ile Ser Gly Val
    610                 615                 620
Glu Asp Arg Phe Asn Ala Ser Leu Gly Thr Tyr His Asp Leu Leu Lys
625                 630                 635                 640
Ile Ile Lys Asp Lys Asp Phe Leu Asp Asn Glu Glu Asn Glu Asp Ile
                645                 650                 655
Leu Glu Asp Ile Val Leu Thr Leu Thr Leu Phe Glu Asp Arg Glu Met
            660                 665                 670
Ile Glu Glu Arg Leu Lys Thr Tyr Ala His Leu Phe Asp Asp Lys Val
        675                 680                 685

Met Lys Gln Leu Lys Arg Arg Arg Tyr Thr Gly Trp Gly Arg Leu Ser
    690                 695                 700
Arg Lys Leu Ile Asn Gly Ile Arg Asp Lys Gln Ser Gly Lys Thr Ile
705                 710                 715                 720
Leu Asp Phe Leu Lys Ser Asp Gly Phe Ala Asn Arg Asn Phe Met Gln
                725                 730                 735
Leu Ile His Asp Asp Ser Leu Thr Phe Lys Glu Asp Ile Gln Lys Ala
            740                 745                 750
Gln Val Ser Gly Gln Gly Asp Ser Leu His Glu His Ile Ala Asn Leu
        755                 760                 765
Ala Gly Ser Pro Ala Ile Lys Lys Gly Ile Leu Gln Thr Val Lys Val
    770                 775                 780
Val Asp Glu Leu Val Lys Val Met Gly Arg His Lys Pro Glu Asn Ile
785                 790                 795                 800
Val Ile Glu Met Ala Arg Glu Asn Gln Thr Thr Gln Lys Gly Gln Lys
                805                 810                 815
Asn Ser Arg Glu Arg Met Lys Arg Ile Glu Glu Gly Ile Lys Glu Leu
            820                 825                 830
Gly Ser Gln Ile Leu Lys Glu His Pro Val Glu Asn Thr Gln Leu Gln
        835                 840                 845
Asn Glu Lys Leu Tyr Leu Tyr Tyr Leu Gln Asn Gly Arg Asp Met Tyr
    850                 855                 860
Val Asp Gln Glu Leu Asp Ile Asn Arg Leu Ser Asp Tyr Asp Val Asp
865                 870                 875                 880
Ala Ile Val Pro Gln Ser Phe Leu Lys Asp Asp Ser Ile Asp Asn Lys
                885                 890                 895
Val Leu Thr Arg Ser Asp Lys Asn Arg Gly Lys Ser Asp Asn Val Pro
            900                 905                 910
Ser Glu Glu Val Val Lys Lys Met Lys Asn Tyr Trp Arg Gln Leu Leu
        915                 920                 925
Asn Ala Lys Leu Ile Thr Gln Arg Lys Phe Asp Asn Leu Thr Lys Ala
    930                 935                 940
Glu Arg Gly Gly Leu Ser Glu Leu Asp Lys Ala Gly Phe Ile Lys Arg
945                 950                 955                 960
Gln Leu Val Glu Thr Arg Gln Ile Thr Lys His Val Ala Gln Ile Leu
                965                 970                 975
Asp Ser Arg Met Asn Thr Lys Tyr Asp Glu Asn Asp Lys Leu Ile Arg
            980                 985                 990
Glu Val Lys Val Ile Thr Leu Lys  Ser Lys Leu Val Ser  Asp Phe Arg
        995                 1000                 1005
Lys Asp  Phe Gln Phe Tyr Lys  Val Arg Glu Ile Asn  Asn Tyr His His
    1010                 1015                 1020
Ala  His Asp Ala Tyr Leu  Asn Ala Val Val Gly  Thr Ala Leu Ile Lys
1025                 1030                 1035                 1040
Lys Tyr Pro Lys Leu  Glu Ser Glu Phe Val  Tyr Gly Asp Tyr Lys  Val
                1045                 1050                 1055
Tyr Asp Val Arg  Lys Met Ile Ala Lys  Ser Glu Gln Glu Ile  Gly Lys
            1060                 1065                 1070
Ala Thr Ala  Lys Tyr Phe Phe Tyr  Ser Asn Ile Met Asn  Phe Phe Lys
        1075                 1080                 1085
Thr Glu  Ile Thr Leu Ala Asn  Gly Glu Ile Arg Lys  Arg Pro Leu Ile
    1090                 1095                 1100
Glu  Thr Asn Gly Glu Thr  Gly Glu Ile Val Trp  Asp Lys Gly Arg Asp
1105                 1110                 1115                 1120
Phe Ala Thr Val Arg  Lys Val Leu Ser Met  Pro Gln Val Asn Ile  Val
                1125                 1130                 1135
Lys Lys Thr Glu  Val Gln Thr Gly Gly  Phe Ser Lys Glu Ser  Ile Leu
            1140                 1145                 1150
Pro Lys Arg  Asn Ser Asp Lys Leu  Ile Ala Arg Lys Lys  Asp Trp Asp
        1155                 1160                 1165
Pro Lys  Lys Tyr Gly Gly Phe  Asp Ser Pro Thr Val  Ala Tyr Ser Val
    1170                 1175                 1180
Leu  Val Val Ala Lys Val  Glu Lys Gly Lys Ser  Lys Lys Leu Lys Ser

1185                1190                1195                1200
Val Lys Glu Leu Leu Gly Ile Thr Ile Met Glu Arg Ser Ser Phe Glu
                1205                1210                1215
Lys Asn Pro Ile Asp Phe Leu Glu Ala Lys Gly Tyr Lys Glu Val Lys
            1220                1225                1230
Lys Asp Leu Ile Ile Lys Leu Pro Lys Tyr Ser Leu Phe Glu Leu Glu
        1235                1240                1245
Asn Gly Arg Lys Arg Met Leu Ala Ser Ala Gly Glu Leu Gln Lys Gly
    1250                1255                1260
Asn Glu Leu Ala Leu Pro Ser Lys Tyr Val Asn Phe Leu Tyr Leu Ala
1265                1270                1275                1280
Ser His Tyr Glu Lys Leu Lys Gly Ser Pro Glu Asp Asn Glu Gln Lys
                1285                1290                1295
Gln Leu Phe Val Glu Gln His Lys His Tyr Leu Asp Glu Ile Ile Glu
            1300                1305                1310
Gln Ile Ser Glu Phe Ser Lys Arg Val Ile Leu Ala Asp Ala Asn Leu
        1315                1320                1325
Asp Lys Val Leu Ser Ala Tyr Asn Lys His Arg Asp Lys Pro Ile Arg
    1330                1335                1340
Glu Gln Ala Glu Asn Ile Ile His Leu Phe Thr Leu Thr Asn Leu Gly
1345                1350                1355                1360
Ala Pro Ala Ala Phe Lys Tyr Phe Asp Thr Thr Ile Asp Arg Lys Arg
                1365                1370                1375
Tyr Thr Ser Thr Lys Glu Val Leu Asp Ala Thr Leu Ile His Gln Ser
            1380                1385                1390
Ile Thr Gly Leu Tyr Glu Thr Arg Ile Asp Leu Ser Gln Leu Gly Gly
        1395                1400                1405
Asp Glu Gly Ala Pro Lys Lys Lys Arg Lys Val Gly Ser Ser Gly Ser
    1410                1415                1420
Pro Lys Lys Lys Arg Lys Val Gly Ser Asp Ala Leu Asp Asp Phe Asp
1425                1430                1435                1440
Leu Asp Met Leu Gly Ser Asp Ala Leu Asp Asp Phe Gly Gly Gly Ser
                1445                1450                1455
Met Asp Ser Leu Leu Met Asn Arg Arg Lys Phe Leu Tyr Gln Phe Lys
            1460                1465                1470
Asn Val Arg Trp Ala Lys Gly Arg Arg Glu Thr Tyr Leu Cys Tyr Val
        1475                1480                1485
Val Lys Arg Arg Asp Ser Ala Thr Ser Phe Ser Leu Asp Phe Gly Tyr
    1490                1495                1500
Leu Arg Asn Lys Asn Gly Cys His Val Glu Leu Leu Phe Leu Arg Tyr
1505                1510                1515                1520
Ile Ser Asp Trp Asp Leu Asp Pro Gly Arg Cys Tyr Arg Val Thr Trp
                1525                1530                1535
Phe Thr Ser Trp Ser Pro Cys Tyr Asp Cys Ala Arg His Val Ala Asp
            1540                1545                1550
Phe Leu Arg Gly Asn Pro Asn Leu Ser Leu Arg Ile Phe Thr Ala Arg
        1555                1560                1565
Leu Tyr Phe Cys Glu Asp Arg Lys Ala Glu Pro Glu Gly Leu Arg Arg
    1570                1575                1580
Leu His Arg Ala Gly Val Gln Ile Ala Ile Met Thr Phe Lys Asp Tyr
1585                1590                1595                1600
Phe Tyr Cys Trp Asn Thr Phe Val Glu Asn His Glu Arg Thr Phe Lys
                1605                1610                1615
Ala Trp Glu Gly Leu His Glu Asn Ser Val Arg Leu Ser Arg Gln Leu
            1620                1625                1630
Arg Arg Ile Leu Leu Pro Asp Tyr Lys Asp Asp Asp Asp Lys
        1635                1640                1645

<210>  28
<211>  4731
<212>  DNA
<213>  Artificial Sequence

<220>
<223>  Coding sequence of AIDx-XTEN-dCas9

<400>  28
   atggacagcc tcttgatgaa ccggaggaag tttctttacc aattcaaaaa tgtccgctgg      60
   gctaagggtc ggcgtgagac ctacctgtgc tacgtagtga agaggcgtga cagtgctaca     120
   tccttttcac tggactttgg ttatcttcgc aataagaacg gctgccacgt ggaattgctc     180
   ttcctccgct acatctcgga ctgggaccta gaccctggcc gctgctaccg cgtcacctgg     240
   ttcacctcct ggagcccctg ctacgactgt gcccgacatg tggccgactt tctgcgaggg     300
   aaccccaacc tcagtctgag gatcttcacc gcgcgcctct acttctgtga ggaccgcaag     360
   gctgagcccg aggggctgcg gcggctgcac cgcgccgggg tgcaaatagc catcatgacc     420
   ttcaaagatt atttttactg ctggaatact tttgtagaaa accatgaaag aactttcaaa     480
   gcctgggaag ggctgcatga aaattcagtt cgtctctcca gacagcttcg gcgcatcctt     540
   ttgcccagcg gcagcgagac tcccgggacc tcagagtccg ccacacccga aagtgataaa     600
   aagtattcta ttggtttagc catcggcact aattccgttg gatgggctgt cataaccgat     660
   gaatacaaag taccttcaaa gaaatttaag gtgttgggga acacagaccg tcattcgatt     720
   aaaaagaatc ttatcggtgc cctcctattc gatagtggcg aaacggcaga ggcgactcgc     780
   ctgaaacgaa ccgctcggag aaggtataca cgtcgcaaga accgaatatg ttacttacaa     840
   gaaattttta gcaatgagat ggccaaagtt gacgattctt tctttcaccg tttggaagag     900
   tccttccttg tcgaagagga caagaaacat gaacggcacc ccatctttgg aaacatagta     960
   gatgaggtgg catatcatga aaagtaccca acgatttatc acctcagaaa aaagctagtt    1020
   gactcaactg ataaagcgga cctgaggtta atctacttgg ctcttgccca tatgataaag    1080
   ttccgtgggc actttctcat tgagggtgat ctaaatccgg acaactcgga tgtcgacaaa    1140
   ctgttcatcc agttagtaca aacctataat cagttgtttg aagagaaccc tataaatgca    1200
   agtggcgtgg atgcgaaggc tattcttagc gcccgcctct ctaaatcccg acggctagaa    1260
   aacctgatcg cacaattacc cggagagaag aaaaatgggt tgttcggtaa ccttatagcg    1320
   ctctcactag gcctgacacc aaattttaag tcgaacttcg acttagctga agatgccaaa    1380
   ttgcagctta gtaaggacac gtacgatgac gatctcgaca atctactggc acaaattgga    1440
   gatcagtatg cggacttatt tttggctgcc aaaaacctta gcgatgcaat cctcctatct    1500
   gacatactga gagttaatac tgagattacc aaggcgccgt tatccgcttc aatgatcaaa    1560
   aggtacgatg aacatcacca agacttgaca cttctcaagg ccctagtccg tcagcaactg    1620
   cctgagaaat ataaggaaat attctttgat cagtcgaaaa acgggtacgc aggttatatt    1680
   gacggcggag cgagtcaaga ggaattctac aagtttatca aacccatatt agagaagatg    1740
   gatgggacgg aagagttgct tgtaaaactc aatcgcgaag atctactgcg aaagcagcgg    1800
   actttcgaca acggtagcat tccacatcaa atccacttag gcgaattgca tgctatactt    1860
   agaaggcagg aggattttta tccgttcctc aaagacaatc gtgaaaagat tgagaaaatc    1920
   ctaacctttc gcatacctta ctatgtggga cccctggccc gagggaactc tcggttcgca    1980
   tggatgacaa gaaagtccga agaaacgatt actccatgga attttgagga agttgtcgat    2040
   aaaggtgcgt cagctcaatc gttcatcgag aggatgacca actttgacaa gaatttaccg    2100
   aacgaaaaag tattgcctaa gcacagttta ctttacgagt atttcacagt gtacaatgaa    2160
   ctcacgaaag ttaagtatgt cactgagggc atgcgtaaac ccgcctttct aagcggagaa    2220
   cagaagaaag caatagtaga tctgttattc aagaccaacc gcaaagtgac agttaagcaa    2280
   ttgaaagagg actactttaa gaaaattgaa tgcttcgatt ctgtcgagat ctccggggta    2340
   gaagatcgat ttaatgcgtc acttggtacg tatcatgacc tcctaaagat aattaaagat    2400
   aaggacttcc tggataacga agagaatgaa gatatcttag aagatatagt gttgactctt    2460
   accctctttg aagatcggga aatgattgag gaaagactaa aaacatacgc tcacctgttc    2520
   gacgataagg ttatgaaaca gttaaagagg cgtcgctata cgggctgggg acgattgtcg    2580
   cggaaactta tcaacgggat aagagacaag caaagtggta aaactattct cgattttcta    2640
   aagagcgacg gcttcgccaa taggaacttt atgcagctga tccatgatga ctctttaacc    2700
   ttcaaagagg atatacaaaa ggcacaggtt tccggacaag gggactcatt gcacgaacat    2760
   attgcgaatc ttgctggttc gccagccatc aaaaagggca tactccagac agtcaaagta    2820
   gtggatgagc tagttaaggt catgggacgt cacaaaccgg aaaacattgt aatcgagatg    2880
   gcacgcgaaa atcaaacgac tcagaagggg caaaaaaaca gtcgagagcg gatgaagaga    2940
   atagaagagg gtattaaaga actgggcagc cagatcttaa aggagcatcc tgtggaaaat    3000
   acccaattgc agaacgagaa actttacctc tattacctac aaaatggaag ggacatgtat    3060
   gttgatcagg aactggacat aaaccgttta tctgattacg acgtcgatgc cattgtaccc    3120
   caatcctttt tgaaggacga ttcaatcgac aataaagtgc ttacacgctc ggataagaac    3180
   cgagggaaaa gtgacaatgt tccaagcgag gaagtcgtaa agaaaatgaa gaactattgg    3240
   cggcagctcc taaatgcgaa actgataacg caaagaaagt tcgataactt aactaaagct    3300
   gagaggggtg gcttgtctga acttgacaag gccggattta ttaaacgtca gctcgtggaa    3360
   acccgccaaa tcacaaagca tgttgcacag atactagatt cccgaatgaa tacgaaatac    3420
   gacgagaacg ataagctgat tcgggaagtc aaagtaatca ctttaaagtc aaaattggtg    3480
   tcggacttca gaaaggattt tcaattctat aaagttaggg agataaataa ctaccaccat    3540
   gcgcacgacg cttatcttaa tgccgtcgta gggaccgcac tcattaagaa atacccgaag 3600
   ctagaaagtg agtttgtgta tggtgattac aaagtttatg acgtccgtaa gatgatcgcg 3660
   aaaagcgaac aggagatagg caaggctaca gccaaatact tcttttattc taacattatg 3720
   aatttcttta agacggaaat cactctggca aacggagaga tacgcaaacg acctttaatt 3780
   gaaaccaatg gggagacagg tgaaatcgta tgggataagg gccgggactt cgcgacggtg 3840
   agaaaagttt tgtccatgcc ccaagtcaac atagtaaaga aaactgaggt gcagaccgga 3900
   gggttttcaa aggaatcgat tcttccaaaa aggaatagtg ataagctcat cgctcgtaaa 3960
   aaggactggg acccgaaaaa gtacggtggc ttcgatagcc ctacagttgc ctattctgtc 4020
   ctagtagtgg caaaagttga gaagggaaaa tccaagaaac tgaagtcagt caaagaatta 4080
   ttggggataa cgattatgga gcgctcgtct tttgaaaaga accccatcga cttccttgag 4140
   gcgaaaggtt acaaggaagt aaaaaaggat ctcataatta aactaccaaa gtatagtctg 4200
   tttgagttag aaaatggccg aaaacggatg ttggctagcg ccggagagct tcaaaagggg 4260
   aacgaactcg cactaccgtc taaatacgtg aatttcctgt atttagcgtc ccattacgag 4320
   aagttgaaag gttcacctga agataacgaa cagaagcaac tttttgttga gcagcacaaa 4380
   cattatctcg acgaaatcat agagcaaatt tcggaattca gtaagagagt catcctagct 4440
   gatgccaatc tggacaaagt attaagcgca tacaacaagc acaggataaa acccatacgt 4500
   gagcaggcgg aaaatattat ccatttgttt actcttacca acctcggcgc tccagccgca 4560
   ttcaagtatt ttgacacaac gatagatcgc aaacgataca cttctaccaa ggaggtgcta 4620
   gacgcgacac tgattcacca atccatcacg ggattatatg aaactcggat agatttgtca 4680
   cagcttgggg gtgactctgg tggttctccc aagaagaaga ggaaagtcta a 4731

<210> 29
<211> 1576
<212> PRT
<213> Artificial Sequence

<220>
<223> Amino acid sequence of AIDx-XTEN-dCas9

<400> 29
Met Asp Ser Leu Leu Met Asn Arg Arg Lys Phe Leu Tyr Gln Phe Lys
1               5                   10                  15
Asn Val Arg Trp Ala Lys Gly Arg Arg Glu Thr Tyr Leu Cys Tyr Val
            20                  25                  30
Val Lys Arg Arg Asp Ser Ala Thr Ser Phe Ser Leu Asp Phe Gly Tyr
        35                  40                  45
Leu Arg Asn Lys Asn Gly Cys His Val Glu Leu Leu Phe Leu Arg Tyr
    50                  55                  60
Ile Ser Asp Trp Asp Leu Asp Pro Gly Arg Cys Tyr Arg Val Thr Trp
65                  70                  75                  80
Phe Thr Ser Trp Ser Pro Cys Tyr Asp Cys Ala Arg His Val Ala Asp
                85                  90                  95
Phe Leu Arg Gly Asn Pro Asn Leu Ser Leu Arg Ile Phe Thr Ala Arg
            100                 105                 110
Leu Tyr Phe Cys Glu Asp Arg Lys Ala Glu Pro Glu Gly Leu Arg Arg
        115                 120                 125
Leu His Arg Ala Gly Val Gln Ile Ala Ile Met Thr Phe Lys Asp Tyr
    130                 135                 140
Phe Tyr Cys Trp Asn Thr Phe Val Glu Asn His Glu Arg Thr Phe Lys
145                 150                 155                 160
Ala Trp Glu Gly Leu His Glu Asn Ser Val Arg Leu Ser Arg Gln Leu
                165                 170                 175
Arg Arg Ile Leu Leu Pro Ser Gly Ser Glu Thr Pro Gly Thr Ser Glu
            180                 185                 190
Ser Ala Thr Pro Glu Ser Asp Lys Lys Tyr Ser Ile Gly Leu Ala Ile
        195                 200                 205
Gly Thr Asn Ser Val Gly Trp Ala Val Ile Thr Asp Glu Tyr Lys Val
    210                 215                 220
Pro Ser Lys Lys Phe Lys Val Leu Gly Asn Thr Asp Arg His Ser Ile
225                 230                 235                 240
Lys Lys Asn Leu Ile Gly Ala Leu Leu Phe Asp Ser Gly Glu Thr Ala
                245                 250                 255
Glu Ala Thr Arg Leu Lys Arg Thr Ala Arg Arg Arg Tyr Thr Arg Arg

260                 265                 270
Lys Asn Arg Ile Cys Tyr Leu Gln Glu Ile Phe Ser Asn Glu Met Ala
        275                 280                 285
Lys Val Asp Asp Ser Phe Phe His Arg Leu Glu Glu Ser Phe Leu Val
    290                 295                 300
Glu Glu Asp Lys Lys His Glu Arg His Pro Ile Phe Gly Asn Ile Val
305                 310                 315                 320
Asp Glu Val Ala Tyr His Glu Lys Tyr Pro Thr Ile Tyr His Leu Arg
                325                 330                 335
Lys Lys Leu Val Asp Ser Thr Asp Lys Ala Asp Leu Arg Leu Ile Tyr
            340                 345                 350
Leu Ala Leu Ala His Met Ile Lys Phe Arg Gly His Phe Leu Ile Glu
        355                 360                 365
Gly Asp Leu Asn Pro Asp Asn Ser Asp Val Asp Lys Leu Phe Ile Gln
    370                 375                 380
Leu Val Gln Thr Tyr Asn Gln Leu Phe Glu Glu Asn Pro Ile Asn Ala
385                 390                 395                 400
Ser Gly Val Asp Ala Lys Ala Ile Leu Ser Ala Arg Leu Ser Lys Ser
                405                 410                 415
Arg Arg Leu Glu Asn Leu Ile Ala Gln Leu Pro Gly Glu Lys Lys Asn
            420                 425                 430
Gly Leu Phe Gly Asn Leu Ile Ala Leu Ser Leu Gly Leu Thr Pro Asn
        435                 440                 445
Phe Lys Ser Asn Phe Asp Leu Ala Glu Asp Ala Lys Leu Gln Leu Ser
    450                 455                 460
Lys Asp Thr Tyr Asp Asp Asp Leu Asp Asn Leu Leu Ala Gln Ile Gly
465                 470                 475                 480
Asp Gln Tyr Ala Asp Leu Phe Leu Ala Ala Lys Asn Leu Ser Asp Ala
                485                 490                 495
Ile Leu Leu Ser Asp Ile Leu Arg Val Asn Thr Glu Ile Thr Lys Ala
            500                 505                 510
Pro Leu Ser Ala Ser Met Ile Lys Arg Tyr Asp Glu His His Gln Asp
        515                 520                 525
Leu Thr Leu Leu Lys Ala Leu Val Arg Gln Gln Leu Pro Glu Lys Tyr
    530                 535                 540
Lys Glu Ile Phe Phe Asp Gln Ser Lys Asn Gly Tyr Ala Gly Tyr Ile
545                 550                 555                 560
Asp Gly Gly Ala Ser Gln Glu Glu Phe Tyr Lys Phe Ile Lys Pro Ile
                565                 570                 575
Leu Glu Lys Met Asp Gly Thr Glu Glu Leu Leu Val Lys Leu Asn Arg
            580                 585                 590
Glu Asp Leu Leu Arg Lys Gln Arg Thr Phe Asp Asn Gly Ser Ile Pro
        595                 600                 605
His Gln Ile His Leu Gly Glu Leu His Ala Ile Leu Arg Arg Gln Glu
    610                 615                 620
Asp Phe Tyr Pro Phe Leu Lys Asp Asn Arg Glu Lys Ile Glu Lys Ile
625                 630                 635                 640
Leu Thr Phe Arg Ile Pro Tyr Tyr Val Gly Pro Leu Ala Arg Gly Asn
                645                 650                 655
Ser Arg Phe Ala Trp Met Thr Arg Lys Ser Glu Glu Thr Ile Thr Pro
            660                 665                 670
Trp Asn Phe Glu Glu Val Val Asp Lys Gly Ala Ser Ala Gln Ser Phe
        675                 680                 685
Ile Glu Arg Met Thr Asn Phe Asp Lys Asn Leu Pro Asn Glu Lys Val
    690                 695                 700
Leu Pro Lys His Ser Leu Leu Tyr Glu Tyr Phe Thr Val Tyr Asn Glu
705                 710                 715                 720
Leu Thr Lys Val Lys Tyr Val Thr Glu Gly Met Arg Lys Pro Ala Phe
                725                 730                 735
Leu Ser Gly Glu Gln Lys Lys Ala Ile Val Asp Leu Leu Phe Lys Thr
            740                 745                 750
Asn Arg Lys Val Thr Val Lys Gln Leu Lys Glu Asp Tyr Phe Lys Lys
        755                 760                 765

Ile Glu Cys Phe Asp Ser Val Glu Ile Ser Gly Val Glu Asp Arg Phe
    770                 775                 780
Asn Ala Ser Leu Gly Thr Tyr His Asp Leu Leu Lys Ile Ile Lys Asp
785                 790                 795                 800
Lys Asp Phe Leu Asp Asn Glu Glu Asn Glu Asp Ile Leu Glu Asp Ile
                805                 810                 815
Val Leu Thr Leu Thr Leu Phe Glu Asp Arg Glu Met Ile Glu Glu Arg
            820                 825                 830
Leu Lys Thr Tyr Ala His Leu Phe Asp Asp Lys Val Met Lys Gln Leu
        835                 840                 845
Lys Arg Arg Arg Tyr Thr Gly Trp Gly Arg Leu Ser Arg Lys Leu Ile
    850                 855                 860
Asn Gly Ile Arg Asp Lys Gln Ser Gly Lys Thr Ile Leu Asp Phe Leu
865                 870                 875                 880
Lys Ser Asp Gly Phe Ala Asn Arg Asn Phe Met Gln Leu Ile His Asp
                885                 890                 895
Asp Ser Leu Thr Phe Lys Glu Asp Ile Gln Lys Ala Gln Val Ser Gly
            900                 905                 910
Gln Gly Asp Ser Leu His Glu His Ile Ala Asn Leu Ala Gly Ser Pro
        915                 920                 925
Ala Ile Lys Lys Gly Ile Leu Gln Thr Val Lys Val Val Asp Glu Leu
    930                 935                 940
Val Lys Val Met Gly Arg His Lys Pro Glu Asn Ile Val Ile Glu Met
945                 950                 955                 960
Ala Arg Glu Asn Gln Thr Thr Gln Lys Gly Gln Lys Asn Ser Arg Glu
                965                 970                 975
Arg Met Lys Arg Ile Glu Glu Gly Ile Lys Glu Leu Gly Ser Gln Ile
            980                 985                 990
Leu Lys Glu His Pro Val Glu Asn Thr Gln Leu Gln Asn Glu Lys Leu
        995                 1000                1005
Tyr Leu Tyr Tyr Leu Gln Asn Gly Arg Asp Met Tyr Val Asp Gln Glu
    1010                1015                1020
Leu Asp Ile Asn Arg Leu Ser Asp Tyr Asp Val Asp Ala Ile Val Pro
1025                1030                1035                1040
Gln Ser Phe Leu Lys Asp Asp Ser Ile Asp Asn Lys Val Leu Thr Arg
                1045                1050                1055
Ser Asp Lys Asn Arg Gly Lys Ser Asp Asn Val Pro Ser Glu Glu Val
            1060                1065                1070
Val Lys Lys Met Lys Asn Tyr Trp Arg Gln Leu Leu Asn Ala Lys Leu
        1075                1080                1085
Ile Thr Gln Arg Lys Phe Asp Asn Leu Thr Lys Ala Glu Arg Gly Gly
    1090                1095                1100
Leu Ser Glu Leu Asp Lys Ala Gly Phe Ile Lys Arg Gln Leu Val Glu
1105                1110                1115                1120
Thr Arg Gln Ile Thr Lys His Val Ala Gln Ile Leu Asp Ser Arg Met
                1125                1130                1135
Asn Thr Lys Tyr Asp Glu Asn Asp Lys Leu Ile Arg Glu Val Lys Val
            1140                1145                1150
Ile Thr Leu Lys Ser Lys Leu Val Ser Asp Phe Arg Lys Asp Phe Gln
        1155                1160                1165
Phe Tyr Lys Val Arg Glu Ile Asn Asn Tyr His His Ala His Asp Ala
    1170                1175                1180
Tyr Leu Asn Ala Val Val Gly Thr Ala Leu Ile Lys Lys Tyr Pro Lys
1185                1190                1195                1200
Leu Glu Ser Glu Phe Val Tyr Gly Asp Tyr Lys Val Tyr Asp Val Arg
                1205                1210                1215
Lys Met Ile Ala Lys Ser Glu Gln Glu Ile Gly Lys Ala Thr Ala Lys
            1220                1225                1230
Tyr Phe Phe Tyr Ser Asn Ile Met Asn Phe Phe Lys Thr Glu Ile Thr
        1235                1240                1245
Leu Ala Asn Gly Glu Ile Arg Lys Arg Pro Leu Ile Glu Thr Asn Gly
    1250                1255                1260
Glu Thr Gly Glu Ile Val Trp Asp Lys Gly Arg Asp Phe Ala Thr Val

1265                1270                1275                1280
Arg Lys Val Leu Ser Met Pro Gln Val Asn Ile Val Lys Lys Thr Glu
                1285                1290                1295
Val Gln Thr Gly Gly Phe Ser Lys Glu Ser Ile Leu Pro Lys Arg Asn
            1300                1305                1310
Ser Asp Lys Leu Ile Ala Arg Lys Lys Asp Trp Asp Pro Lys Lys Tyr
        1315                1320                1325
Gly Gly Phe Asp Ser Pro Thr Val Ala Tyr Ser Val Leu Val Val Ala
    1330                1335                1340
Lys Val Glu Lys Gly Lys Ser Lys Lys Leu Lys Ser Val Lys Glu Leu
1345                1350                1355                1360
Leu Gly Ile Thr Ile Met Glu Arg Ser Ser Phe Glu Lys Asn Pro Ile
                1365                1370                1375
Asp Phe Leu Glu Ala Lys Gly Tyr Lys Glu Val Lys Lys Asp Leu Ile
            1380                1385                1390
Ile Lys Leu Pro Lys Tyr Ser Leu Phe Glu Leu Glu Asn Gly Arg Lys
        1395                1400                1405
Arg Met Leu Ala Ser Ala Gly Glu Leu Gln Lys Gly Asn Glu Leu Ala
    1410                1415                1420
Leu Pro Ser Lys Tyr Val Asn Phe Leu Tyr Leu Ala Ser His Tyr Glu
1425                1430                1435                1440
Lys Leu Lys Gly Ser Pro Glu Asp Asn Glu Gln Lys Gln Leu Phe Val
                1445                1450                1455
Glu Gln His Lys His Tyr Leu Asp Glu Ile Ile Glu Gln Ile Ser Glu
            1460                1465                1470
Phe Ser Lys Arg Val Ile Leu Ala Asp Ala Asn Leu Asp Lys Val Leu
        1475                1480                1485
Ser Ala Tyr Asn Lys His Arg Asp Lys Pro Ile Arg Glu Gln Ala Glu
    1490                1495                1500
Asn Ile Ile His Leu Phe Thr Leu Thr Asn Leu Gly Ala Pro Ala Ala
1505                1510                1515                1520
Phe Lys Tyr Phe Asp Thr Thr Ile Asp Arg Lys Arg Tyr Thr Ser Thr
                1525                1530                1535
Lys Glu Val Leu Asp Ala Thr Leu Ile His Gln Ser Ile Thr Gly Leu
            1540                1545                1550
Tyr Glu Thr Arg Ile Asp Leu Ser Gln Leu Gly Gly Asp Ser Gly Gly
        1555                1560                1565
Ser Pro Lys Lys Lys Arg Lys Val
    1570                1575

<210>  30
<211>  4890
<212>  DNA
<213>  Artificial Sequence

<220>
<223>  Coding sequence of dCas9-XTEN-AID P182X K10E T82I E156G

<400>  30
atggactata aggaccacga cggagactac aaggatcatg atattgatta caaagacgat      60
gacgataaga tggccccaaa gaagaagcgg aaggtcggta tccacggagt cccagcagct     120
accatggaca agaagtattc tatcggactg gccatcggga ctaatagcgt cgggtgggcc     180
gtgatcactg acgagtacaa ggtgccctct aagaagttca aggtgctcgg gaacaccgac     240
cggcattcca tcaagaaaaa tctgatcgga gctctcctct ttgattcagg ggagaccgct     300
gaagcaaccc gcctcaagcg gactgctaga cggcggtaca ccaggaggaa gaaccggatt     360
tgttaccttc aagagatatt ctccaacgaa atggcaaagg tcgacgacag cttcttccat     420
aggctggaag aatcattcct cgtggaagag gataagaagc atgaacggca tcccatcttc     480
ggtaatatcg tcgacgaggt ggcctatcac gagaaatacc caaccatcta ccatcttcgc     540
aaaaagctgg tggactcaac cgacaaggca gacctccggc ttatctacct ggccctggcc     600
cacatgatca agttcagagg ccacttcctg atcgagggcg acctcaatcc tgacaatagc     660
gatgtggata aactgttcat ccagctggtg cagacttaca accagctctt tgaagagaac     720
cccatcaatg caagcggagt cgatgccaag gccattctgt cagcccggct gtcaaagagc     780
cgcagacttg agaatcttat cgctcagctg ccgggtgaaa agaaaaatgg actgttcggg     840

aacctgattg ctctttcact tgggctgact cccaatttca agtctaattt cgacctggca 900
gaggatgcca agctgcaact gtccaaggac acctatgatg acgatctcga caacctcctg 960
gcccagatcg gtgaccaata cgccgacctt ttccttgctg ctaagaatct ttctgacgcc 1020
atcctgctgt ctgacattct ccgcgtgaac actgaaatca ccaaggcccc tctttcagct 1080
tcaatgatta agcggtatga tgagcaccac caggacctga ccctgcttaa ggcactcgtc 1140
cggcagcagc ttccggagaa gtacaaggaa atcttctttg accagtcaaa gaatggatac 1200
gccggctaca tcgacggagg tgcctcccaa gaggaatttt ataagtttat caaacctatc 1260
cttgagaaga tggacggcac cgaagagctc ctcgtgaaac tgaatcggga ggatctgctg 1320
cggaagcagc gcactttcga caatgggagc attccccacc agatccatct tggggagctt 1380
cacgccatcc ttcggcgcca agaggacttc tacccctttc ttaaggacaa cagggagaag 1440
attgagaaaa ttctcacttt ccgcatcccc tactacgtgg gacccctcgc cagaggaaat 1500
agccggtttg cttggatgac cagaaagtca gaagaaacta tcactccctg gaacttcgaa 1560
gaggtggtgg acaagggagc cagcgctcag tcattcatcg aacggatgac taacttcgat 1620
aagaacctcc ccaatgagaa ggtcctgccg aaacattccc tgctctacga gtactttacc 1680
gtgtacaacg agctgaccaa ggtgaaatat gtcaccgaag ggatgaggaa gcccgcattc 1740
ctgtcaggcg aacaaaagaa ggcaattgtg gaccttctgt tcaagaccaa tagaaaggtg 1800
accgtgaagc agctgaagga ggactatttc aagaaaattg aatgcttcga ctctgtggag 1860
attagcgggg tcgaagatcg gttcaacgca agcctgggta cctaccatga tctgcttaag 1920
atcatcaagg acaaggattt tctggacaat gaggagaaag aggacatcct tgaggacatt 1980
gtcctgactc tcactctgtt cgaggaccgg gaaatgatcg aggagaggct taagacctac 2040
gcccatctgt tcgacgataa agtgatgaag caacttaaac ggagaagata taccggatgg 2100
ggacgcctta gccgcaaact catcaacgga atccgggaca aacagagcgg aaagaccatt 2160
cttgatttcc ttaagagcga cggattcgct aatcgcaact tcatgcaact tatccatgat 2220
gattccctga cctttaagga ggacatccag aaggcccaag tgtctggaca aggtgactca 2280
ctgcacgagc atatcgcaaa tctggctggt tcacccgcta ttaagaaggg tattctccag 2340
accgtgaaag tcgtggacga gctggtcaag gtgatgggtc gccataaacc agagaacatt 2400
gtcatcgaga tggccaggga aaaccagact acccagaagg gacagaagaa cagcagggag 2460
cggatgaaaa gaattgagga agggattaag gagctcgggt cacagatcct taaagagcac 2520
ccggtggaaa acacccagct tcagaatgag aagctctatc tgtactacct tcaaaatgga 2580
cgcgatatgt atgtggacca agagcttgat atcaacaggc tctcagacta cgacgtggac 2640
gccatcgtcc ctcagagctt cctcaaagac gactcaattg acaataaggt gctgactcgc 2700
tcagacaaga accggggaaa gtcagataac gtgccctcag aggaagtcgt gaaaaagatg 2760
aagaactatt ggcgccagct tctgaacgca aagctgatca ctcagcggaa gttcgacaat 2820
ctcactaagg ctgagagggg cggactgagc gaactggaca aagcaggatt cattaaacgg 2880
caacttgtgg agactcggca gattactaaa catgtagccc aaatccttga ctcacgcatg 2940
aataccaagt acgacgaaaa cgacaaactt atccgcgagg tgaaggtgat taccctgaag 3000
tccaagctgg tcagcgattt cagaaaggac tttcaattct acaaagtgcg ggagatcaat 3060
aactatcatc atgctcatga cgcatatctg aatgccgtgg tgggaaccgc cctgatcaag 3120
aagtacccaa agctggaaag cgagttcgtg tacggagact acaaggtcta cgacgtgcgc 3180
aagatgattg ccaaatctga gcaggagatc ggaaaggcca ccgcaaagta cttcttctac 3240
agcaacatca tgaatttctt caagaccgaa atcacccttg caaacggtga gatccggaag 3300
aggccgctca tcgagactaa tggggagact ggcgaaatcg tgtgggacaa gggcagagat 3360
ttcgctaccg tgcgcaaagt gctttctatg cctcaagtga acatcgtgaa gaaaaccgag 3420
gtgcaaaccg gaggcttttc taaggaatca atcctcccca agcgcaactc cgacaagctc 3480
attgcaagga agaaggattg ggaccctaag aagtacggcg gattcgattc accaactgtg 3540
gcttattctg tcctggtcgt ggctaaggtg gaaaaaggaa agtctaagaa gctcaagagc 3600
gtgaaggaac tgctgggtat caccattatg gagcgcagct ccttcgagaa gaacccaatt 3660
gactttctcg aagccaaagg ttacaaggaa gtcaagaagg accttatcat caagctccca 3720
aagtatagcc tgttcgaact ggagaatggg cggaagcgga tgctcgcctc cgctggcgaa 3780
cttcagaagg gtaatgagct ggctctcccc tccaagtacg tgaatttcct ctaccttgca 3840
agccattacg agaagctgaa ggggagcccc gaggacaacg agcaaaagca actgtttgtg 3900
gagcagcata agcattatct ggacgagatc attgagcaga tttccgagtt ttctaaacgc 3960
gtcattctcg ctgatgccaa cctcgataaa gtccttagcg catacaataa gcacagagac 4020
aaaccaattc gggagcaggc tgagaatatc atccacctgt tcaccctcac caatcttggt 4080
gcccctgccg cattcaagta cttcgacacc accatcgacc ggaaacgcta tacctccacc 4140
aaagaagtgc tggacgccac cctcatccac cagagcatca ccggacttta cgaaactcgg 4200
attgacctct cacagctcgg aggggatgag ggagctccca agaaaaagcg caaggtaggt 4260
agttccggat ctccgaaaaa gaaacgcaaa gttagcggca gcgagactcc cgggacctca 4320
gagtccgcca cacccgaaag tatggacagc ctcttgatga accggaggga gtttctttac 4380
caattcaaaa atgtccgctg ggctaagggt cggcgtgaga cctacctgtg ctacgtagtg 4440
aagaggcgtg acagtgctac atccttttca ctggactttg gttatcttcg caataagaac 4500
ggctgccacg tggaattgct cttcctccgc tacatctcgg actgggacct agaccctggc 4560
cgctgctacc gcgtcacctg gttcatctcc tggagcccct gctacgactg tgcccgacat 4620

gtggccgact ttctgcgagg gaaccccaac ctcagtctga ggatcttcac cgcgcgcctc 4680
tacttctgtg aggaccgcaa ggctgagccc gaggggctgc ggcggctgca ccgcgccggg 4740
gtgcaaatag ccatcatgac cttcaaagat tatttttact gctggaatac ttttgtagaa 4800
aaccatggaa gaactttcaa agcctgggaa gggctgcatg aaaattcagt tcgtctctcc 4860
agacagcttc ggcgcatcct tttgccctga 4890

<210> 31
<211> 1629
<212> PRT
<213> Artificial Sequence

<220>
<223> Amino acid sequence of dCas9-XTEN-AID P182X K10E T82I E156G

<400> 31
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Gly Val Pro Ala Ala Thr Met Asp Lys Lys Tyr Ser Ile
        35                  40                  45
Gly Leu Ala Ile Gly Thr Asn Ser Val Gly Trp Ala Val Ile Thr Asp
    50                  55                  60
Glu Tyr Lys Val Pro Ser Lys Lys Phe Lys Val Leu Gly Asn Thr Asp
65                  70                  75                  80
Arg His Ser Ile Lys Lys Asn Leu Ile Gly Ala Leu Leu Phe Asp Ser
                85                  90                  95
Gly Glu Thr Ala Glu Ala Thr Arg Leu Lys Arg Thr Ala Arg Arg Arg
            100                 105                 110
Tyr Thr Arg Arg Lys Asn Arg Ile Cys Tyr Leu Gln Glu Ile Phe Ser
        115                 120                 125
Asn Glu Met Ala Lys Val Asp Asp Ser Phe Phe His Arg Leu Glu Glu
    130                 135                 140
Ser Phe Leu Val Glu Glu Asp Lys Lys His Glu Arg His Pro Ile Phe
145                 150                 155                 160
Gly Asn Ile Val Asp Glu Val Ala Tyr His Glu Lys Tyr Pro Thr Ile
                165                 170                 175
Tyr His Leu Arg Lys Lys Leu Val Asp Ser Thr Asp Lys Ala Asp Leu
            180                 185                 190
Arg Leu Ile Tyr Leu Ala Leu Ala His Met Ile Lys Phe Arg Gly His
        195                 200                 205
Phe Leu Ile Glu Gly Asp Leu Asn Pro Asp Asn Ser Asp Val Asp Lys
    210                 215                 220
Leu Phe Ile Gln Leu Val Gln Thr Tyr Asn Gln Leu Phe Glu Glu Asn
225                 230                 235                 240
Pro Ile Asn Ala Ser Gly Val Asp Ala Lys Ala Ile Leu Ser Ala Arg
                245                 250                 255
Leu Ser Lys Ser Arg Arg Leu Glu Asn Leu Ile Ala Gln Leu Pro Gly
            260                 265                 270
Glu Lys Lys Asn Gly Leu Phe Gly Asn Leu Ile Ala Leu Ser Leu Gly
        275                 280                 285
Leu Thr Pro Asn Phe Lys Ser Asn Phe Asp Leu Ala Glu Asp Ala Lys
    290                 295                 300
Leu Gln Leu Ser Lys Asp Thr Tyr Asp Asp Asp Leu Asp Asn Leu Leu
305                 310                 315                 320
Ala Gln Ile Gly Asp Gln Tyr Ala Asp Leu Phe Leu Ala Ala Lys Asn
                325                 330                 335
Leu Ser Asp Ala Ile Leu Leu Ser Asp Ile Leu Arg Val Asn Thr Glu
            340                 345                 350
Ile Thr Lys Ala Pro Leu Ser Ala Ser Met Ile Lys Arg Tyr Asp Glu
        355                 360                 365
His His Gln Asp Leu Thr Leu Leu Lys Ala Leu Val Arg Gln Gln Leu
    370                 375                 380

Pro Glu Lys Tyr Lys Glu Ile Phe Phe Asp Gln Ser Lys Asn Gly Tyr
385                 390                 395                 400
Ala Gly Tyr Ile Asp Gly Gly Ala Ser Gln Glu Glu Phe Tyr Lys Phe
                405                 410                 415
Ile Lys Pro Ile Leu Glu Lys Met Asp Gly Thr Glu Glu Leu Leu Val
            420                 425                 430
Lys Leu Asn Arg Glu Asp Leu Leu Arg Lys Gln Arg Thr Phe Asp Asn
        435                 440                 445
Gly Ser Ile Pro His Gln Ile His Leu Gly Glu Leu His Ala Ile Leu
    450                 455                 460
Arg Arg Gln Glu Asp Phe Tyr Pro Phe Leu Lys Asp Asn Arg Glu Lys
465                 470                 475                 480
Ile Glu Lys Ile Leu Thr Phe Arg Ile Pro Tyr Tyr Val Gly Pro Leu
                485                 490                 495
Ala Arg Gly Asn Ser Arg Phe Ala Trp Met Thr Arg Lys Ser Glu Glu
            500                 505                 510
Thr Ile Thr Pro Trp Asn Phe Glu Glu Val Val Asp Lys Gly Ala Ser
        515                 520                 525
Ala Gln Ser Phe Ile Glu Arg Met Thr Asn Phe Asp Lys Asn Leu Pro
    530                 535                 540
Asn Glu Lys Val Leu Pro Lys His Ser Leu Leu Tyr Glu Tyr Phe Thr
545                 550                 555                 560
Val Tyr Asn Glu Leu Thr Lys Val Lys Tyr Val Thr Glu Gly Met Arg
                565                 570                 575
Lys Pro Ala Phe Leu Ser Gly Glu Gln Lys Lys Ala Ile Val Asp Leu
            580                 585                 590
Leu Phe Lys Thr Asn Arg Lys Val Thr Val Lys Gln Leu Lys Glu Asp
        595                 600                 605
Tyr Phe Lys Lys Ile Glu Cys Phe Asp Ser Val Glu Ile Ser Gly Val
    610                 615                 620
Glu Asp Arg Phe Asn Ala Ser Leu Gly Thr Tyr His Asp Leu Leu Lys
625                 630                 635                 640
Ile Ile Lys Asp Lys Asp Phe Leu Asp Asn Glu Glu Lys Glu Asp Ile
                645                 650                 655
Leu Glu Asp Ile Val Leu Thr Leu Thr Leu Phe Glu Asp Arg Glu Met
            660                 665                 670
Ile Glu Glu Arg Leu Lys Thr Tyr Ala His Leu Phe Asp Asp Lys Val
        675                 680                 685
Met Lys Gln Leu Lys Arg Arg Arg Tyr Thr Gly Trp Gly Arg Leu Ser
    690                 695                 700
Arg Lys Leu Ile Asn Gly Ile Arg Asp Lys Gln Ser Gly Lys Thr Ile
705                 710                 715                 720
Leu Asp Phe Leu Lys Ser Asp Gly Phe Ala Asn Arg Asn Phe Met Gln
                725                 730                 735
Leu Ile His Asp Asp Ser Leu Thr Phe Lys Glu Asp Ile Gln Lys Ala
            740                 745                 750
Gln Val Ser Gly Gln Gly Asp Ser Leu His Glu His Ile Ala Asn Leu
        755                 760                 765
Ala Gly Ser Pro Ala Ile Lys Lys Gly Ile Leu Gln Thr Val Lys Val
    770                 775                 780
Val Asp Glu Leu Val Lys Val Met Gly Arg His Lys Pro Glu Asn Ile
785                 790                 795                 800
Val Ile Glu Met Ala Arg Glu Asn Gln Thr Thr Gln Lys Gly Gln Lys
                805                 810                 815
Asn Ser Arg Glu Arg Met Lys Arg Ile Glu Glu Gly Ile Lys Glu Leu
            820                 825                 830
Gly Ser Gln Ile Leu Lys Glu His Pro Val Glu Asn Thr Gln Leu Gln
        835                 840                 845
Asn Glu Lys Leu Tyr Leu Tyr Tyr Leu Gln Asn Gly Arg Asp Met Tyr
    850                 855                 860
Val Asp Gln Glu Leu Asp Ile Asn Arg Leu Ser Asp Tyr Asp Val Asp
865                 870                 875                 880
Ala Ile Val Pro Gln Ser Phe Leu Lys Asp Asp Ser Ile Asp Asn Lys

885                 890                 895
Val Leu Thr Arg Ser Asp Lys Asn Arg Gly Lys Ser Asp Asn Val Pro
            900                 905                 910
Ser Glu Glu Val Val Lys Lys Met Lys Asn Tyr Trp Arg Gln Leu Leu
        915                 920                 925
Asn Ala Lys Leu Ile Thr Gln Arg Lys Phe Asp Asn Leu Thr Lys Ala
    930                 935                 940
Glu Arg Gly Gly Leu Ser Glu Leu Asp Lys Ala Gly Phe Ile Lys Arg
945                 950                 955                 960
Gln Leu Val Glu Thr Arg Gln Ile Thr Lys His Val Ala Gln Ile Leu
                965                 970                 975
Asp Ser Arg Met Asn Thr Lys Tyr Asp Glu Asn Asp Lys Leu Ile Arg
            980                 985                 990
Glu Val Lys Val Ile Thr Leu Lys  Ser Lys Leu Val Ser  Asp Phe Arg
        995                 1000                1005
Lys Asp  Phe Gln Phe Tyr Lys  Val Arg Glu Ile Asn  Asn Tyr His His
    1010                1015                1020
Ala  His Asp Ala Tyr Leu  Asn Ala Val Val Gly  Thr Ala Leu Ile Lys
1025                1030                1035                1040
Lys Tyr Pro Lys Leu  Glu Ser Glu Phe Val  Tyr Gly Asp Tyr Lys  Val
                1045                1050                1055
Tyr Asp Val Arg  Lys Met Ile Ala Lys  Ser Glu Gln Glu Ile  Gly Lys
            1060                1065                1070
Ala Thr Ala  Lys Tyr Phe Phe Tyr  Ser Asn Ile Met Asn  Phe Phe Lys
        1075                1080                1085
Thr Glu  Ile Thr Leu Ala Asn  Gly Glu Ile Arg Lys  Arg Pro Leu Ile
    1090                1095                1100
Glu  Thr Asn Gly Glu Thr  Gly Glu Ile Val Trp  Asp Lys Gly Arg Asp
1105                1110                1115                1120
Phe Ala Thr Val Arg  Lys Val Leu Ser Met  Pro Gln Val Asn Ile  Val
                1125                1130                1135
Lys Lys Thr Glu  Val Gln Thr Gly Gly  Phe Ser Lys Glu Ser  Ile Leu
            1140                1145                1150
Pro Lys Arg  Asn Ser Asp Lys Leu  Ile Ala Arg Lys Lys  Asp Trp Asp
        1155                1160                1165
Pro Lys  Lys Tyr Gly Gly Phe  Asp Ser Pro Thr Val  Ala Tyr Ser Val
    1170                1175                1180
Leu  Val Val Ala Lys Val  Glu Lys Gly Lys Ser  Lys Lys Leu Lys Ser
1185                1190                1195                1200
Val Lys Glu Leu Leu  Gly Ile Thr Ile Met  Glu Arg Ser Ser Phe  Glu
                1205                1210                1215
Lys Asn Pro Ile  Asp Phe Leu Glu Ala  Lys Gly Tyr Lys Glu  Val Lys
            1220                1225                1230
Lys Asp Leu  Ile Ile Lys Leu Pro  Lys Tyr Ser Leu Phe  Glu Leu Glu
        1235                1240                1245
Asn Gly  Arg Lys Arg Met Leu  Ala Ser Ala Gly Glu  Leu Gln Lys Gly
    1250                1255                1260
Asn  Glu Leu Ala Leu Pro  Ser Lys Tyr Val Asn  Phe Leu Tyr Leu Ala
1265                1270                1275                1280
Ser His Tyr Glu Lys  Leu Lys Gly Ser Pro  Glu Asp Asn Glu Gln  Lys
                1285                1290                1295
Gln Leu Phe Val  Glu Gln His Lys His  Tyr Leu Asp Glu Ile  Ile Glu
            1300                1305                1310
Gln Ile Ser  Glu Phe Ser Lys Arg  Val Ile Leu Ala Asp  Ala Asn Leu
        1315                1320                1325
Asp Lys  Val Leu Ser Ala Tyr  Asn Lys His Arg Asp  Lys Pro Ile Arg
    1330                1335                1340
Glu  Gln Ala Glu Asn Ile  Ile His Leu Phe Thr  Leu Thr Asn Leu Gly
1345                1350                1355                1360
Ala Pro Ala Ala Phe  Lys Tyr Phe Asp Thr  Thr Ile Asp Arg Lys  Arg
                1365                1370                1375
Tyr Thr Ser Thr  Lys Glu Val Leu Asp  Ala Thr Leu Ile His  Gln Ser
            1380                1385                1390

Ile Thr Gly Leu Tyr Glu Thr Arg Ile Asp Leu Ser Gln Leu Gly Gly
        1395                1400                1405
Asp Glu Gly Ala Pro Lys Lys Lys Arg Lys Val Gly Ser Ser Gly Ser
    1410                1415                1420
Pro Lys Lys Lys Arg Lys Val Ser Gly Ser Glu Thr Pro Gly Thr Ser
1425                1430                1435                1440
Glu Ser Ala Thr Pro Glu Ser Met Asp Ser Leu Leu Met Asn Arg Arg
                1445                1450                1455
Glu Phe Leu Tyr Gln Phe Lys Asn Val Arg Trp Ala Lys Gly Arg Arg
            1460                1465                1470
Glu Thr Tyr Leu Cys Tyr Val Val Lys Arg Arg Asp Ser Ala Thr Ser
        1475                1480                1485
Phe Ser Leu Asp Phe Gly Tyr Leu Arg Asn Lys Asn Gly Cys His Val
    1490                1495                1500
Glu Leu Leu Phe Leu Arg Tyr Ile Ser Asp Trp Asp Leu Asp Pro Gly
1505                1510                1515                1520
Arg Cys Tyr Arg Val Thr Trp Phe Ile Ser Trp Ser Pro Cys Tyr Asp
                1525                1530                1535
Cys Ala Arg His Val Ala Asp Phe Leu Arg Gly Asn Pro Asn Leu Ser
            1540                1545                1550
Leu Arg Ile Phe Thr Ala Arg Leu Tyr Phe Cys Glu Asp Arg Lys Ala
        1555                1560                1565
Glu Pro Glu Gly Leu Arg Arg Leu His Arg Ala Gly Val Gln Ile Ala
    1570                1575                1580
Ile Met Thr Phe Lys Asp Tyr Phe Tyr Cys Trp Asn Thr Phe Val Glu
1585                1590                1595                1600
Asn His Gly Arg Thr Phe Lys Ala Trp Glu Gly Leu His Glu Asn Ser
                1605                1610                1615
Val Arg Leu Ser Arg Gln Leu Arg Arg Ile Leu Leu Pro
            1620                1625

<210>  32
<211>  4917
<212>  DNA
<213>  Artificial Sequence

<220>
<223>  Coding sequence of ncas9-P182x

<400>  32
atggactata aggaccacga cggagactac aaggatcatg atattgatta caaagacgat      60
gacgataaga tggccccaaa gaagaagcgg aaggtcggta tccacggagt cccagcagct     120
accatggaca agaagtattc tatcggactg gccatcggga ctaatagcgt cgggtgggcc     180
gtgatcactg acgagtacaa ggtgccctct aagaagttca aggtgctcgg gaacaccgac     240
cggcattcca tcaagaaaaa tctgatcgga gctctcctct ttgattcagg ggagaccgct     300
gaagcaaccc gcctcaagcg gactgctaga cggcggtaca ccaggaggaa gaaccggatt     360
tgttaccttc aagagatatt ctccaacgaa atggcaaagg tcgacgacag cttcttccat     420
aggctggaag aatcattcct cgtggaagag gataagaagc atgaacggca tcccatcttc     480
ggtaatatcg tcgacgaggt ggcctatcac gagaaatacc caaccatcta ccatcttcgc     540
aaaaagctgg tggactcaac cgacaaggca gacctccggc ttatctacct ggccctggcc     600
cacatgatca agttcagagg ccacttcctg atcgagggcg acctcaatcc tgacaatagc     660
gatgtggata aactgttcat ccagctggtg cagacttaca accagctctt tgaagagaac     720
cccatcaatg caagcggagt cgatgccaag gccattctgt cagcccggct gtcaaagagc     780
cgcagacttg agaatcttat cgctcagctg ccgggtgaaa agaaaaatgg actgttcggg     840
aacctgattg ctctttcact tgggctgact cccaatttca agtctaattt cgacctggca     900
gaggatgcca agctgcaact gtccaaggac acctatgatg acgatctcga caacctcctg     960
gcccagatcg gtgaccaata cgccgacctt ttccttgctg ctaagaatct ttctgacgcc    1020
atcctgctgt ctgacattct ccgcgtgaac actgaaatca ccaaggcccc tctttcagct    1080
tcaatgatta agcggtatga tgagcaccac caggacctga ccctgcttaa ggcactcgtc    1140
cggcagcagc ttccggagaa gtacaaggaa atcttctttg accagtcaaa gaatggatac    1200
gccggctaca tcgacggagg tgcctcccaa gaggaatttt ataagtttat caaacctatc    1260
cttgagaaga tggacggcac cgaagagctc ctcgtgaaac tgaatcggga ggatctgctg    1320
cggaagcagc gcactttcga caatgggagc attccccacc agatccatct tggggagctt    1380

cacgccatcc ttcggcgcca agaggacttc tacccctttc ttaaggacaa cagggagaag 1440
attgagaaaa ttctcacttt ccgcatcccc tactacgtgg gacccctcgc cagaggaaat 1500
agccggtttg cttggatgac cagaaagtca gaagaaacta tcactccctg gaacttcgaa 1560
gaggtggtgg acaagggagc cagcgctcag tcattcatcg aacggatgac taacttcgat 1620
aagaacctcc ccaatgagaa ggtcctgccg aaacattccc tgctctacga gtactttacc 1680
gtgtacaacg agctgaccaa ggtgaaatat gtcaccgaag ggatgaggaa gcccgcattc 1740
ctgtcaggcg aacaaaagaa ggcaattgtg gaccttctgt tcaagaccaa tagaaaggtg 1800
accgtgaagc agctgaagga ggactatttc aagaaaattg aatgcttcga ctctgtggag 1860
attagcgggg tcgaagatcg gttcaacgca agcctgggta cctaccatga tctgcttaag 1920
atcatcaagg acaaggattt tctggacaat gaggagaaag aggacatcct tgaggacatt 1980
gtcctgactc tcactctgtt cgaggaccgg gaaatgatcg aggagaggct taagacctac 2040
gcccatctgt tcgacgataa agtgatgaag caacttaaac ggagaagata taccggatgg 2100
ggacgcctta gccgcaaact catcaacgga atccgggaca aacagagcgg aaagaccatt 2160
cttgatttcc ttaagagcga cggattcgct aatcgcaact tcatgcaact tatccatgat 2220
gattccctga cctttaagga ggacatccag aaggcccaag tgtctggaca aggtgactca 2280
ctgcacgagc atatcgcaaa tctggctggt tcacccgcta ttaagaaggg tattctccag 2340
accgtgaaag tcgtggacga gctggtcaag gtgatgggtc gccataaacc agagaacatt 2400
gtcatcgaga tggccaggga aaaccagact acccagaagg gacagaagaa cagcagggag 2460
cggatgaaaa gaattgagga agggattaag gagctcgggt cacagatcct taaagagcac 2520
ccggtggaaa acacccagct tcagaatgag aagctctatc tgtactacct tcaaaatgga 2580
cgcgatatgt atgtggacca agagcttgat atcaacaggc tctcagacta cgacgtggac 2640
catatcgtcc ctcagagctt cctcaaagac gactcaattg acaataaggt gctgactcgc 2700
tcagacaaga accggggaaa gtcagataac gtgccctcag aggaagtcgt gaaaaagatg 2760
aagaactatt ggcgccagct tctgaacgca aagctgatca ctcagcggaa gttcgacaat 2820
ctcactaagg ctgagagggg cggactgagc gaactggaca aagcaggatt cattaaacgg 2880
caacttgtgg agactcggca gattactaaa catgtagccc aaatccttga ctcacgcatg 2940
aataccaagt acgacgaaaa cgacaaactt atccgcgagg tgaaggtgat taccctgaag 3000
tccaagctgg tcagcgattt cagaaaggac tttcaattct acaaagtgcg ggagatcaat 3060
aactatcatc atgctcatga cgcatatctg aatgccgtgg tgggaaccgc cctgatcaag 3120
aagtacccaa agctggaaag cgagttcgtg tacggagact acaaggtcta cgacgtgcgc 3180
aagatgattg ccaaatctga gcaggagatc ggaaaggcca ccgcaaagta cttcttctac 3240
agcaacatca tgaatttctt caagaccgaa atcacccttg caaacggtga gatccggaag 3300
aggccgctca tcgagactaa tggggagact ggcgaaatcg tgtgggacaa gggcagagat 3360
ttcgctaccg tgcgcaaagt gctttctatg cctcaagtga acatcgtgaa gaaaaccgag 3420
gtgcaaaccg gaggcttttc taaggaatca atcctcccca agcgcaactc cgacaagctc 3480
attgcaagga agaaggattg ggaccctaag aagtacggcg gattcgattc accaactgtg 3540
gcttattctg tcctggtcgt ggctaaggtg gaaaaaggaa agtctaagaa gctcaagagc 3600
gtgaaggaac tgctgggtat caccattatg gagcgcagct ccttcgagaa gaacccaatt 3660
gactttctcg aagccaaagg ttacaaggaa gtcaagaagg accttatcat caagctccca 3720
aagtatagcc tgttcgaact ggagaatggg cggaagcgga tgctcgcctc cgctggcgaa 3780
cttcagaagg gtaatgagct ggctctcccc tccaagtacg tgaatttcct ctaccttgca 3840
agccattacg agaagctgaa ggggagcccc gaggacaacg agcaaaagca actgtttgtg 3900
gagcagcata agcattatct ggacgagatc attgagcaga tttccgagtt ttctaaacgc 3960
gtcattctcg ctgatgccaa cctcgataaa gtccttagcg catacaataa gcacagagac 4020
aaaccaattc gggagcaggc tgagaatatc atccacctgt tcaccctcac caatcttggt 4080
gcccctgccg cattcaagta cttcgacacc accatcgacc ggaaacgcta tacctccacc 4140
aaagaagtgc tggacgccac cctcatccac cagagcatca ccggacttta cgaaactcgg 4200
attgacctct cacagctcgg aggggatgag ggagctccca agaaaaagcg caaggtaggt 4260
agttccggat ctccgaaaaa gaaacgcaaa gttggtagtg atgctttaga cgattttgac 4320
ttagatatgc ttggttcaga cgcgttagac gacttcggtg gaggatccat ggacagcctc 4380
ttgatgaacc ggaggaagtt tctttaccaa ttcaaaaatg tccgctgggc taagggtcgg 4440
cgtgagacct acctgtgcta cgtagtgaag aggcgtgaca gtgctacatc cttttcactg 4500
gactttggtt atcttcgcaa taagaacggc tgccacgtgg aattgctctt cctccgctac 4560
atctcggact gggacctaga ccctggccgc tgctaccgcg tcacctggtt cacctcctgg 4620
agcccctgct acgactgtgc ccgacatgtg gccgactttc tgcgagggaa ccccaacctc 4680
agtctgagga tcttcaccgc gcgcctctac ttctgtgagg accgcaaggc tgagcccgag 4740
gggctgcggc ggctgcaccg cgccggggtg caaatagcca tcatgacctt caaagattat 4800
ttttactgct ggaatacttt tgtagaaaac catgaaagaa ctttcaaagc ctgggaaggg 4860
ctgcatgaaa attcagttcg tctctccaga cagcttcggc gcatcctttt gccctga 4917

<210> 33
<211> 1638
<212> PRT

<213>  Artificial Sequence

<220>
<223>  Amino acid sequence of ncas9-P182x

<400>  33
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Gly Val Pro Ala Ala Thr Met Asp Lys Lys Tyr Ser Ile
        35                  40                  45
Gly Leu Ala Ile Gly Thr Asn Ser Val Gly Trp Ala Val Ile Thr Asp
    50                  55                  60
Glu Tyr Lys Val Pro Ser Lys Lys Phe Lys Val Leu Gly Asn Thr Asp
65                  70                  75                  80
Arg His Ser Ile Lys Lys Asn Leu Ile Gly Ala Leu Leu Phe Asp Ser
                85                  90                  95
Gly Glu Thr Ala Glu Ala Thr Arg Leu Lys Arg Thr Ala Arg Arg Arg
            100                 105                 110
Tyr Thr Arg Arg Lys Asn Arg Ile Cys Tyr Leu Gln Glu Ile Phe Ser
        115                 120                 125
Asn Glu Met Ala Lys Val Asp Asp Ser Phe Phe His Arg Leu Glu Glu
    130                 135                 140
Ser Phe Leu Val Glu Glu Asp Lys Lys His Glu Arg His Pro Ile Phe
145                 150                 155                 160
Gly Asn Ile Val Asp Glu Val Ala Tyr His Glu Lys Tyr Pro Thr Ile
                165                 170                 175
Tyr His Leu Arg Lys Lys Leu Val Asp Ser Thr Asp Lys Ala Asp Leu
            180                 185                 190
Arg Leu Ile Tyr Leu Ala Leu Ala His Met Ile Lys Phe Arg Gly His
        195                 200                 205
Phe Leu Ile Glu Gly Asp Leu Asn Pro Asp Asn Ser Asp Val Asp Lys
    210                 215                 220
Leu Phe Ile Gln Leu Val Gln Thr Tyr Asn Gln Leu Phe Glu Glu Asn
225                 230                 235                 240
Pro Ile Asn Ala Ser Gly Val Asp Ala Lys Ala Ile Leu Ser Ala Arg
                245                 250                 255
Leu Ser Lys Ser Arg Arg Leu Glu Asn Leu Ile Ala Gln Leu Pro Gly
            260                 265                 270
Glu Lys Lys Asn Gly Leu Phe Gly Asn Leu Ile Ala Leu Ser Leu Gly
        275                 280                 285
Leu Thr Pro Asn Phe Lys Ser Asn Phe Asp Leu Ala Glu Asp Ala Lys
    290                 295                 300
Leu Gln Leu Ser Lys Asp Thr Tyr Asp Asp Asp Leu Asp Asn Leu Leu
305                 310                 315                 320
Ala Gln Ile Gly Asp Gln Tyr Ala Asp Leu Phe Leu Ala Ala Lys Asn
                325                 330                 335
Leu Ser Asp Ala Ile Leu Leu Ser Asp Ile Leu Arg Val Asn Thr Glu
            340                 345                 350
Ile Thr Lys Ala Pro Leu Ser Ala Ser Met Ile Lys Arg Tyr Asp Glu
        355                 360                 365
His His Gln Asp Leu Thr Leu Leu Lys Ala Leu Val Arg Gln Gln Leu
    370                 375                 380
Pro Glu Lys Tyr Lys Glu Ile Phe Phe Asp Gln Ser Lys Asn Gly Tyr
385                 390                 395                 400
Ala Gly Tyr Ile Asp Gly Gly Ala Ser Gln Glu Glu Phe Tyr Lys Phe
                405                 410                 415
Ile Lys Pro Ile Leu Glu Lys Met Asp Gly Thr Glu Glu Leu Leu Val
            420                 425                 430
Lys Leu Asn Arg Glu Asp Leu Leu Arg Lys Gln Arg Thr Phe Asp Asn
        435                 440                 445
Gly Ser Ile Pro His Gln Ile His Leu Gly Glu Leu His Ala Ile Leu

450                 455                 460
Arg Arg Gln Glu Asp Phe Tyr Pro Phe Leu Lys Asp Asn Arg Glu Lys
465                 470                 475                 480
Ile Glu Lys Ile Leu Thr Phe Arg Ile Pro Tyr Tyr Val Gly Pro Leu
                485                 490                 495
Ala Arg Gly Asn Ser Arg Phe Ala Trp Met Thr Arg Lys Ser Glu Glu
            500                 505                 510
Thr Ile Thr Pro Trp Asn Phe Glu Glu Val Val Asp Lys Gly Ala Ser
        515                 520                 525
Ala Gln Ser Phe Ile Glu Arg Met Thr Asn Phe Asp Lys Asn Leu Pro
    530                 535                 540
Asn Glu Lys Val Leu Pro Lys His Ser Leu Leu Tyr Glu Tyr Phe Thr
545                 550                 555                 560
Val Tyr Asn Glu Leu Thr Lys Val Lys Tyr Val Thr Glu Gly Met Arg
                565                 570                 575
Lys Pro Ala Phe Leu Ser Gly Glu Gln Lys Lys Ala Ile Val Asp Leu
            580                 585                 590
Leu Phe Lys Thr Asn Arg Lys Val Thr Val Lys Gln Leu Lys Glu Asp
        595                 600                 605
Tyr Phe Lys Lys Ile Glu Cys Phe Asp Ser Val Glu Ile Ser Gly Val
    610                 615                 620
Glu Asp Arg Phe Asn Ala Ser Leu Gly Thr Tyr His Asp Leu Leu Lys
625                 630                 635                 640
Ile Ile Lys Asp Lys Asp Phe Leu Asp Asn Glu Glu Lys Glu Asp Ile
                645                 650                 655
Leu Glu Asp Ile Val Leu Thr Leu Thr Leu Phe Glu Asp Arg Glu Met
            660                 665                 670
Ile Glu Glu Arg Leu Lys Thr Tyr Ala His Leu Phe Asp Asp Lys Val
        675                 680                 685
Met Lys Gln Leu Lys Arg Arg Arg Tyr Thr Gly Trp Gly Arg Leu Ser
    690                 695                 700
Arg Lys Leu Ile Asn Gly Ile Arg Asp Lys Gln Ser Gly Lys Thr Ile
705                 710                 715                 720
Leu Asp Phe Leu Lys Ser Asp Gly Phe Ala Asn Arg Asn Phe Met Gln
                725                 730                 735
Leu Ile His Asp Asp Ser Leu Thr Phe Lys Glu Asp Ile Gln Lys Ala
            740                 745                 750
Gln Val Ser Gly Gln Gly Asp Ser Leu His Glu His Ile Ala Asn Leu
        755                 760                 765
Ala Gly Ser Pro Ala Ile Lys Lys Gly Ile Leu Gln Thr Val Lys Val
    770                 775                 780
Val Asp Glu Leu Val Lys Val Met Gly Arg His Lys Pro Glu Asn Ile
785                 790                 795                 800
Val Ile Glu Met Ala Arg Glu Asn Gln Thr Thr Gln Lys Gly Gln Lys
                805                 810                 815
Asn Ser Arg Glu Arg Met Lys Arg Ile Glu Glu Gly Ile Lys Glu Leu
            820                 825                 830
Gly Ser Gln Ile Leu Lys Glu His Pro Val Glu Asn Thr Gln Leu Gln
        835                 840                 845
Asn Glu Lys Leu Tyr Leu Tyr Tyr Leu Gln Asn Gly Arg Asp Met Tyr
    850                 855                 860
Val Asp Gln Glu Leu Asp Ile Asn Arg Leu Ser Asp Tyr Asp Val Asp
865                 870                 875                 880
His Ile Val Pro Gln Ser Phe Leu Lys Asp Asp Ser Ile Asp Asn Lys
                885                 890                 895
Val Leu Thr Arg Ser Asp Lys Asn Arg Gly Lys Ser Asp Asn Val Pro
            900                 905                 910
Ser Glu Glu Val Val Lys Lys Met Lys Asn Tyr Trp Arg Gln Leu Leu
        915                 920                 925
Asn Ala Lys Leu Ile Thr Gln Arg Lys Phe Asp Asn Leu Thr Lys Ala
    930                 935                 940
Glu Arg Gly Gly Leu Ser Glu Leu Asp Lys Ala Gly Phe Ile Lys Arg
945                 950                 955                 960

Gln Leu Val Glu Thr Arg Gln Ile Thr Lys His Val Ala Gln Ile Leu
                965                 970                 975
Asp Ser Arg Met Asn Thr Lys Tyr Asp Glu Asn Asp Lys Leu Ile Arg
            980                 985                 990
Glu Val Lys Val Ile Thr Leu Lys Ser Lys Leu Val Ser Asp Phe Arg
        995                 1000                1005
Lys Asp Phe Gln Phe Tyr Lys Val Arg Glu Ile Asn Asn Tyr His His
    1010                1015                1020
Ala His Asp Ala Tyr Leu Asn Ala Val Val Gly Thr Ala Leu Ile Lys
1025                1030                1035                1040
Lys Tyr Pro Lys Leu Glu Ser Glu Phe Val Tyr Gly Asp Tyr Lys Val
                1045                1050                1055
Tyr Asp Val Arg Lys Met Ile Ala Lys Ser Glu Gln Glu Ile Gly Lys
            1060                1065                1070
Ala Thr Ala Lys Tyr Phe Phe Tyr Ser Asn Ile Met Asn Phe Phe Lys
        1075                1080                1085
Thr Glu Ile Thr Leu Ala Asn Gly Glu Ile Arg Lys Arg Pro Leu Ile
    1090                1095                1100
Glu Thr Asn Gly Glu Thr Gly Glu Ile Val Trp Asp Lys Gly Arg Asp
1105                1110                1115                1120
Phe Ala Thr Val Arg Lys Val Leu Ser Met Pro Gln Val Asn Ile Val
                1125                1130                1135
Lys Lys Thr Glu Val Gln Thr Gly Gly Phe Ser Lys Glu Ser Ile Leu
            1140                1145                1150
Pro Lys Arg Asn Ser Asp Lys Leu Ile Ala Arg Lys Lys Asp Trp Asp
        1155                1160                1165
Pro Lys Lys Tyr Gly Gly Phe Asp Ser Pro Thr Val Ala Tyr Ser Val
    1170                1175                1180
Leu Val Val Ala Lys Val Glu Lys Gly Lys Ser Lys Lys Leu Lys Ser
1185                1190                1195                1200
Val Lys Glu Leu Leu Gly Ile Thr Ile Met Glu Arg Ser Ser Phe Glu
                1205                1210                1215
Lys Asn Pro Ile Asp Phe Leu Glu Ala Lys Gly Tyr Lys Glu Val Lys
            1220                1225                1230
Lys Asp Leu Ile Ile Lys Leu Pro Lys Tyr Ser Leu Phe Glu Leu Glu
        1235                1240                1245
Asn Gly Arg Lys Arg Met Leu Ala Ser Ala Gly Glu Leu Gln Lys Gly
    1250                1255                1260
Asn Glu Leu Ala Leu Pro Ser Lys Tyr Val Asn Phe Leu Tyr Leu Ala
1265                1270                1275                1280
Ser His Tyr Glu Lys Leu Lys Gly Ser Pro Glu Asp Asn Glu Gln Lys
                1285                1290                1295
Gln Leu Phe Val Glu Gln His Lys His Tyr Leu Asp Glu Ile Ile Glu
            1300                1305                1310
Gln Ile Ser Glu Phe Ser Lys Arg Val Ile Leu Ala Asp Ala Asn Leu
        1315                1320                1325
Asp Lys Val Leu Ser Ala Tyr Asn Lys His Arg Asp Lys Pro Ile Arg
    1330                1335                1340
Glu Gln Ala Glu Asn Ile Ile His Leu Phe Thr Leu Thr Asn Leu Gly
1345                1350                1355                1360
Ala Pro Ala Ala Phe Lys Tyr Phe Asp Thr Thr Ile Asp Arg Lys Arg
                1365                1370                1375
Tyr Thr Ser Thr Lys Glu Val Leu Asp Ala Thr Leu Ile His Gln Ser
            1380                1385                1390
Ile Thr Gly Leu Tyr Glu Thr Arg Ile Asp Leu Ser Gln Leu Gly Gly
        1395                1400                1405
Asp Glu Gly Ala Pro Lys Lys Lys Arg Lys Val Gly Ser Ser Gly Ser
    1410                1415                1420
Pro Lys Lys Lys Arg Lys Val Gly Ser Asp Ala Leu Asp Asp Phe Asp
1425                1430                1435                1440
Leu Asp Met Leu Gly Ser Asp Ala Leu Asp Asp Phe Gly Gly Gly Ser
                1445                1450                1455
Met Asp Ser Leu Leu Met Asn Arg Arg Lys Phe Leu Tyr Gln Phe Lys

1460                    1465                    1470
Asn Val Arg  Trp Ala Lys Gly Arg  Arg Glu Thr Tyr Leu  Cys Tyr Val
        1475                    1480                    1485
Val Lys  Arg Arg Asp Ser Ala  Thr Ser Phe Ser Leu  Asp Phe Gly Tyr
    1490                    1495                    1500
Leu  Arg Asn Lys Asn Gly  Cys His Val Glu Leu  Leu Phe Leu Arg Tyr
1505                    1510                    1515                    1520
Ile Ser Asp Trp Asp  Leu Asp Pro Gly Arg  Cys Tyr Arg Val Thr  Trp
                1525                    1530                    1535
Phe Thr Ser Trp  Ser Pro Cys Tyr Asp  Cys Ala Arg His Val  Ala Asp
            1540                    1545                    1550
Phe Leu Arg  Gly Asn Pro Asn Leu  Ser Leu Arg Ile Phe  Thr Ala Arg
        1555                    1560                    1565
Leu Tyr  Phe Cys Glu Asp Arg  Lys Ala Glu Pro Glu  Gly Leu Arg Arg
    1570                    1575                    1580
Leu  His Arg Ala Gly Val  Gln Ile Ala Ile Met  Thr Phe Lys Asp Tyr
1585                    1590                    1595                    1600
Phe Tyr Cys Trp Asn  Thr Phe Val Glu Asn  His Glu Arg Thr Phe  Lys
                1605                    1610                    1615
Ala Trp Glu Gly  Leu His Glu Asn Ser  Val Arg Leu Ser Arg  Gln Leu
            1620                    1625                    1630
Arg Arg Ile  Leu Leu Pro
        1635

<210>  34
<211>  3
<212>  DNA
<213>  Artificial Sequence

<220>
<223>  PAM sequence

<220>
<221>  misc_feature
<222>  (1)..(1)
<223>  n is a, c, g or t

<400>  34
ngg                                                                     3

<210>  35
<211>  5
<212>  DNA
<213>  Artificial Sequence

<220>
<223>  PAM sequence

<220>
<221>  misc_feature
<222>  (1)..(2)
<223>  n is a, c, g or t

<220>
<221>  misc_feature
<222>  (4)..(5)
<223>  r is a or g

<400>  35
nngrr                                                                   5

<210>  36
<211>  6

<212>  DNA
<213>  Artificial Sequence

<220>
<223>  PAM sequence

<220>
<221>  misc_feature
<222>  (1)..(2)
<223>  n is a, c, g or t

<400>  36
nnagaa                                                                  6

<210>  37
<211>  6
<212>  DNA
<213>  Artificial Sequence

<220>
<223>  PAM sequence

<220>
<221>  misc_feature
<222>  (1)..(2)
<223>  n is a, c, g or t

<220>
<221>  misc_feature
<222>  (4)..(5)
<223>  r is a or g

<400>  37
nngrrt                                                                  6

<210>  38
<211>  3
<212>  DNA
<213>  Artificial Sequence

<220>
<223>  PAM sequence

<400>  38
tgg                                                                     3

<210>  39
<211>  6
<212>  DNA
<213>  Artificial Sequence

<220>
<223>  PAM sequence

<220>
<221>  misc_feature
<222>  (1)..(3)
<223>  n is a, c, g or t

<220>
<221>  misc_feature
<222>  (4)..(5)
<223>  r is a or g

<400>  39
nnnrrt                                                                  6

<210>  40
<211>  4
<212>  PRT
<213>  Artificial Sequence

<220>
<223>  linker

<400>  40
Ser Gly Gly Ser
1

<210>  41
<211>  5
<212>  PRT
<213>  Artificial Sequence

<220>
<223>  linker

<400>  41
Gly Ser Ser Gly Ser
1               5

<210>  42
<211>  4
<212>  PRT
<213>  Artificial Sequence

<220>
<223>  linker

<400>  42
Gly Gly Gly Ser
1

<210>  43
<211>  5
<212>  PRT
<213>  Artificial Sequence

<220>
<223>  linker

<400>  43
Gly Gly Gly Gly Ser
1               5

<210>  44
<211>  5
<212>  PRT
<213>  Artificial Sequence

<220>
<223>  linker

<400>  44
Ser Ser Ser Ser Gly
1               5

<210>  45
<211>  5
<212>  PRT
<213>  Artificial Sequence

<220>
<223>  linker

<400>  45
Gly Ser Gly Ser Ala
1               5

<210>  46
<211>  5
<212>  PRT
<213>  Artificial Sequence

<220>
<223>  linker

<400>  46
Gly Gly Ser Gly Gly
1               5

<210>  47
<211>  4890
<212>  DNA
<213>  Artificial Sequence

<220>
<223>  Coding sequence of dCas9-XTEN-AID P182X

<400>  47
atggactata aggaccacga cggagactac aaggatcatg atattgatta caaagacgat      60
gacgataaga tggccccaaa gaagaagcgg aaggtcggta tccacggagt cccagcagct     120
accatggaca agaagtattc tatcggactg gccatcggga ctaatagcgt cgggtgggcc      180
gtgatcactg acgagtacaa ggtgccctct aagaagttca aggtgctcgg gaacaccgac     240
cggcattcca tcaagaaaaa tctgatcgga gctctcctct ttgattcagg ggagaccgct     300
gaagcaaccc gcctcaagcg gactgctaga cggcggtaca ccaggaggaa gaaccggatt     360
tgttaccttc aagagatatt ctccaacgaa atggcaaagg tcgacgacag cttcttccat     420
aggctggaag aatcattcct cgtggaagag gataagaagc atgaacggca tcccatcttc     480
ggtaatatcg tcgacgaggt ggcctatcac gagaaatacc caaccatcta ccatcttcgc     540
aaaaagctgg tggactcaac cgacaaggca gacctccggc ttatctacct ggccctggcc     600
cacatgatca agttcagagg ccacttcctg atcgagggcg acctcaatcc tgacaatagc     660
gatgtggata aactgttcat ccagctggtg cagacttaca accagctctt tgaagagaac     720
cccatcaatg caagcggagt cgatgccaag gccattctgt cagcccggct gtcaaagagc     780
cgcagacttg agaatcttat cgctcagctg ccgggtgaaa agaaaaatgg actgttcggg     840
aacctgattg ctctttcact tgggctgact cccaatttca agtctaattt cgacctggca     900
gaggatgcca agctgcaact gtccaaggac acctatgatg acgatctcga caacctcctg     960
gcccagatcg gtgaccaata cgccgacctt ttccttgctg ctaagaatct ttctgacgcc    1020
atcctgctgt ctgacattct ccgcgtgaac actgaaatca ccaaggcccc tctttcagct    1080
tcaatgatta agcggtatga tgagcaccac caggacctga ccctgcttaa ggcactcgtc    1140
cggcagcagc ttccggagaa gtacaaggaa atcttctttg accagtcaaa gaatggatac    1200
gccggctaca tcgacggagg tgcctcccaa gaggaatttt ataagtttat caaacctatc    1260
cttgagaaga tggacggcac cgaagagctc ctcgtgaaac tgaatcggga ggatctgctg    1320
cggaagcagc gcactttcga caatgggagc attccccacc agatccatct tggggagctt    1380
cacgccatcc ttcggcgcca agaggacttc tacccctttc ttaaggacaa cagggagaag    1440
attgagaaaa ttctcacttt ccgcatcccc tactacgtgg gacccctcgc cagaggaaat    1500
agccggtttg cttggatgac cagaaagtca gaagaaacta tcactccctg gaacttcgaa    1560
gaggtggtgg acaagggagc cagcgctcag tcattcatcg aacggatgac taacttcgat    1620
aagaacctcc ccaatgagaa ggtcctgccg aaacattccc tgctctacga gtactttacc    1680
gtgtacaacg agctgaccaa ggtgaaatat gtcaccgaag ggatgaggaa gcccgcattc    1740

ctgtcaggcg aacaaaagaa ggcaattgtg gaccttctgt tcaagaccaa tagaaaggtg 1800
accgtgaagc agctgaagga ggactatttc aagaaaattg aatgcttcga ctctgtggag 1860
attagcgggg tcgaagatcg gttcaacgca agcctgggta cctaccatga tctgcttaag 1920
atcatcaagg acaaggattt tctggacaat gaggagaaag aggacatcct tgaggacatt 1980
gtcctgactc tcactctgtt cgaggaccgg gaaatgatcg aggagaggct taagacctac 2040
gcccatctgt tcgacgataa agtgatgaag caacttaaac ggagaagata taccggatgg 2100
ggacgcctta gccgcaaact catcaacgga atccgggaca aacagagcgg aaagaccatt 2160
cttgatttcc ttaagagcga cggattcgct aatcgcaact tcatgcaact tatccatgat 2220
gattccctga cctttaagga ggacatccag aaggcccaag tgtctggaca aggtgactca 2280
ctgcacgagc atatcgcaaa tctggctggt tcacccgcta ttaagaaggg tattctccag 2340
accgtgaaag tcgtggacga gctggtcaag gtgatgggtc gccataaacc agagaacatt 2400
gtcatcgaga tggccaggga aaaccagact acccagaagg gacagaagaa cagcagggag 2460
cggatgaaaa gaattgagga agggattaag gagctcgggt cacagatcct taaagagcac 2520
ccggtggaaa acacccagct tcagaatgag aagctctatc tgtactacct tcaaaatgga 2580
cgcgatatgt atgtggacca agagcttgat atcaacaggc tctcagacta cgacgtggac 2640
gccatcgtcc ctcagagctt cctcaaagac gactcaattg acaataaggt gctgactcgc 2700
tcagacaaga accggggaaa gtcagataac gtgccctcag aggaagtcgt gaaaaagatg 2760
aagaactatt ggcgccagct tctgaacgca aagctgatca ctcagcggaa gttcgacaat 2820
ctcactaagg ctgagagggg cggactgagc gaactggaca aagcaggatt cattaaacgg 2880
caacttgtgg agactcggca gattactaaa catgtagccc aaatccttga ctcacgcatg 2940
aataccaagt acgacgaaaa cgacaaactt atccgcgagg tgaaggtgat taccctgaag 3000
tccaagctgg tcagcgattt cagaaaggac tttcaattct acaaagtgcg ggagatcaat 3060
aactatcatc atgctcatga cgcatatctg aatgccgtgg tgggaaccgc cctgatcaag 3120
aagtacccaa agctggaaag cgagttcgtg tacggagact acaaggtcta cgacgtgcgc 3180
aagatgattg ccaaatctga gcaggagatc ggaaaggcca ccgcaaagta cttcttctac 3240
agcaacatca tgaatttctt caagaccgaa atcacccttg caaacggtga gatccggaag 3300
aggccgctca tcgagactaa tggggagact ggcgaaatcg tgtgggacaa gggcagagat 3360
ttcgctaccg tgcgcaaagt gctttctatg cctcaagtga acatcgtgaa gaaaaccgag 3420
gtgcaaaccg gaggcttttc taaggaatca atcctcccca agcgcaactc cgacaagctc 3480
attgcaagga agaaggattg ggaccctaag aagtacggcg gattcgattc accaactgtg 3540
gcttattctg tcctggtcgt ggctaaggtg gaaaaaggaa agtctaagaa gctcaagagc 3600
gtgaaggaac tgctgggtat caccattatg gagcgcagct ccttcgagaa gaacccaatt 3660
gactttctcg aagccaaagg ttacaaggaa gtcaagaagg accttatcat caagctccca 3720
aagtatagcc tgttcgaact ggagaatggg cggaagcgga tgctcgcctc cgctggcgaa 3780
cttcagaagg gtaatgagct ggctctcccc tccaagtacg tgaatttcct ctaccttgca 3840
agccattacg agaagctgaa ggggagcccc gaggacaacg agcaaaagca actgtttgtg 3900
gagcagcata agcattatct ggacgagatc attgagcaga tttccgagtt ttctaaacgc 3960
gtcattctcg ctgatgccaa cctcgataaa gtccttagcg catacaataa gcacagagac 4020
aaaccaattc gggagcaggc tgagaatatc atccacctgt tcaccctcac caatcttggt 4080
gcccctgccg cattcaagta cttcgacacc accatcgacc ggaaacgcta tacctccacc 4140
aaagaagtgc tggacgccac cctcatccac cagagcatca ccggacttta cgaaactcgg 4200
attgacctct cacagctcgg aggggatgag ggagctccca agaaaaagcg caaggtaggt 4260
agttccggat ctccgaaaaa gaaacgcaaa gttagcggca gcgagactcc cgggacctca 4320
gagtccgcca cacccgaaag tatggacagc ctcttgatga accggaggaa gtttctttac 4380
caattcaaaa atgtccgctg ggctaagggt cggcgtgaga cctacctgtg ctacgtagtg 4440
aagaggcgtg acagtgctac atccttttca ctggactttg gttatcttcg caataagaac 4500
ggctgccacg tggaattgct cttcctccgc tacatctcgg actgggacct agaccctggc 4560
cgctgctacc gcgtcacctg gttcacctcc tggagcccct gctacgactg tgcccgacat 4620
gtggccgact ttctgcgagg gaaccccaac ctcagtctga ggatcttcac cgcgcgcctc 4680
tacttctgtg aggaccgcaa ggctgagccc gaggggctgc ggcggctgca ccgcgccggg 4740
gtgcaaatag ccatcatgac cttcaaagat tatttttact gctggaatac ttttgtagaa 4800
aaccatgaaa gaactttcaa agcctgggaa gggctgcatg aaaattcagt tcgtctctcc 4860
agacagcttc ggcgcatcct tttgccctga 4890

<210> 48
<211> 1629
<212> PRT
<213> Artificial Sequence

<220>
<223> Amino acid sequence of dCas9-XTEN-AID P182X

<400> 48

Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Gly Val Pro Ala Ala Thr Met Asp Lys Lys Tyr Ser Ile
        35                  40                  45
Gly Leu Ala Ile Gly Thr Asn Ser Val Gly Trp Ala Val Ile Thr Asp
    50                  55                  60
Glu Tyr Lys Val Pro Ser Lys Lys Phe Lys Val Leu Gly Asn Thr Asp
65                  70                  75                  80
Arg His Ser Ile Lys Lys Asn Leu Ile Gly Ala Leu Leu Phe Asp Ser
                85                  90                  95
Gly Glu Thr Ala Glu Ala Thr Arg Leu Lys Arg Thr Ala Arg Arg Arg
            100                 105                 110
Tyr Thr Arg Arg Lys Asn Arg Ile Cys Tyr Leu Gln Glu Ile Phe Ser
        115                 120                 125
Asn Glu Met Ala Lys Val Asp Asp Ser Phe Phe His Arg Leu Glu Glu
    130                 135                 140
Ser Phe Leu Val Glu Glu Asp Lys Lys His Glu Arg His Pro Ile Phe
145                 150                 155                 160
Gly Asn Ile Val Asp Glu Val Ala Tyr His Glu Lys Tyr Pro Thr Ile
                165                 170                 175
Tyr His Leu Arg Lys Lys Leu Val Asp Ser Thr Asp Lys Ala Asp Leu
            180                 185                 190
Arg Leu Ile Tyr Leu Ala Leu Ala His Met Ile Lys Phe Arg Gly His
        195                 200                 205
Phe Leu Ile Glu Gly Asp Leu Asn Pro Asp Asn Ser Asp Val Asp Lys
    210                 215                 220
Leu Phe Ile Gln Leu Val Gln Thr Tyr Asn Gln Leu Phe Glu Glu Asn
225                 230                 235                 240
Pro Ile Asn Ala Ser Gly Val Asp Ala Lys Ala Ile Leu Ser Ala Arg
                245                 250                 255
Leu Ser Lys Ser Arg Arg Leu Glu Asn Leu Ile Ala Gln Leu Pro Gly
            260                 265                 270
Glu Lys Lys Asn Gly Leu Phe Gly Asn Leu Ile Ala Leu Ser Leu Gly
        275                 280                 285
Leu Thr Pro Asn Phe Lys Ser Asn Phe Asp Leu Ala Glu Asp Ala Lys
    290                 295                 300
Leu Gln Leu Ser Lys Asp Thr Tyr Asp Asp Asp Leu Asp Asn Leu Leu
305                 310                 315                 320
Ala Gln Ile Gly Asp Gln Tyr Ala Asp Leu Phe Leu Ala Ala Lys Asn
                325                 330                 335
Leu Ser Asp Ala Ile Leu Leu Ser Asp Ile Leu Arg Val Asn Thr Glu
            340                 345                 350
Ile Thr Lys Ala Pro Leu Ser Ala Ser Met Ile Lys Arg Tyr Asp Glu
        355                 360                 365
His His Gln Asp Leu Thr Leu Leu Lys Ala Leu Val Arg Gln Gln Leu
    370                 375                 380
Pro Glu Lys Tyr Lys Glu Ile Phe Phe Asp Gln Ser Lys Asn Gly Tyr
385                 390                 395                 400
Ala Gly Tyr Ile Asp Gly Gly Ala Ser Gln Glu Glu Phe Tyr Lys Phe
                405                 410                 415
Ile Lys Pro Ile Leu Glu Lys Met Asp Gly Thr Glu Glu Leu Leu Val
            420                 425                 430
Lys Leu Asn Arg Glu Asp Leu Leu Arg Lys Gln Arg Thr Phe Asp Asn
        435                 440                 445
Gly Ser Ile Pro His Gln Ile His Leu Gly Glu Leu His Ala Ile Leu
    450                 455                 460
Arg Arg Gln Glu Asp Phe Tyr Pro Phe Leu Lys Asp Asn Arg Glu Lys
465                 470                 475                 480
Ile Glu Lys Ile Leu Thr Phe Arg Ile Pro Tyr Tyr Val Gly Pro Leu
                485                 490                 495
Ala Arg Gly Asn Ser Arg Phe Ala Trp Met Thr Arg Lys Ser Glu Glu

                    500                     505                     510
Thr Ile Thr Pro Trp Asn Phe Glu Glu Val Val Asp Lys Gly Ala Ser
        515                     520                     525
Ala Gln Ser Phe Ile Glu Arg Met Thr Asn Phe Asp Lys Asn Leu Pro
    530                     535                     540
Asn Glu Lys Val Leu Pro Lys His Ser Leu Leu Tyr Glu Tyr Phe Thr
545                     550                     555                     560
Val Tyr Asn Glu Leu Thr Lys Val Lys Tyr Val Thr Glu Gly Met Arg
                565                     570                     575
Lys Pro Ala Phe Leu Ser Gly Glu Gln Lys Lys Ala Ile Val Asp Leu
            580                     585                     590
Leu Phe Lys Thr Asn Arg Lys Val Thr Val Lys Gln Leu Lys Glu Asp
        595                     600                     605
Tyr Phe Lys Lys Ile Glu Cys Phe Asp Ser Val Glu Ile Ser Gly Val
    610                     615                     620
Glu Asp Arg Phe Asn Ala Ser Leu Gly Thr Tyr His Asp Leu Leu Lys
625                     630                     635                     640
Ile Ile Lys Asp Lys Asp Phe Leu Asp Asn Glu Glu Lys Glu Asp Ile
                645                     650                     655
Leu Glu Asp Ile Val Leu Thr Leu Thr Leu Phe Glu Asp Arg Glu Met
            660                     665                     670
Ile Glu Glu Arg Leu Lys Thr Tyr Ala His Leu Phe Asp Asp Lys Val
        675                     680                     685
Met Lys Gln Leu Lys Arg Arg Arg Tyr Thr Gly Trp Gly Arg Leu Ser
    690                     695                     700
Arg Lys Leu Ile Asn Gly Ile Arg Asp Lys Gln Ser Gly Lys Thr Ile
705                     710                     715                     720
Leu Asp Phe Leu Lys Ser Asp Gly Phe Ala Asn Arg Asn Phe Met Gln
                725                     730                     735
Leu Ile His Asp Asp Ser Leu Thr Phe Lys Glu Asp Ile Gln Lys Ala
            740                     745                     750
Gln Val Ser Gly Gln Gly Asp Ser Leu His Glu His Ile Ala Asn Leu
        755                     760                     765
Ala Gly Ser Pro Ala Ile Lys Lys Gly Ile Leu Gln Thr Val Lys Val
    770                     775                     780
Val Asp Glu Leu Val Lys Val Met Gly Arg His Lys Pro Glu Asn Ile
785                     790                     795                     800
Val Ile Glu Met Ala Arg Glu Asn Gln Thr Thr Gln Lys Gly Gln Lys
                805                     810                     815
Asn Ser Arg Glu Arg Met Lys Arg Ile Glu Glu Gly Ile Lys Glu Leu
            820                     825                     830
Gly Ser Gln Ile Leu Lys Glu His Pro Val Glu Asn Thr Gln Leu Gln
        835                     840                     845
Asn Glu Lys Leu Tyr Leu Tyr Tyr Leu Gln Asn Gly Arg Asp Met Tyr
    850                     855                     860
Val Asp Gln Glu Leu Asp Ile Asn Arg Leu Ser Asp Tyr Asp Val Asp
865                     870                     875                     880
Ala Ile Val Pro Gln Ser Phe Leu Lys Asp Asp Ser Ile Asp Asn Lys
                885                     890                     895
Val Leu Thr Arg Ser Asp Lys Asn Arg Gly Lys Ser Asp Asn Val Pro
            900                     905                     910
Ser Glu Glu Val Val Lys Lys Met Lys Asn Tyr Trp Arg Gln Leu Leu
        915                     920                     925
Asn Ala Lys Leu Ile Thr Gln Arg Lys Phe Asp Asn Leu Thr Lys Ala
    930                     935                     940
Glu Arg Gly Gly Leu Ser Glu Leu Asp Lys Ala Gly Phe Ile Lys Arg
945                     950                     955                     960
Gln Leu Val Glu Thr Arg Gln Ile Thr Lys His Val Ala Gln Ile Leu
                965                     970                     975
Asp Ser Arg Met Asn Thr Lys Tyr Asp Glu Asn Asp Lys Leu Ile Arg
            980                     985                     990
Glu Val Lys Val Ile Thr Leu Lys  Ser Lys Leu Val Ser  Asp Phe Arg
        995                     1000                    1005

Lys Asp Phe Gln Phe Tyr Lys Val Arg Glu Ile Asn Asn Tyr His His
    1010                1015                1020
Ala His Asp Ala Tyr Leu Asn Ala Val Val Gly Thr Ala Leu Ile Lys
1025                1030                1035                1040
Lys Tyr Pro Lys Leu Glu Ser Glu Phe Val Tyr Gly Asp Tyr Lys Val
                1045                1050                1055
Tyr Asp Val Arg Lys Met Ile Ala Lys Ser Glu Gln Glu Ile Gly Lys
            1060                1065                1070
Ala Thr Ala Lys Tyr Phe Phe Tyr Ser Asn Ile Met Asn Phe Phe Lys
        1075                1080                1085
Thr Glu Ile Thr Leu Ala Asn Gly Glu Ile Arg Lys Arg Pro Leu Ile
    1090                1095                1100
Glu Thr Asn Gly Glu Thr Gly Glu Ile Val Trp Asp Lys Gly Arg Asp
1105                1110                1115                1120
Phe Ala Thr Val Arg Lys Val Leu Ser Met Pro Gln Val Asn Ile Val
                1125                1130                1135
Lys Lys Thr Glu Val Gln Thr Gly Gly Phe Ser Lys Glu Ser Ile Leu
            1140                1145                1150
Pro Lys Arg Asn Ser Asp Lys Leu Ile Ala Arg Lys Lys Asp Trp Asp
        1155                1160                1165
Pro Lys Lys Tyr Gly Gly Phe Asp Ser Pro Thr Val Ala Tyr Ser Val
    1170                1175                1180
Leu Val Val Ala Lys Val Glu Lys Gly Lys Ser Lys Lys Leu Lys Ser
1185                1190                1195                1200
Val Lys Glu Leu Leu Gly Ile Thr Ile Met Glu Arg Ser Ser Phe Glu
                1205                1210                1215
Lys Asn Pro Ile Asp Phe Leu Glu Ala Lys Gly Tyr Lys Glu Val Lys
            1220                1225                1230
Lys Asp Leu Ile Ile Lys Leu Pro Lys Tyr Ser Leu Phe Glu Leu Glu
        1235                1240                1245
Asn Gly Arg Lys Arg Met Leu Ala Ser Ala Gly Glu Leu Gln Lys Gly
    1250                1255                1260
Asn Glu Leu Ala Leu Pro Ser Lys Tyr Val Asn Phe Leu Tyr Leu Ala
1265                1270                1275                1280
Ser His Tyr Glu Lys Leu Lys Gly Ser Pro Glu Asp Asn Glu Gln Lys
                1285                1290                1295
Gln Leu Phe Val Glu Gln His Lys His Tyr Leu Asp Glu Ile Ile Glu
            1300                1305                1310
Gln Ile Ser Glu Phe Ser Lys Arg Val Ile Leu Ala Asp Ala Asn Leu
        1315                1320                1325
Asp Lys Val Leu Ser Ala Tyr Asn Lys His Arg Asp Lys Pro Ile Arg
    1330                1335                1340
Glu Gln Ala Glu Asn Ile Ile His Leu Phe Thr Leu Thr Asn Leu Gly
1345                1350                1355                1360
Ala Pro Ala Ala Phe Lys Tyr Phe Asp Thr Thr Ile Asp Arg Lys Arg
                1365                1370                1375
Tyr Thr Ser Thr Lys Glu Val Leu Asp Ala Thr Leu Ile His Gln Ser
            1380                1385                1390
Ile Thr Gly Leu Tyr Glu Thr Arg Ile Asp Leu Ser Gln Leu Gly Gly
        1395                1400                1405
Asp Glu Gly Ala Pro Lys Lys Lys Arg Lys Val Gly Ser Ser Gly Ser
    1410                1415                1420
Pro Lys Lys Lys Arg Lys Val Ser Gly Ser Glu Thr Pro Gly Thr Ser
1425                1430                1435                1440
Glu Ser Ala Thr Pro Glu Ser Met Asp Ser Leu Leu Met Asn Arg Arg
                1445                1450                1455
Lys Phe Leu Tyr Gln Phe Lys Asn Val Arg Trp Ala Lys Gly Arg Arg
            1460                1465                1470
Glu Thr Tyr Leu Cys Tyr Val Val Lys Arg Arg Asp Ser Ala Thr Ser
        1475                1480                1485
Phe Ser Leu Asp Phe Gly Tyr Leu Arg Asn Lys Asn Gly Cys His Val
    1490                1495                1500
Glu Leu Leu Phe Leu Arg Tyr Ile Ser Asp Trp Asp Leu Asp Pro Gly

1505                1510                1515                1520
Arg Cys Tyr Arg Val  Thr Trp Phe Thr Ser  Trp Ser Pro Cys Tyr  Asp
                1525                1530                1535
Cys Ala Arg His  Val Ala Asp Phe Leu  Arg Gly Asn Pro Asn  Leu Ser
            1540                1545                1550
Leu Arg Ile  Phe Thr Ala Arg Leu  Tyr Phe Cys Glu Asp  Arg Lys Ala
        1555                1560                1565
Glu Pro  Glu Gly Leu Arg Arg  Leu His Arg Ala Gly  Val Gln Ile Ala
    1570                1575                1580
Ile  Met Thr Phe Lys Asp  Tyr Phe Tyr Cys Trp  Asn Thr Phe Val Glu
1585                1590                1595                1600
Asn His Glu Arg Thr  Phe Lys Ala Trp Glu  Gly Leu His Glu Asn  Ser
                1605                1610                1615
Val Arg Leu Ser  Arg Gln Leu Arg Arg  Ile Leu Leu Pro
            1620                1625

<210>  49
<211>  4089
<212>  DNA
<213>  Artificial Sequence

<220>
<223>  Coding sequence of AIDx-saCas9(KKH nickase)-Ugi

<400>  49
atggacagcc tcttgatgaa ccggaggaag tttctttacc aattcaaaaa tgtccgctgg      60
gctaagggtc ggcgtgagac ctacctgtgc tacgtagtga agaggcgtga cagtgctaca     120
tccttttcac tggactttgg ttatcttcgc aataagaacg gctgccacgt ggaattgctc     180
ttcctccgct acatctcgga ctgggaccta gaccctggcc gctgctaccg cgtcacctgg     240
ttcacctcct ggagcccctg ctacgactgt gcccgacatg tggccgactt tctgcgaggg     300
aaccccaacc tcagtctgag gatcttcacc gcgcgcctct acttctgtga ggaccgcaag     360
gctgagcccg aggggctgcg gcggctgcac cgcgccgggg tgcaaatagc catcatgacc     420
ttcaaagatt atttttactg ctggaatact tttgtagaaa accatgaaag aactttcaaa     480
gcctgggaag ggctgcatga aaattcagtt cgtctctcca gacagcttcg gcgcatcctt     540
ttgcccagcg gcagcgagac tcccgggacc tcagagtccg ccacacccga aagtgggaaa     600
cggaactaca tcctggggct tgacattggg ataaccagcg ttggctacgg aattattgat     660
tatgagacac gcgatgtgat tgacgccggg gttaggctgt tcaaagaggc caacgttgaa     720
aacaacgagg gaagacggag taagcgcgga gcaagaagac tcaagcgcag acggagacat     780
cggattcaga gggtgaaaaa gctgctcttc gattacaatc tcctgaccga tcatagtgag     840
ctgagcggaa tcaaccccta cgaggcgcga gtgaaagggc tttcccagaa gctgtccgaa     900
gaggagttct ccgccgcgtt gctgcacctg gccaaacgga ggggggttca caatgtaaac     960
gaagtggagg aggacacggg caatgaactt agtacgaaag aacagatcag taggaactct    1020
aaggctctcg aagagaaata cgtcgctgag ttgcagcttg agagactgaa aaaagacggc    1080
gaagtacgcg gatctattaa taggttcaag acttcagatt acgtaaagga agccaagcag    1140
ctcctgaaag tacagaaagc gtaccatcag ctcgatcaga gcttcatcga tacctacata    1200
gatttgctgg agacacggag gacatactac gagggcccag gggaaggatc tccttttggg    1260
tggaaggaca tcaaggaatg gtacgagatg cttatgggac attgtacata ttttccggag    1320
gagctcagga gcgtcaagta cgcctacaat gccgacctgt acaatgccct caatgacctc    1380
aataacctcg tgattaccag ggacgagaac gagaagctgg agtactatga aaagttccag    1440
attatcgaga atgtgtttaa gcagaagaag aagccgacac ttaagcagat tgcaaaggaa    1500
atcctcgtga atgaggaaga tatcaaggga tacagagtga caagtacagg caagcccgag    1560
ttcacaaatc tgaaggtgta ccacgatatt aaggacataa ccgcacgaaa ggagataatc    1620
gaaaacgctg agctcctcga tcagatcgca aaaattctta ccatctacca gtctagtgag    1680
gacattcagg aggaactgac taatctgaac agtgagctca cccaagagga aattgagcag    1740
atttcaaacc tgaaaggcta caccggaacg cacaatctga gcctcaaagc aatcaacctc    1800
attctggatg aactttggca cacaaatgac aaccaaattg ccatattcaa ccgcctgaaa    1860
ctggtgccaa aaaaagtgga tctgtcacag caaaaggaaa tccctacaac cttggttgac    1920
gattttattc tgtcccccgt tgtcaagcgg agcttcatcc agtcaatcaa ggtgatcaat    1980
gccatcatta aaaaatacgg attgccaaac gatataatta tcgagcttgc acgagagaag    2040
aactcaaagg acgcccagaa gatgattaac gaaatgcaga agcgcaaccg ccagacaaac    2100
gaacgcatag aggaaattat aagaacaacc ggcaaagaga atgccaagta tctgatcgag    2160
aaaatcaagc tgcacgacat gcaagaaggc aagtgcctgt actctctgga agctatccca    2220
ctcgaagatc tgctgaataa tccattcaat tacgaggtgg accacatcat ccctagatcc    2280

gtaagctttg acaattcctt caataacaaa gttctggtta aacaggagga aaattctaaa 2340
aaagggaacc ggaccccgtt ccagtacctg agctccagtg acagcaagat tagctacgag 2400
acttttaaga aacatattct gaatctggcc aaaggcaaag gcaggatcag caagaccaag 2460
aaggagtacc tcctcgaaga acgcgacatt aacagattta gtgtgcagaa agatttcatc 2520
aaccgaaacc ttgtcgatac tcggtacgcc acgagaggcc tgatgaatct cctcaggagc 2580
tacttccgcg tcaataatct ggacgttaaa gtcaagagca taaatggggg attcaccagc 2640
tttctgagga gaaagtggaa gtttaagaag gaacgaaaca aaggatacaa gcaccatgct 2700
gaggatgctt tgatcatcgc taacgcggac tttatcttta aggaatggaa aaagctggat 2760
aaggcaaaga aagtgatgga aaaccagatg ttcgaggaga agcaggcaga gtcaatgcct 2820
gagatcgaga cagagcagga atacaaggaa attttcatca cccctcatca gattaaacac 2880
ataaaggact tcaaagacta taaatactct catagggtgg acaaaaaacc caatcgcaag 2940
ctcattaatg acaccctgta ctcaacacgg aaggatgata aaggtaatac cttgattgtg 3000
aataatctta atggattgta tgacaaagat aacgacaagc tcaagaagct gatcaacaag 3060
tctccagaga agctccttat gtatcaccac gacccacaga cttatcagaa attgaaactg 3120
atcatggagc aatacgggga tgagaagaac ccactctaca aatattatga ggaaacaggt 3180
aattacctga ccaagtactc caagaaggat aacggaccag tgatcaaaaa gataaagtac 3240
tatggcaaca aacttaatgc gcatttggac ataactgacg attaccccaa ttctcgaaac 3300
aaggttgtga agctctccct gaagccttat agatttgacg tgtacctgga taatggggtt 3360
tataaattcg tcaccgtgaa aaatctggac gtgatcaaaa aggagaacta ttatgaagta 3420
aactcaaagt gctatgagga ggcgaagaag ctgaagaaga tctccaatca ggccgagttc 3480
atcgcttcct tctataagaa cgatctcatc aagatcaatg gagagcttta tcgcgtcatt 3540
ggtgtgaaca atgacttgct gaacaggatc gaagtcaata tgatagacat tacctaccgg 3600
gagtatctcg aaaacatgaa tgataaacgg ccgcctcaca tcatcaagac aatcgcatct 3660
aaaactcagt caataaaaaa gtactctacc gatatcctgg ggaatctcta tgaagtgaag 3720
tcaaagaagc acccacaaat cattaaaaaa ggtggatcct ctggtggttc tactaatctg 3780
tcagatatta ttgaaaagga gaccggtaag caactggtta tccaggaatc catcctcatg 3840
ctcccagagg aggtggaaga agtcattggg aacaagccgg aaagcgatat actcgtgcac 3900
accgcctacg acgagagcac cgacgagaat gtcatgcttc tgactagcga cgcccctgaa 3960
tacaagcctt gggctctggt catacaggat agcaacggtg agaacaagat taagatgctc 4020
tctggtggtt ctcccaagaa gaagaggaaa gtcggatcct acccatacga tgttccagat 4080
tacgcttaa 4089

<210> 50
<211> 1362
<212> PRT
<213> Artificial Sequence

<220>
<223> Amino acid sequence of AIDx-saCas9(KKH nickase)-Ugi

<400> 50
Met Asp Ser Leu Leu Met Asn Arg Arg Lys Phe Leu Tyr Gln Phe Lys
1               5                   10                  15
Asn Val Arg Trp Ala Lys Gly Arg Arg Glu Thr Tyr Leu Cys Tyr Val
            20                  25                  30
Val Lys Arg Arg Asp Ser Ala Thr Ser Phe Ser Leu Asp Phe Gly Tyr
        35                  40                  45
Leu Arg Asn Lys Asn Gly Cys His Val Glu Leu Leu Phe Leu Arg Tyr
    50                  55                  60
Ile Ser Asp Trp Asp Leu Asp Pro Gly Arg Cys Tyr Arg Val Thr Trp
65                  70                  75                  80
Phe Thr Ser Trp Ser Pro Cys Tyr Asp Cys Ala Arg His Val Ala Asp
                85                  90                  95
Phe Leu Arg Gly Asn Pro Asn Leu Ser Leu Arg Ile Phe Thr Ala Arg
            100                 105                 110
Leu Tyr Phe Cys Glu Asp Arg Lys Ala Glu Pro Glu Gly Leu Arg Arg
        115                 120                 125
Leu His Arg Ala Gly Val Gln Ile Ala Ile Met Thr Phe Lys Asp Tyr
    130                 135                 140
Phe Tyr Cys Trp Asn Thr Phe Val Glu Asn His Glu Arg Thr Phe Lys
145                 150                 155                 160
Ala Trp Glu Gly Leu His Glu Asn Ser Val Arg Leu Ser Arg Gln Leu
                165                 170                 175

Arg Arg Ile Leu Leu Pro Ser Gly Ser Glu Thr Pro Gly Thr Ser Glu
            180                 185                 190
Ser Ala Thr Pro Glu Ser Gly Lys Arg Asn Tyr Ile Leu Gly Leu Asp
        195                 200                 205
Ile Gly Ile Thr Ser Val Gly Tyr Gly Ile Ile Asp Tyr Glu Thr Arg
    210                 215                 220
Asp Val Ile Asp Ala Gly Val Arg Leu Phe Lys Glu Ala Asn Val Glu
225                 230                 235                 240
Asn Asn Glu Gly Arg Arg Ser Lys Arg Gly Ala Arg Arg Leu Lys Arg
                245                 250                 255
Arg Arg Arg His Arg Ile Gln Arg Val Lys Lys Leu Leu Phe Asp Tyr
            260                 265                 270
Asn Leu Leu Thr Asp His Ser Glu Leu Ser Gly Ile Asn Pro Tyr Glu
        275                 280                 285
Ala Arg Val Lys Gly Leu Ser Gln Lys Leu Ser Glu Glu Glu Phe Ser
    290                 295                 300
Ala Ala Leu Leu His Leu Ala Lys Arg Arg Gly Val His Asn Val Asn
305                 310                 315                 320
Glu Val Glu Glu Asp Thr Gly Asn Glu Leu Ser Thr Lys Glu Gln Ile
                325                 330                 335
Ser Arg Asn Ser Lys Ala Leu Glu Glu Lys Tyr Val Ala Glu Leu Gln
            340                 345                 350
Leu Glu Arg Leu Lys Lys Asp Gly Glu Val Arg Gly Ser Ile Asn Arg
        355                 360                 365
Phe Lys Thr Ser Asp Tyr Val Lys Glu Ala Lys Gln Leu Leu Lys Val
    370                 375                 380
Gln Lys Ala Tyr His Gln Leu Asp Gln Ser Phe Ile Asp Thr Tyr Ile
385                 390                 395                 400
Asp Leu Leu Glu Thr Arg Arg Thr Tyr Tyr Glu Gly Pro Gly Glu Gly
                405                 410                 415
Ser Pro Phe Gly Trp Lys Asp Ile Lys Glu Trp Tyr Glu Met Leu Met
            420                 425                 430
Gly His Cys Thr Tyr Phe Pro Glu Glu Leu Arg Ser Val Lys Tyr Ala
        435                 440                 445
Tyr Asn Ala Asp Leu Tyr Asn Ala Leu Asn Asp Leu Asn Asn Leu Val
    450                 455                 460
Ile Thr Arg Asp Glu Asn Glu Lys Leu Glu Tyr Tyr Glu Lys Phe Gln
465                 470                 475                 480
Ile Ile Glu Asn Val Phe Lys Gln Lys Lys Lys Pro Thr Leu Lys Gln
                485                 490                 495
Ile Ala Lys Glu Ile Leu Val Asn Glu Glu Asp Ile Lys Gly Tyr Arg
            500                 505                 510
Val Thr Ser Thr Gly Lys Pro Glu Phe Thr Asn Leu Lys Val Tyr His
        515                 520                 525
Asp Ile Lys Asp Ile Thr Ala Arg Lys Glu Ile Ile Glu Asn Ala Glu
    530                 535                 540
Leu Leu Asp Gln Ile Ala Lys Ile Leu Thr Ile Tyr Gln Ser Ser Glu
545                 550                 555                 560
Asp Ile Gln Glu Glu Leu Thr Asn Leu Asn Ser Glu Leu Thr Gln Glu
                565                 570                 575
Glu Ile Glu Gln Ile Ser Asn Leu Lys Gly Tyr Thr Gly Thr His Asn
            580                 585                 590
Leu Ser Leu Lys Ala Ile Asn Leu Ile Leu Asp Glu Leu Trp His Thr
        595                 600                 605
Asn Asp Asn Gln Ile Ala Ile Phe Asn Arg Leu Lys Leu Val Pro Lys
    610                 615                 620
Lys Val Asp Leu Ser Gln Gln Lys Glu Ile Pro Thr Thr Leu Val Asp
625                 630                 635                 640
Asp Phe Ile Leu Ser Pro Val Val Lys Arg Ser Phe Ile Gln Ser Ile
                645                 650                 655
Lys Val Ile Asn Ala Ile Ile Lys Lys Tyr Gly Leu Pro Asn Asp Ile
            660                 665                 670
Ile Ile Glu Leu Ala Arg Glu Lys Asn Ser Lys Asp Ala Gln Lys Met

        675                 680                 685
Ile Asn Glu Met Gln Lys Arg Asn Arg Gln Thr Asn Glu Arg Ile Glu
    690                 695                 700
Glu Ile Ile Arg Thr Thr Gly Lys Glu Asn Ala Lys Tyr Leu Ile Glu
705                 710                 715                 720
Lys Ile Lys Leu His Asp Met Gln Glu Gly Lys Cys Leu Tyr Ser Leu
                725                 730                 735
Glu Ala Ile Pro Leu Glu Asp Leu Leu Asn Asn Pro Phe Asn Tyr Glu
            740                 745                 750
Val Asp His Ile Ile Pro Arg Ser Val Ser Phe Asp Asn Ser Phe Asn
        755                 760                 765
Asn Lys Val Leu Val Lys Gln Glu Glu Asn Ser Lys Lys Gly Asn Arg
    770                 775                 780
Thr Pro Phe Gln Tyr Leu Ser Ser Ser Asp Ser Lys Ile Ser Tyr Glu
785                 790                 795                 800
Thr Phe Lys Lys His Ile Leu Asn Leu Ala Lys Gly Lys Gly Arg Ile
                805                 810                 815
Ser Lys Thr Lys Lys Glu Tyr Leu Leu Glu Glu Arg Asp Ile Asn Arg
            820                 825                 830
Phe Ser Val Gln Lys Asp Phe Ile Asn Arg Asn Leu Val Asp Thr Arg
        835                 840                 845
Tyr Ala Thr Arg Gly Leu Met Asn Leu Leu Arg Ser Tyr Phe Arg Val
    850                 855                 860
Asn Asn Leu Asp Val Lys Val Lys Ser Ile Asn Gly Gly Phe Thr Ser
865                 870                 875                 880
Phe Leu Arg Arg Lys Trp Lys Phe Lys Lys Glu Arg Asn Lys Gly Tyr
                885                 890                 895
Lys His His Ala Glu Asp Ala Leu Ile Ile Ala Asn Ala Asp Phe Ile
            900                 905                 910
Phe Lys Glu Trp Lys Lys Leu Asp Lys Ala Lys Lys Val Met Glu Asn
        915                 920                 925
Gln Met Phe Glu Glu Lys Gln Ala Glu Ser Met Pro Glu Ile Glu Thr
    930                 935                 940
Glu Gln Glu Tyr Lys Glu Ile Phe Ile Thr Pro His Gln Ile Lys His
945                 950                 955                 960
Ile Lys Asp Phe Lys Asp Tyr Lys Tyr Ser His Arg Val Asp Lys Lys
                965                 970                 975
Pro Asn Arg Lys Leu Ile Asn Asp Thr Leu Tyr Ser Thr Arg Lys Asp
            980                 985                 990
Asp Lys Gly Asn Thr Leu Ile Val Asn Asn Leu Asn Gly Leu Tyr Asp
        995                 1000                1005
Lys Asp Asn Asp Lys Leu Lys Lys Leu Ile Asn Lys Ser Pro Glu Lys
    1010                1015                1020
Leu Leu Met Tyr His His Asp Pro Gln Thr Tyr Gln Lys Leu Lys Leu
1025                1030                1035                1040
Ile Met Glu Gln Tyr Gly Asp Glu Lys Asn Pro Leu Tyr Lys Tyr Tyr
                1045                1050                1055
Glu Glu Thr Gly Asn Tyr Leu Thr Lys Tyr Ser Lys Lys Asp Asn Gly
            1060                1065                1070
Pro Val Ile Lys Lys Ile Lys Tyr Tyr Gly Asn Lys Leu Asn Ala His
        1075                1080                1085
Leu Asp Ile Thr Asp Asp Tyr Pro Asn Ser Arg Asn Lys Val Val Lys
    1090                1095                1100
Leu Ser Leu Lys Pro Tyr Arg Phe Asp Val Tyr Leu Asp Asn Gly Val
1105                1110                1115                1120
Tyr Lys Phe Val Thr Val Lys Asn Leu Asp Val Ile Lys Lys Glu Asn
                1125                1130                1135
Tyr Tyr Glu Val Asn Ser Lys Cys Tyr Glu Glu Ala Lys Lys Leu Lys
            1140                1145                1150
Lys Ile Ser Asn Gln Ala Glu Phe Ile Ala Ser Phe Tyr Lys Asn Asp
        1155                1160                1165
Leu Ile Lys Ile Asn Gly Glu Leu Tyr Arg Val Ile Gly Val Asn Asn
    1170                1175                1180

Asp Leu Leu Asn Arg Ile Glu Val Asn Met Ile Asp Ile Thr Tyr Arg
1185                1190                1195                1200
Glu Tyr Leu Glu Asn Met Asn Asp Lys Arg Pro Pro His Ile Ile Lys
                1205                1210                1215
Thr Ile Ala Ser Lys Thr Gln Ser Ile Lys Lys Tyr Ser Thr Asp Ile
            1220                1225                1230
Leu Gly Asn Leu Tyr Glu Val Lys Ser Lys Lys His Pro Gln Ile Ile
        1235                1240                1245
Lys Lys Gly Gly Ser Ser Gly Gly Ser Thr Asn Leu Ser Asp Ile Ile
    1250                1255                1260
Glu Lys Glu Thr Gly Lys Gln Leu Val Ile Gln Glu Ser Ile Leu Met
1265                1270                1275                1280
Leu Pro Glu Glu Val Glu Glu Val Ile Gly Asn Lys Pro Glu Ser Asp
                1285                1290                1295
Ile Leu Val His Thr Ala Tyr Asp Glu Ser Thr Asp Glu Asn Val Met
            1300                1305                1310
Leu Leu Thr Ser Asp Ala Pro Glu Tyr Lys Pro Trp Ala Leu Val Ile
        1315                1320                1325
Gln Asp Ser Asn Gly Glu Asn Lys Ile Lys Met Leu Ser Gly Gly Ser
    1330                1335                1340
Pro Lys Lys Lys Arg Lys Val Gly Ser Tyr Pro Tyr Asp Val Pro Asp
1345                1350                1355                1360
Tyr Ala

<210> 51
<211> 20
<212> DNA
<213> Artificial Sequence

<220>
<223> sgRNA DMD EXON50 5'SS

<400> 51
acttacaggc tccaatagtg 20

<210> 52
<211> 103
<212> DNA
<213> Artificial Sequence

<220>
<223> sgRNA Backbond sequence

<220>
<221> misc_feature
<222> (1)..(20)
<223> n is a, c, g or t

<400> 52
nnnnnnnnnn nnnnnnnnnn gttatagtac tctggaaaca gaatctacta taacaaggca 60
aaatgccgtg tttatctcgt caacttgttg gcgagatttt ttt 103